# EUROPEAN PATENT APPLICATION

(11) **EP 1 939 202 A1**
(43) Date of publication of application: **02.07.2008**
(21) Application number: 06810299.5
(22) Date of filing: 20.09.2006
(51) Int. Cl.: C07D 473/18, A61K 31/522, A61K 31/5377, A61K 31/5386, A61K 31/5513, A61P 11/02, A61P 11/06, A61P 17/00, A61P 27/02, A61P 31/12, A61P 31/18, A61P 35/00, A61P 37/02, A61P 37/08, A61P 43/00, C07D 473/16, C07D 473/24, C07D 473/34, C07D 519/00

(54) **NOVEL ADENINE COMPOUND**

(30) Priority: 22.09.2005 JP 2005276170
(71) Applicant: Dainippon Sumitomo Pharma Co., Ltd., Osaka 541-8524 (JP); AstraZeneca AB, 151 85 Södertälje (SE)
(72) Inventor: HASHIMOTO, Kazuki, Konohana-ku, Osaka-shi, Osaka, 554-0022 (JP); NAKAMURA, Tomoaki, Osaka-shi, Osaka, 554-0022 (JP); NAKAMURA, Kei, Osaka-shi, Osaka, 554-0022 (JP); KURIMOTO, Ayumu, Osaka-shi, Osaka, 554-0022 (JP); ISOBE, Yoshiaki, Osaka-shi, Osaka, 554-0022 (JP)
(74) Representative: Vossius & Partner
(86) International application number: PCT/JP2006/318603
(87) International publication number: WO 2007/034817

(57) **Abstract**

An adenine compound useful for a medicament represented by the following formula (1): 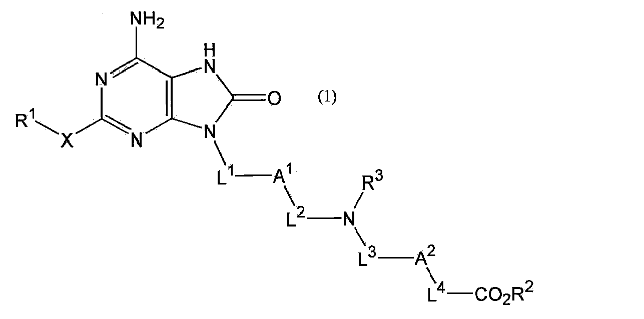 , wherein A¹ and A² are independently, optionally substituted cyclic aromatic hydrocarbon group or optionally substituted aromatic heterocyclic group;
L¹, L² and L³ are independently, alkylene or a single optionally substituted alkylene or a single bond, and any methylene or methyne in L² or L³ may be bound with N atom adjacent to L² and L³ to form 4 to 7 membered saturated hetero ring containing N atom;
L⁴ is alkylene or a single bond;
R¹ is optionally substituted alkyl group, optionally substituted alkenyl group, optionally substituted aryl group, etc.;
R² is hydrogen atom or optionally substituted alkyl group;
R³ is optionally substituted alkyl group, etc.;
X is oxygen atom, etc.;
or its pharmaceutically acceptable salt.

## Description

### TECHNICAL FIELD

The present invention relates to a novel adenine compound useful as a prophylactic or therapeutic agent for allergic diseases, viral diseases, cancers, etc.

### BACKGROUND ART

When a foreign substance such as bacteria, virus or parasite invades into a living body, immune system works to defend from the foreign substance. In acquired immune system, once a foreign substance invades, antigen is processed by antigen presenting cells such as dendritic cells (DC), and naive cells, via mutual action of DC/Th cells, functionally differentiate into Th1 cells or Th2 cells which contribute the main role to immune response in a living body. In this processing, when immune balance deviates to either one of Th1 cells or Th2 cells, it is considered that immune diseases develop.

Namely, in a body of a patient suffering from an allergic disease, cytokines such as interleukin-4 (IL-4) and interleukin-5 (IL-5) secreted from Th2 cells are excessively secreted. Therefore, compounds suppressing an immune response of Th2 cell can be expected as an agent for treating allergic diseases. On the other hand, compounds enhancing an immune response of Th1 cell can be expected as an agent for treating viral diseases, cancers, etc.

Natural immune system has been considered due to non specific phagocytosis. However, the presence of Toll-like receptor (TLR) is confirmed, and activation of the natural immune response is found to be mainly done via TLR. Once TLR recognizes ligands, it induces inflammatory cytokines such as IL-12, TNF, etc. As IL-12 induces naive T cells into Th1 cells, ligands of TLR have a function as a Th1/Th2 differentiation controlling agent, the ligands are expected as a prophylaxis or therapeutic agent for immune diseases. In fact it is known that Th2 cells are dominant in the patients suffering from asthma or atopic dermatitis, and asthma-targeted clinical trials are carried out for DNA (CpG DNA) derived from microorganism, TLR9 agonist. It is also known that imidazoquinoline derivatives, TLR7/8 agonist (See Patent Document 1) show an activity suppressing the production of Th2 cytokines, i.e. interleukin 4 (IL-4) and interleukin 5 (IL-5), and in fact are effective for treatment of allergic diseases in animal model.

On the other hand, compounds having an adenine structure and effective for treatment of immune diseases such as viral diseases or allergic diseases are disclosed in following patent documents 2 to 4.
[Patent Document 1] US Patent 4,689,338
[Patent Document 2] WO 98/01448
[Patent Document 3] WO 99/28321
[Patent Document 4] WO 04/029054

### DISCLOSURE OF INVENTION

The problem to be solved by the present invention is to provide TLR activating agents, in more detail, the novel adenine compounds having TLR7 activating effect, an immune modulator containing them, such as prophylactic or therapeutic agents for allergic diseases such as asthma, COPD, allergic rhinitis, allergic conjunctivitis and atopic dermatosis, viral diseases such as hepatitis B, hepatitis C, HIV and HPV, bacterial infectious diseases, cancers and dermatosis.

The present inventors earnestly investigated in order to find a therapeutic or prophylactic agent for allergic diseases, viral diseases or cancers, having excellent TLR activating effect and succeeded in finding a novel compound of the present invention. Namely the compound of the present invention is useful for therapeutic and prophylactic agent of allergic diseases, viral diseases and cancers.

Thus the present invention has been completed based on the above findings.

Namely the present invention relates to the following invention or embodiments.
[1] An adenine compound represented by the following formula (1): 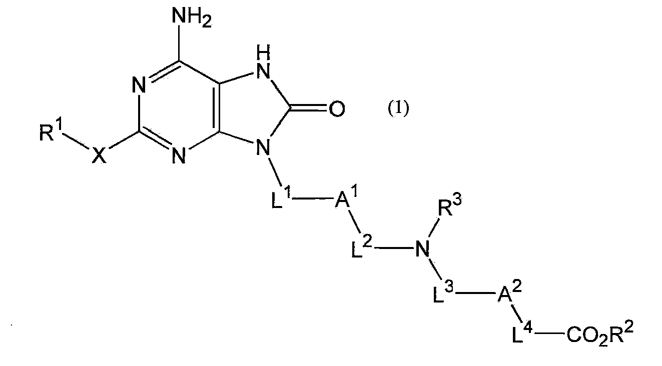 [wherein
   A¹ and A² are independently, optionally substituted aromatic carbocyclic group or optionally substituted aromatic heterocyclic group;
   L⁴ is optionally substituted alkylene or a single bond;
   R¹ is halogen atom, optionally substituted alkyl group, optionally substituted alkenyl group, optionally substituted alkynyl group, optionally substituted cycloalkyl group, optionally substituted saturated heterocyclic group, optionally substituted aryl group or optionally substituted heteroaryl group;
   R² is hydrogen atom or optionally substituted alkyl group;
   R³ is optionally substituted alkyl group, optionally substituted alkenyl group, optionally substituted alkynyl group, optionally substituted cycloalkyl group, optionally substituted saturated heterocyclic group, optionally substituted aryl group or optionally substituted heteroaryl group;
   X is oxygen atom, sulfur atom, NR⁹ (wherein R⁹ is hydrogen atom or alkyl group), SO, SO₂ or a single bond, provided that X is a single bond when R¹ is halogen atom;
   and
   L¹, L² and L³ are independently, alkylene, alkenylene, alkynylene or a single bond and any methylene group in said group may be substituted by oxygen atom, sulfur atom, SO, SO₂, carbonyl group, NR⁴, NR⁴CO, CONR⁴, NR⁴SO₂, SO₂NR⁴, NR⁴CO₂, OCONR⁴, NR⁵CONR⁴, NR⁶C(=NR⁴)NR⁵ or C(=NR⁴)NR⁵ (wherein R⁴, R⁵ and R⁶ are independently, hydrogen atom or optionally substituted alkyl group), and any methylene, methyne or imino in L² or L³ may be bound with N atom adjacent to L² and L³ to form 4 to 7 membered saturated nitrogen containing hetero cycle, and when methylene in L³ is substituted by NR⁴, optionally substituted alkyl group in R⁴ may be bound with carbon atom in L³ to form 4 to 7 membered saturated nitrogen containing heterocycle.]
   or its pharmaceutically acceptable salt.
[2] The adenine compound or its pharmaceutically acceptable salt described in the above [1], wherein substituted aromatic carbocyclic group or substituted aromatic heterocyclic group in A¹ and A² of the formula (1) is substituted by one or more substituents selected from the group consisting of halogen atom, hydroxy group, C₁₋₆ alkyl group, C₁₋₆ alkoxy group, C₁₋₆ haloalkyl group, C₁₋₆ haloalkoxy group, carboxy group, C₂₋₆ alkoxycarbonyl group, nitro group and amino group optionally substituted by one or more C₁₋₆ alkyl groups;
   substituted alkyl group in R⁴, R⁵ and R⁶ is substituted by one or more substituents independently selected from the group consisting of halogen atom, hydroxy group, C₁₋₆ alkoxy group and optionally substituted amino group;
   substituent of the above substituted amino group is a group selected from the group (a') or a group (b');
   (a') C₁₋₆ alkyl group, C₂₋₆ alkenyl group, C₂₋₆ alkynyl group, C₂₋₆ alkylcarbonyl group, C₂₋₆ alkoxycarbonyl group, C₁₋₆ alkylsulfonyl group,
      C₁₋₆ alkylsulfinyl group, 3 to 8 membered cycloalkyl group, 3 to 8 membered cycloalkylcarbonyl group, 3 to 8 membered cycloalkoxycarbonyl group, 3 to 8 membered cycloalkylsulfonyl group and 3 to 8 membered cycloalkylsulfinyl group (wherein the group in this group may be substituted by halogen atom, hydroxy group, C₁₋₆ alkyl group, C₁₋₆ alkoxy group, carboxy group and C₂₋₆ alkoxycarbonyl group.);
   (b') 4 to 7 membered saturated heterocyclic group having one to three hetero atoms selected from 1 to 3 nitrogen atoms, 0 to 1 oxygen atom and 0 to 1 sulfur atom which is formed by combining two substituents with the nitrogen atom (said saturated heterocyclic group may be substituted on its carbon atom or nitrogen atom, if chemically stable, by halogen atom, hydroxy group, carboxy group, C₁₋₆ alkyl group, C₁₋₆ alkoxy group, C₂₋₆ alkoxycarbonyl group or C₂₋₆ alkylcarbonyl group.),
   substituted alkyl group in R¹, R² and R³, and substituted alkenyl group and substituted alkynyl group in R¹ and R³ is substituted by one or more substituents independently selected from the group consisting of groups (a) to (c) below;
   (a) halogen atom, hydroxy group, carboxy group, C₁₋₆ haloalkoxy group and mercapt group;
   (b) C₁₋₆ alkoxy group, C₁₋₆ alkylthio group, C₂₋₆ alkylcarbonyl group, C₂₋₆ alkylcarbonyloxy group, C₁₋₆ alkylsulfonyl group, C₁₋₆ alkylsulfinyl group, and C₂₋₆ alkoxycarbonyl group (wherein the group of this group may be substituted by one or more substituents independently selected from the group consisting of halogen atom, hydroxy group, carboxy group, C₁₋₆ alkoxy group, C₂₋₆ alkoxycarbonyl group, amino group optionally substituted by the same or different and one or two C₁₋₆ alkyl groups, carbamoyl group optionally substituted by the same or different and one or two C₁₋₆ alkyl groups, sulfamoyl group optionally substituted by the same or different and one or two C₁₋₆ alkyl groups, and C₁₋₆ alkylsulfonyl group.);
   (c) optionally substituted amino group, optionally substituted carbamoyl group and optionally substituted sulfamoyl group (wherein the group of this group may be substituted by one or more substituents selected from the group consisting of groups (j), (k) and (l) below), optionally substituted 3 to 8 membered cycloalkyl group and optionally substituted 4 to 8 membered saturated heterocyclic group (wherein the group of this group may be substituted by one or more substituents selected from the group consisting of groups (d), (e) and (f) below), and optionally substituted 6 to 10 membered aryl group, optionally substituted 5 to 10 membered heteroaryl group, optionally substituted 6 to 10 membered aryloxy group and optionally substituted 5 to 10 membered heteroaryloxy group (wherein the group of this group may be substituted by one or more substituents selected from the group consisting of groups (g), (h) and (i) below);
      substituted 3 to 8 membered cycloalkyl group and substituted 4 to 8 membered saturated heterocyclic group in R¹ and R³ is substituted by one or more substituents independently selected from the group consisting of groups (d) to (f) below;
   (d) halogen atom, hydroxy group, carboxy group, mercapto group, C₁₋₆ haloalkyl group and C₁₋₆ haloalkoxy group;
   (e) C₁₋₆ alkyl group, C₂₋₆ alkenyl group, C₂₋₆ alkynyl group, C₁₋₆ alkoxy group, C₂₋₆ alkylcarbonyl group, C₂₋₆ alkylcarbonyloxy group, C₂₋₆ alkoxycarbonyl group, C₁₋₆ alkylthio group, C₁₋₆ alkylsulfonyl group, and C₁₋₆ alkylsulfinyl group (wherein the group of this group may be substituted by one or more substituents independently selected from the group consisting of halogen atom, hydroxy group, carboxy group, C₁₋₆ alkoxy group, C₂₋₆ alkoxycarbonyl group, amino group optionally substituted by the same or different and one or two C₁₋₆ alkyl groups, carbamoyl group optionally substituted by the same or different and one or two C₁₋₆ alkyl groups, sulfamoyl group optionally substituted by the same or different and one or two C₁₋₆ alkyl groups, and C₁₋₆ alkylsulfonyl group.);
   (f) optionally substituted amino group, optionally substituted carbamoyl group and optionally substituted sulfamoyl group (wherein the group of this group may be substituted by one or two substituents selected from the group consisting of groups (j), (k) and (1) below), and optionally substituted 6 to 10 membered aryl group and optionally substituted 5 to 10 membered heteroaryl group (the group of this group may be substituted by one or more substituents selected from the group consisting of groups (g), (h) and (i) below);
      substituted aryl group and substituted heteroaryl group in R¹ and R³ are substituted by one or more substituents independently selected from the group consisting of groups (g) to (i) below;
   (g) halogen atom, hydroxy group, mercapto group, cyano group, nitro group, C₁₋₆ haloalkyl group, and C₁₋₆ haloalkoxy group;
   (h) C₁₋₆ alkyl group, C₂₋₆ alkenyl group, C₂₋₆ alkynyl group, C₁₋₆ alkoxy group, C₂₋₆ alkylcarbonyl group, C₂₋₆ alkylcarbonyloxy group, C₁₋₆ alkylthio group, C₁₋₆ alkylsulfonyl group, C₁₋₆ alkylsulfinyl group, 3 to 8 membered cycloalkyl group and 4 to 8 membered saturated heterocyclic group (wherein the group of this group may be substituted by one or more substituents independently selected from a group consisting of halogen atom, hydroxy group, C₁₋₆ alkyl group, C₁₋₆ alkoxy group, amino group optionally substituted by the same or different and one or two C₁₋₆ alkyl groups, carbamoyl group optionally substituted by the same or different and one or two C₁₋₆ alkyl groups, sulfamoyl group optionally substituted by the same or different and one or two C₁₋₆ alkyl groups, and C₁₋₆ alkylsulfonyl group.);
   (i) optionally substituted amino group, optionally substituted carbamoyl group, and optionally substituted sulfamoyl group (the group of this group may be substituted by one or two substituents selected from group consisting of groups (j), (k) and (l) below);
      substituted amino group, substituted carbamoyl group and substituted sulfamoyl group in the above groups (a) to (i) are substituted by one or two substituents independently selected from the group consisting of groups (j) to (l) below;
   (j) C₁₋₆ alkyl group, C₂₋₆ alkenyl group, C₂₋₆ alkynyl group, C₂₋₆ alkylcarbonyl group, C₂₋₆ alkoxycarbonyl group, C₁₋₆ alkylsulfonyl group, C₁₋₆ alkylsulfinyl group, 3 to 8 membered cycloalkyl group, 3 to 8 membered cycloalkylcarbonyl group, 3 to 8 membered cycloalkoxycarbonyl group, 3 to 8 membered cycloalkylsulfonyl group, 3 to 8 membered cycloalkylsulfinyl group (wherein the group of this group may be substituted by one or more substituents independently selected from the group consisting of halogen atom, hydroxy group, carboxy group, C₁₋₆ alkyl group, C₁₋₆ alkoxy group, C₂₋₆ alkoxycarbonyl group, amino group optionally substituted by the same or different and one or two alkyl groups, carbamoyl group optionally substituted by the same or different and one or two alkyl groups, sulfamoyl group optionally substituted by the same or different and one or two alkyl groups, and alkylsulfonyl group.);
   (k) 6 to 10 membered aryl group, 6 to 10 membered arylcarbonyl group, 6 to 10 membered aryloxycarbonyl group, 6 to 10 membered arylsulfonyl group, 6 to 10 membered arylsulfinyl group, 5 to 10 membered heteroaryl group, 5 to 10 membered heteroarylcarbonyl group, 5 to 10 membered heteroaryloxycarbonyl group, 5 to 10 membered heteroarylsulfonyl group, and 5 to 10 membered heteroarylsulfinyl group (wherein the group of this group may be substituted by halogen atom, hydroxy group, C₁₋₆ alkyl group, C₁₋₆ alkoxy group, amino group optionally substituted by the same or different and one or two C₁₋₆ alkyl groups, carbamoyl group optionally substituted by the same or different and one or two C₁₋₆ alkyl groups, sulfamoyl group optionally substituted by the same or different and one or two C₁₋₆ alkyl groups, or C₁₋₆ alkylsulfonyl group);
   (l) 4 to 7 membered saturated heterocyclic group containing 1 to 4 hetero atoms selected from 1 to 2 nitrogen atoms, 0 to 1 oxygen atom, and 0 to 1 sulfur atom which is formed by combining two substituents with the nitrogen atom (said saturated heterocyclic group may be substituted on appropriate carbon atom or nitrogen atom, if chemically stable, by halogen atom, hydroxy group, carboxy group, C₁₋₆ alkyl group, C₁₋₆ alkoxy group, C₂₋₆ alkoxycarbonyl group, C₂₋₆ alkylcarbonyl group, amino group which may be substituted by the same or different and one or two C₁₋₆ alkyl groups, carbamoyl group which may be substituted by the same or different and one or two C₁₋₆ alkyl groups, sulfamoyl group which may be substituted by the same or different and one or two C₁₋₆ alkyl groups, or C₁₋₆ alkylsulfonyl group.
[3] The adenine compound or its pharmaceutically acceptable salt described in the above [1] or [2], wherein in the formula (1), A¹ and A² are independently, optionally substituted benzene ring, or optionally substituted 5 to 6 membered aromatic heterocyclic group containing 1 to 3 hetero atoms selected from 0 to 3 nitrogen atoms, 0 to 1 oxygen atom and 0 to 1 sulfur atom.
[4] The adenine compound or its pharmaceutically acceptable salt described in any one of the above [1] to [3], wherein in the formula (1), R² is C₁₋₄ alkyl group.
[5] The adenine compound or its pharmaceutically acceptable salt described in the above [4], wherein in the formula (1), R² is methyl group.
[6] The adenine compound or its pharmaceutically acceptable salt described in any one of in the above [1] to [5] wherein in the formula (1), L¹ is C₁₋₄ alkylene.
[7] The adenine compound or its pharmaceutically acceptable salt described in any one of in the above [1] to [6] wherein in the formula (1), L⁴ is a single bond or C₁₋₄ alkylene.
[8] The adenine compound or its pharmaceutically acceptable salt described in [7] wherein in the formula (1), L⁴ is methylene.
[9] The adenine compound or its pharmaceutically acceptable salt described in any one of the above [1] to [8], wherein in the formula (1), -L²-NR³-L³- is a formula selected from the formulas (2)~(7):

   formula (2): -(O)ₚ-(CH₂)ₘ-NR³'-(CH₂)ₙ-(O)_{q}-

   wherein R³' is hydrogen atom; C₁₋₆ alkyl group; or C₂₋₆ alkyl group substituted by halogen atom, optionally substituted amino group, or hydroxy group, p and q are independently 0 or 1, and m and n are independently an integer of 1 to 4, provided that when p is 1, m is 2 or more, and when q is 1, n is 2 or more.; 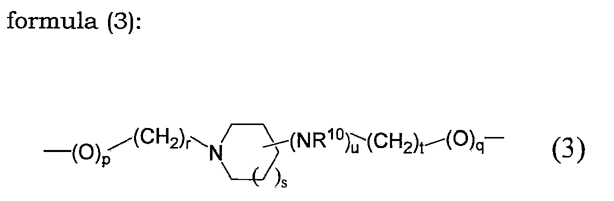 wherein R¹⁰ is hydrogen atom or C₁₋₆ alkyl group, p and q are the same as defined above, r and t are independently an integer of 0 to 4, s is an integer of 0 to 2, u is 0 or 1, provided that when p is 1, r is 2 or more, and when both u and q are 1, t is 2 or more; 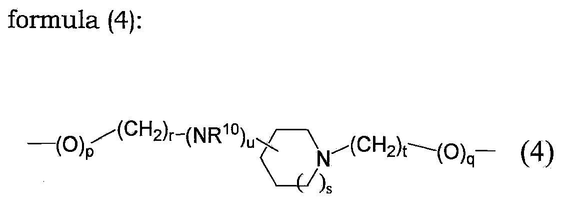 wherein R¹⁰, p and q are the same as defined above, r and t are independently an integer of 0 to 4, s is an integer of 0 to 2, u is 0 or 1, provided that when both p and u are 1, r is 2 or more, and when q is 1, t is 2 or more; 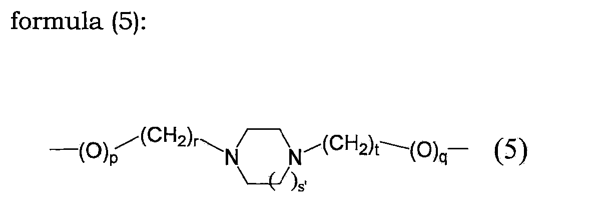 wherein p and q are the same as defined above, r and t are independently an integer of 0 to 4, s' is 1 or 2, provided that when p is 1, r is 2 or more, and when q is 1, t is 2 or more;

   formula (6): -≡-(CH₂)ᵥ-NR³'-(CH₂)ₙ-(O)_{q}- (6)

   wherein R^{3'}, q and n are the same as defined above, and v is 0~3 integers, provided that when q is 1, n is 2 or more;

   formula (7): -CO-NR³'-(CH₂)ₙ-(O)_{q}-

   wherein R^{3'}, q and n are the same as defined above, provided that when q is 1, n is 2 or more.
[10] The adenine compound or its pharmaceutically acceptable salt described in the above [1], which is represented by formula (8): 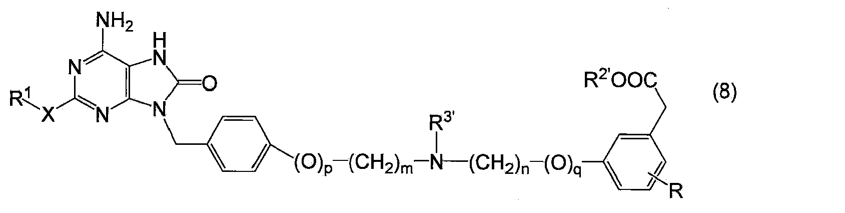 wherein R¹ and X is the same as defined in the formula (1), R is hydrogen atom, halogen atom, C₁₋₆ alkyl group or C₁₋₆ alkoxy group, R²' is hydrogen atom or methyl group, and R³', m, n, p and q are the same as the definition in the formula (2).
[11] The adenine compound or its pharmaceutically acceptable salt described in the above [1] selected from the group of the following compounds:
   2-Butoxy-7,8-dihydro-[(3-[{N-methyl-N-[3-(methoxycarbonylmethyl) benzyl]}amino] propoxy)benzyl]-8-oxoadenine;
   2-Butoxy-7,8-dihydro-[(3-[{N-[3-(carboxymethyl)benzyl]-N-methyl}amino]propoxy)benzyl]-8-oxoadenine;
   2-Butoxy-7,8-dihydro-9-([3-{N-[3-(methoxycarbonylmethyl)benzyl]amino}propoxy]benzyl)-8-oxoadenine;
   2-Butoxy-7,8-dihydro-9-([3-{N-[3-(carboxymethyl)benzyl]amino}propoxy]benzyl)-8-oxoadenine;
   2-Butoxy-7,8-dihydro-9-(4-{N-(3-hydroxypropyl)-N-[3-(methoxycarbonylmethyl)benzyl]aminomethyl}benzyl)-8-oxoadenine);
   2-Butoxy-9-(4-{N-(3-chloropropyl)-N-[3-(methoxycarbonylmethyl) benzyl]aminomethyl}benzyl)-7,8-dihydro-8-oxoadenine;
   2-Butoxy-7,8-dihydro-9-(4-{N-[3-(methoxycarbonylmethyl)benzyl]-N-(3-morpholin-4-ylpropyl)aminomethyl}benzyl)-8-oxoadenine;
   2-Butoxy-7,8-dihydro-9-(4-{N-[3-(hydroxycarbonylmethyl)benzyl]-N-(3-morpholin-4-ylpropyl)aminomethyl}benzyl)-8-oxoadenine;
   2-Butoxy-7,8-dihydro-9-[4-(N-{2-[3-(methoxycarbonylmethyl)phenoxy]ethyl}-N-methylaminomethyl)benzyl]-8-oxoadenine;
   2-Butoxy-7,8-dihydro-9-[4-(N-{2-[3-(hydoxycarbonylmethyl)phenoxy]ethyl}-N-methylaminomethyl)benzyl]-8-oxoadenine;
   2-Butoxy-7,8-dihydro-9-[4-(N-{2-[2-methoxy-5-(methoxycarbonylmethyl)phenoxy]ethyl}-N-methylaminomethyl)benzyl]-8-oxoadenine;
   2-Butoxy-7,8-dihydro-9-{6-[4-(3-methoxycarbonylmethylbenzyl)aminobutoxy]pyridin-3-ylmethyl}-8-oxoadenine;
   2-Butoxy-7,8-dihydro-9-(6-{3-[N-methyl-N-(3-methoxycarbonylmethyl)phenyloxyethyl]aminopropoxy}pyridin-3-ylmethyl)-8-oxoadenine;
   2-Butoxy-7,8-dihydro-9-(6-{3-[N-methyl-N-(3-hydroxycarbonylmethyl)phenyloxyethyl]aminopropoxy}pyridin-3-ylmethyl)-8-oxoadenine;
   2-Butoxy-7,8-dihydro-9-[6-(4-[{N-methyl-N-[3-(methoxycarbonylmethyl)benzyl]}amino]butoxy)pyridin-3-ylmethyl]-8-oxoadenine;
   2-Butoxy-7,8-dihydro-9-[6-(4-[{N-[3-(carboxymethyl)benzyl-N-methyl]}amino]butoxy)pyridin-3-ylmethyl]-8-oxoadenine;
   2-Butoxy-7,8-dihydro-9-(6-[4-{[N-(3-methoxycarbonylmethylbenzyl)-N-(3-morpholonopropyl)]amino}butoxy]pyridin-3-ylmethyl)-8-oxoadenine;
   2-Butoxy-7,8-dihydro-9-(6-{4-[N-(3-hydroxycarbonylmethylbenzyl)-N-(3-morpholinopropyl)] aminobutoxy}pyridin-3-ylmethyl)-8-oxoadenine;
   7,8-Dihydro-9-[4-{N-[2-(3-methoxycarbonylmethylphenyl-1-yl)oxyethyl]-N-methylamino}methylbenzyl]-2-(2-methoxyethoxy)-8-oxoadenine;
   7, 8-Dihydro-9-[4-{N-[2-(3-hydroxycarbonylmethylphenyl-1-yl)oxyethyl]-N-methylamino}methylbenzyl]-2-(2-methoxyethoxy)-8-oxoadenine;
   2-Butoxy-7,8-dihydro-9-(4-{4-[3-(methoxycarbonylmethyl) phenoxymethyl] piperidin-1-ylmethyl}benzyl)-8-oxoadenine;
   2-Butoxy-7,8-dihydro-9-(4-{4-[3-(carboxymethyl)phenoxymethyl]piperidin-1-ylmethyl}benzyl)-8-oxoadenine;
   2-Butoxy-7,8-dihydro-9-[4-(N-{2-[1-methoxy-5-(methoxycarbonylmethyl)phenoxy]ethyl}aminomethyl)benzyl]-8-oxoadenine;
   2-Butoxy-7,8-dihydro-9-[4-(N-{2-[1-methoxy-5-(carboxymethyl)phenoxy]ethyl}aminomethyl)benzyl]-8-oxoadenine;
   7, 8-Dihydro-9-(4-{4-[3-(methoxycarbonylmethyl)phenoxy]piperidin-1-ylmethyl}benzyl)-2-(2-methoxyethoxy)-8-oxoadenine;
   7,8-Dihydro-9-(4-{4-[3-(carboxymethyl)phenoxy]piperidinylmethyl}benzyl)-2-(2-methoxyethoxy)-8-oxoadenine;
   2-Butoxy-7,8-dihydro-9-(3-methoxycarbonyl-4-{N-[3-(niethoxycarbonylmethyl)benzyl]-N-methylaminonomethylpropargyl}benzyl)-8-oxoadenine;
   2-Butoxy-7,8-dihydro-9-[3-fluoro-4-{4-[3-(methoxycarbonylmethyl) phenoxymethyl] piperidin-1-yl}methylbenzyl]-8-oxoadenine;
   2-Butoxy-7, 8-dihydro-9-[3-fluoro-4-{4-[3-(carboxymethyl)phenoxymethyl] piperidin-1-yl}methylbenzyl] -8-oxoadenine;
   [3-(4-{[4-(6-Amino-2-butoxy-8-oxo-7,8-dihydropurin-9-ylmethyl)benzyl]ethylamino}butoxy)phenyl]acetic acid methyl ester;
   [3-(3-{4-[4-(6-Amino-2-butoxy-8-oxo-7, 8-dihydropurin-9-ylmethyl)benzyl]piperazin-1-yl}propoxy)phenyl]acetic acid methyl ester;
   [3-(2-{4-[5-(6-Amino-2-butoxy-8-oxo-7, 8-dihydropurin-9-ylmethyl)pyridin-2-yl]-[1,4]diazepan-1-yl}ethoxy)phenyl]acetic acid methyl ester;
   [3-(2-{4-[4-(6-Amino-2-butoxy-8-oxo-7, 8-dihydropurin-9-ylmethyl)-2-nitrophenyl]-[1,4]diazepan-1-yl}ethoxy)phenyl]acetic acid methyl ester;
   [3-(2-{4-[2-Amino-4-(6-amino-2-butoxy-8-oxo-7,8-dihydropurin-9-ylmethyl)phenyl]-[1,4]diazepan-1-yl}ethoxy)phenyl]acetic acid methyl ester;
   (3-{2-[(1-{4-[(6-Amino-2-butoxy-8-oxo-7,8-dihydro-9H-purin-9-yl)methyl]benzyl}piperidin-4-yl)(methyl)amino]ethoxy}phenyl)acetic acid methyl ester;
   (3-{2-[{2-[{4-[(6-Amino-2-butoxy-8-oxo-7,8-dihydro-9H-purin-9-yl)methyl]benzyl}(methyl)amino]ethyl}(methyl)amino]ethoxy}phenyl)acetic acid methyl ester;
   (3-{2-[4-{4-[(6-Amino-2-butoxy-8-oxo-7,8-dihydro-9H-puhn-9-yl)methyl]benzyl}(methyl)aminopiperidin-1-yl]ethoxy}phenyl)acetic acid methyl ester;
   (3-{2-[{[1-(4-{[6-Amino-2-(2-methoxyethoxy)-8-oxo-7,8-dihydro-9H-purin-9-yl] methyl}benzyl)-4-hydroxypiperidin-4-yl]methyl}(methyl)amino]ethoxy}phenyl)acetic acid methyl ester;
   (3-{2-[9-(4-{[6-Amino-2-(2-methoxyethoxy)-8-oxo-7,8-dihydro-9H-purin-9-yl] methyl}benzyl)-1-oxa-4,9-diazaspiro[5.5]undec-4-yl] ethoxy}phenyl)acetic acid methyl ester;
   {3-[({3-[(4-{[6-Amino-2-(2-methoxyethoxy)-8-oxo-7,8-dihydro-9H-purin-9-yl]methyl}benzyl)(methyl)amino]propyl}amino)methyl]phenyl}acetic acid methyl ester;
   (3-{2-[(3-{[5-(6-Amino-2-butoxy-8-oxo-7,8-dihydropurin-9-ylmethyl)pyridin-2-yl]methylamino}propyl)methylamino]ethoxy}phenyl)acetic acid methyl ester;
   (3-{2-[(3-{[5-(6-Amino-2-butoxy-8-oxo-7,8-dihydropurin-9-ylmethyl)pyridin-2-yl]methylamino}propyl)methylamino]ethoxy}phenyl)acetic acid;
   7,8-Dihydro-2-methoxyethylamino-9-(4-[4-{2-[3-(methoxycarbonylmethyl)phenoxy]ethyl}piperazinylmethyl]benzyl)-8-oxoadenine; and
   7,8-Dihydro-2-(4-pyridylmethylamino)-9-(4-[N-methyl-N-{4-[3-(methoxycarbonylmethyl)phenoxy]butyl}aminomethyl]benzyl)-8-oxoadenine.
[12] A pharmaceutical composition containing the adenine compound or a pharmaceutically acceptable salt thereof as described in any one of the above [1] to [11] as an active ingredient.
[13] A TLR7 activator containing the adenine compound or a pharmaceutically acceptable salt thereof as described in any one of the above [1] to [11] as an active ingredient.
[14] An immuno-modifier containing the adenine compound or a pharmaceutically acceptable salt thereof as described in any one of the above [1] to [11] as an active ingredient.
[15] A therapeutic or prophylactic agent for allergic diseases, viral diseases or cancers containing the adenine compound or a pharmaceutically acceptable salt thereof as described in any one of the above [1] to [11] as an active ingredient.
[16] A therapeutic or prophylactic agent for asthma, COPD, allergic rhinitis, allergic conjunctivitis, atopic dermatosis, cancer, hepatitis B virus, hepatitis C virus, HIV, HPV, a bacterial infectious disease, or dermatosis containing the adenine compound or a pharmaceutically acceptable salt thereof as described in any one of the above [1] to [11] as an active ingredient.
[17] A medicament for topical administration containing the adenine compound or a pharmaceutically acceptable salt thereof as described in any one of the above [1]to [11] as an active ingredient.

### EFFECT OF THE INVENTION

According to the present invention it is possible to provide a novel adenine compound useful as a prophylactic or therapeutic agent for allergic diseases, viral diseases, cancers, etc.

### THE BEST MODE FOR CARRYING OUT THE INVENTION

The present invention is described in detail below.

"Halogen atom" in the present specification includes fluorine atom, chlorine atom, bromine atom, or iodine atom, preferably fluorine atom or chlorine atom.

"Alkyl group" includes C₁₋₁₂ straight or branched chain alkyl group, such as methyl group, ethyl group, propyl group, 1-methylethyl group, butyl group, 2-methylpropyl group, 1-methylpropyl group, 1,1-dimethylethyl group, pentyl group, 3-methylbutyl group, 2-methylbutyl group, 2,2-dimethylpropyl group, 1-ethylpropyl group, 1,1-dimethylpropyl group, hexyl group, 4-methylpentyl group, 3-methylpentyl group, 2-methylpentyl group, 1-methylpentyl group, 3,3-dimethylbutyl group, 2,2-dimethylbutyl group, 1,1-dimethylbutyl group, 1,2-dimethylbutyl group, heptyl group, 1-methylhexyl group, 1-ethylpentyl group, octyl group, 1-methylheptyl group, 2-ethylhexyl group, nonyl group, decyl group, etc., preferably C₁₋₆ alkyl group, more preferably C₁₋₄ alkyl group.

"Alkenyl group" includes C₂₋₁₀ straight or branched chain alkenyl group, such as ethenyl group, propenyl group, 1-methylethenyl group, butenyl group, 2-methylpropenyl group, 1-methylpropenyl group, pentenyl group, 3-methylbutenyl group, 2-methylbutenyl group, 1-ethylpropenyl group, hexenyl group, 4-methylpentenyl group, 3-methylpentenyl group, 2-methylpentenyl group, 1-methylpentenyl group, 3,3-dimethylbutenyl group, 1,2-dimethylbutenyl group, heptenyl group, 1-methylhexenyl group, 1-ethylpentenyl group, octenyl group, 1-methylheptenyl group, 2-ethylhexenyl group, nonenyl group, decenyl group, etc., preferably C₂₋₆ alkenyl group, more preferably C₂₋₄ alkenyl group.

"Alkynyl group" includes C₁₋₁₀ straight or branched chain alkynyl group, such as ethynyl group, propynyl group, butynyl group, pentynyl group, 3-methylbutynyl group, hexynyl group, 4-methylpentynyl group, 3-methylpentynyl group, 3,3-dimethylbutynyl group, heptynyl group, octynyl group, 3-methylheptynyl group, 3-ethylhexynyl group, nonynyl group, decynyl group, etc., preferably C₂₋₆ alkynyl group, more preferably, C₂₋₄ alkynyl group.

"Cycloalkyl group" includes 3 to 8 membered monocyclic cycloalkyl group, such as cyclopropyl group, cyclobutyl group, cyclopentyl group, cyclohexyl group, cycloheptyl group, or cyclooctyl group.

"Cycloalkoxy group" includes 3 to 8 membered monocyclic cycloalkoxy group, such as cyclopropyloxy group, cyclobutyloxy group, cyclopentyloxy group, cyclohexyloxy group, cycloheptyloxy group, or cyclooctyloxy group.

"Aryl group" includes 6 to 10 membered mono or bicyclic aryl group, such as phenyl group, 1-naphthyl group, or 2-naphthyl group.

"Heteroaryl group" includes 5 to 10 membered monocyclic or bicyclic heteroaryl group containing 1 to 4 hetero atoms selected from 0 to 2 nitrogen atoms, 0 to 1 oxygen atom and 0 to 1 sulfur atom, such as furyl group, thienyl group, pyrrolyl group, pyridyl group, indolyl group, isoindolyl group, quinolyl group, isoquinolyl group, pyrazolyl group, imidazolyl group, pyrimidinyl group, pyrazinyl group, pyridazinyl group, thiazolyl group, oxazolyl group, etc. The binding position in the heteroaryl group is not specifically limited, if chemically stable.

"Saturated heterocyclic group" includes 4 to 10 membered, preferably 4 to 6 membered mono or bicyclic saturated heterocyclic group containing 1 to 3 hetero atoms selected from 0 to 3 nitrogen atoms, 0 to 1 oxygen atom and 0 to 1 sulfur atom such as pyrrolidinyl group, piperidinyl group, piperazinyl group, morpholinyl group, thiomorpholinyl group, 1-oxothiomorpholinyl group, 1,1-dioxothiomorpholinyl group, tetrahydrofuranyl group, etc. The binding position on the heterocyclic group is not specifically limited and it may be on any of nitrogen or carbon atoms, if chemically stable.

"Alkylene" includes straight or branched chain C₁₋₁₂ alkylene, such as methylene, ethylene, trimethylene, tetramethylene, pentamethylene, hexamethylene, heptamethylene, octamethylene, nonamethylene, decamethylene, 1-methylmethylene, 1-ethylmethylene, 1-propylmethylene, 1-methylethylene, 2-methylethylene, 1-methyltrimethylene, 2-methyltrimethylene, 2-methyltetramethylene, 3-methylpentamethylene, etc., preferably C₁₋₆ alkylene.

"Alkenylene" includes straight or branched chain C₂₋₁₂ alkenylene, such as vinylene, propenylene, 1-butenylene, 2-butenylene, 1-pentenylene, 2-pentenylene, 1-hexenylene, 1-heptenylene, etc., preferably C₂₋₆ alkenylene.

"Alkynylene" includes straight or branched chain C₂₋₁₂ alkynylene, such as ethynylene, propynylene, 1-butynylene, 2-butynylene, 1-pentynylene, 2-pentynylene, 1-hexynylene, 1-heptynylene, etc., preferably C₂₋₆ alkynylene.

"Haloalkyl group" includes C₁₋₆ alkyl group substituted by the same or different and 1 to 5 halogen atoms, such as trifluoromethyl group, 2,2,2-trifluoroethyl group, 2,2-difluoroethyl group, pentafluoroethyl group, etc.

"Alkoxy group" includes C₁₋₁₀ straight or branched chain alkoxy group, for example methoxy group, ethoxy group, propoxy group, 1-methylethoxy group, butoxy group, 2-methylpropoxy group, 1-methylpropoxy group, 1,1-dimethylethoxy group, pentoxy group, 3-methylbutoxy group, 2-methylbutoxy group, 2,2-dimethylpropoxy group, 1-ethylpropoxy group, 1,1-dimethylprogoxy group, hexyloxy group, 4-methylpentyloxy group, 3-methylpentyloxy group, 2-methylpentyloxy group, 1-methylpentyloxy group, 3,3-dimethylbutoxy group, 2,2-dimethylbutoxy group, 1,1-dimethylbutoxy group, 1,2-dimethylbutoxy group, heptyloxy group, 1-methylhexyloxy group, 1-ethylpentyloxy group, octyloxy group, 1-methylheptyloxy group, 2-ethylhexyloxy group, nonyloxy group, decyloxy group, etc, preferably C₁₋₆ alkoxy group, more preferably C₁₋₄ alkoxy group.

"Haloalkoxy group" included C₁₋₆ alkoxy group substituted by the same or different and 1 to 5 halogen atoms, such as trifluoromethoxy group, 2,2,2-trifluoroethoxy group, 2,2-difluoroethoxy group, 2-fluoroethoxy, pentafluoroethoxy group, etc.

"Alkylthio group" includes straight or branched chain C₁₋₁₀ alkylthio group, such as methylthio group, ethylthio group, propylthio group, 1-methylethylthio group, butylthio group, 2-methylpropylthio group, 1-methylpropylthio group, 1,1-dimethylethylthio group, pentylthio group, 3-methylbutylthio group, 2-methylbutylthio group, 2,2-dimethylpropylthio group, 1-ethylpropylthio group, 1,1-dimethylpropylthio group, hexylthio group, 4-methylpentylthio group, 3-methylpentylthio group, 2-methylpentylthio group, 1-methylpentylthio group, 3,3-dimethylbutylthio group, 2,2-dimethylbutylthio group, 1,1-dimethylbutylthio group, 1,2-dimethylbutylthio group, heptylthio group, 1-methylhexylthio group, 1-ethylpentylthio group, octylthio group, 1-methylheptylthio group, 2-ethylhexylthio group, nonylthio group, decylthio group, etc., preferably C₁₋₆ alkylthio group, more preferably C₁₋₄ alkylthio group.

"Alkyl moiety" in "alkylcarbonyl group", "alkylcarbonyloxy group", "alkylsulfonyl group" or "alkylsulfinyl group" includes the same as the alkyl group as mentioned above.

"Alkylcarbonyl group" includes such as acetyl group, propanoyl group, butanoyl group, 2-methylpropanoyl group, pentanoyl group, 3-methylbutanoyl group, 2-methylbutanoyl group, 2,2-dimethylpropanoyl (pivaloyl) group, hexanoyl group, 4-methylpentanoyl group, 3-methylpentanoyl group, 2-methylpentanoyl group, 3,3-dimethylbutanoyl group, 2,2-dimethylbutanoyl group, heptanoyl group, octanoyl group, 2-ethylhexanoyl group, nonanoyl group, decanoyl group, etc., preferably C₂₋₆ alkylcarbonyl group, more preferably, straight or branched chain C₂₋₅ alkylcarbonyl group.

"Alkylcarbonyloxy group" includes such as acetoxy group, propanoyloxy group, butanoyloxy group, 2-methylpropanoyloxy group, pentanoyloxy group, 3-methylbutanoyloxy group, 2-methylbutanoyloxy group, 2,2-dimethylpropanoyloxy (pivaloyloxy) group, hexanoyloxy group, 4-methylpentanoyloxy group, 3-methylpentanoyloxy group, 2-methylpentanoyloxy group, 3,3-dimethylbutanoyloxy group, 2,2-dimethylbutanoyloxy group, heptanoyloxy group, octanoyloxy group, 2-ethylhexanoyloxy group, nonanoyloxy group, decanoyloxy group, etc., preferably C₂₋₆ alkylcarbonyloxy group, more preferably straight or branched chain C₂₋₅ alkylcarbonyloxy group.

"Alkylsulfonyl group" includes such as methanesulfonyl group, ethanesulfonyl group, propylsulfonyl group, 1-methylethylsulfonyl group, butylsulfonyl group, 2-methylpropylsulfonyl group, 1-methylpropylsulfonyl group, 1,1-dimethylethylsulfonyl group, pentylsulfonyl group, 3-methylbutylsulfonyl group, 2-methylbutylsulfonyl group, 2,2-dimethylpropylsulfonyl group, 1-ethylpropylsulfonyl group, 1,1-dimethylpropylsulfonyl group, hexylsulfonyl group, 4-methylpentylsulfonyl group, 3-methylpentylsulfonyl group, 2-methylpentylsulfonyl group, 1-methylpentylsulfonyl group, 3,3-dimethylbutylsulfonyl group, 2,2-dimethylbutylsulfonyl group, 1,1-dimethylbutylsulfonyl group, 1,2-dimethylbutylsulfonyl group, heptylsulfonyl group, 1-methylhexylsulfonyl group, 1-ethylpentylsulfonyl group, octylsulfonyl group, 1-methylheptylsulfonyl group, 2-ethylhexylsulfonyl group, nonylsulfonyl group, decylsulfonyl group, etc., preferably C₁₋₆ alkylsulfonyl group, more preferably straight or branched chain C₁₋₄ alkylsulfonyl group.

"Alkylsulfinyl group" includes such as methylsulfinyl group, ethylsulfinyl group, propylsulfinyl group, 1-methylethylsulfinyl group, butylsulfinyl group, 2-methylpropylsulfinyl group, 1-methylpropylsulfinyl group, 1,1-dimethylethylsulfinyl group, pentylsulfinyl group, 3-methylbutylsulfinyl group, 2-methylbutylsulfinyl group, 2,2-dimethylpropylsulfinyl group, 1-ethylpropylsulfinyl group, 1,1-dimethylpropylsulfinyl group, hexylsulfinyl group, 4-methylpentylsulfinyl group, 3-methylpentylsulfinyl group, 2-methylpentylsulfinyl group, 1-methylpentylsulfinyl group, 3,3-dimethylbutylsulfinyl group, 2,2-dimethylbutylsulfinyl group, 1,1-dimethylbutylsulfinyl group, 1,2-dimethylbutylsulfinyl group, heptylsulfinyl group, 1-methylhexylsulfinyl group, 1-ethylpentylsulfinyl group, octylsulfinyl group, 1-methylheptylsulfinyl group, 2-ethylhexylsulfinyl group, nonylsulfinyl group, decylsulfinyl group, etc., preferably C₁₋₆ alkylsulfinyl group, more preferably straight or branched chain C₁₋₄ alkylsulfinyl group.

"Alkoxy moiety" in "alkoxycarbonyl group" is the same as the alkoxy group mentioned above. Examples of "alkoxycarbonyl group" are methoxycarbonyl group, ethoxycarbonyl group, propoxycarbonyl group, 1-methylethoxycarbonyl group, butoxycarbonyl group, 2-methylpropoxycarbonyl group, 1-methylpropoxycarbonyl group, 1,1-dimethylethoxycarbonyl group, pentoxycarbonyl group, 3-methylbutoxycarbonyl group, 2-methylbutoxycarbonyl group, 2,2-dimethylpropoxycarbonyl group, 1-ethylpropoxycarbonyl group, 1,1-dimethylpropoxycarbonyl group, hexyloxycarbonyl group, 4-methylpentyloxycarbonyl group, 3-methylpentyloxycarbonyl group, 2-methylpentyloxycarbonyl group, 1-methylpentyloxycarbonyl group, 3,3-dimethylbutoxycarbonyl group, 2,2-dimethylbutoxycarbonyl group, 1,1-dimethylbutoxycarbonyl group, 1,2-dimethylbutoxycarbonyl group, heptyloxycarbonyl group, 1-methylhexyloxycarbonyl group, 1-ethylpentyloxycarbonyl group, octyloxycarbonyl group, 1-methylheptyloxycarbonyl group, 2-ethylhexyloxycarbonyl group, nonyloxycarbonyl group, decyloxycarbonyl group, etc., preferably C₂₋₆ alkoxycarbonyl group, more preferably straight or branched chain C₂₋₄ alkoxycarbonyl group.

"Alkenyl moiety" in "alkenyloxy group", "alkenylcarbonyl group", "alkenylcarbonyloxy group", "alkenylsulfonyl group", "alkenylsulfinyl group" and "alkenyloxycarbonyl group" is the same as the alkenyl group mentioned above.

"Alkenyloxy group" includes for example, ethenyloxy group, propenyloxy group, 1-methylethenyloxy group, butenyloxy group, 2-methylpropenyloxy group, 1-methylpropenyloxy group, pentenyloxy group, 3-methylbutenyloxy group, 2-methylbutenyloxy group, 1-ethylpropenyloxy group, hexenyloxy group, 4-methylpentenyloxy group, 3-methylpentenyloxy group, 2-methylpentenyloxy group, 1-methylpentenyloxy group, 3,3-dimethylbutenyloxy group, 1, 2-dimethylbutenyloxy group, heptenyloxy group, 1-methylhexenyloxy group, 1-ethylpentenyloxy group, octenyloxy group, 1-methylheptenyloxy group, 2-ethylhexenyloxy group, nonenyloxy group, decenyloxy group, etc., preferably C₂₋₆, more preferably C₂₋₅ alkenyloxy group.

"Alkenylcarbonyl group" includes such as, ethenylcarbonyl group, propenylcarbonyl group, 1-methylethenylcarbonyl group, butenylcarbonyl group, 2-methylpropenylcarbonyl group, 1-methylpropenylcarbonyl group, pentenylcarbonyl group, 3-methylbutenylcarbonyl group, 2-methylbutenylcarbonyl group, 1-ethylpropenylcarbonyl group, hexenylcarbonyl group, 4-methylpentenylcarbonyl group, 3-methylpentenylcarbonyl group, 2-methylpentenylcarbonyl group, 1-methylpentenylcarbonyl group, 3,3-dimethylbutenylcarbonyl group, 1,2-dimethylbutenylcarbonyl group, heptenylcarbonyl group, 1-methylhexenylcarbonyl group, 1-ethylpentenylcarbonyl group, octenylcarbonyl group, 1-methylheptenylcarbonyl group, 2-ethylhexenylcarbonyl group, nonenylcarbonyl group, decenylcarbonyl group, etc., preferably C₃₋₆, and more preferably C₃₋₅ alkenylcarbonyl group.

"Alkenylcarbonyloxy group" includes one constituted by binding an oxygen atom to carbonyl moiety of alkenylcarbonyl group, preferably, C₃₋₆, and more preferably C₃₋₅ alkenylcarbonyloxy group.

"Alkenylsulfonyl group" includes such as ethenylsulfonyl group, propenylsulfonyl group, 1-methylethenylsulfonyl group, butenylsulfonyl group, 2-methylpropenylsulfonyl group, 1-methylpropenylsulfonyl group, pentenylsulfonyl group, 3-methylbutenylsulfonyl group, 2-methylbutenylsulfonyl group, 1-ethylpropenylsulfonyl group, hexenylsulfonyl group, 4-methylpentenylsulfonyl group, 3-methylpentenylsulfonyl group, 2-methylpentenylsulfonyl group, 1-methylpentenylsulfonyl group, 3,3-dimethylbutenylsulfonyl group, 1,2-dimethylbutenylsulfonyl group, heptenylsulfonyl group, 1-methylhexenylsulfonyl group, 1-ethylpentenylsulfonyl group, octenylsulfonyl group, 1-methylheptenylsulfonyl group, 2-ethylhexenylsulfonyl group, nonenylsulfonyl group, decenylsulfonyl group, etc., more preferably C₂₋₆, more preferably C₂₋₅ alkenylsulfonyl group.

"Alkenylsulfinyl group" includes such as ethenylsulfinyl group, propenylsulfinyl group, 1-methylethenylsulfinyl group, butenylsulfinyl group, 2-methylpropenylsulfinyl group, 1-methylpropenylsulfinyl group, pentenylsulfinyl group, 3-methylbutenylsulfinyl group, 2-methylbutenylsulfinyl group, 1-ethylpropenylsulfinyl group, hexenylsulfinyl group, 4-methylpentenylsulfinyl group, 3-methylpentenylsulfinyl group, 2-methylpentenylsulfinyl group, 1-methylpentenylsulfinyl group, 3,3-dimethylbutenylsulfinyl group, 1,2-dimethylbutenylsulfinyl group, heptenylsulfinyl group, 1-methylhexenylsulfinyl group, 1-ethylpentenylsulfinyl group, octenylsulfinyl group, 1-methylheptenylsulfinyl group, 2-ethylhexenylsulfinyl group, nonenylsulfinyl group, decenylsulfinyl group, etc., preferably C₂₋₆, more preferably C₂₋₅ alkenylsulfinyl group.

"Alkenyloxycarbonyl group" includes such as ethenyloxycarbonyl group, propenyloxycarbonyl group, 1-methylethenyloxycarbonyl group, butenyloxycarbonyl group, 2-methylpropenyloxycarbonyl group, 1-methylpropenyloxycarbonyl group, pentenyloxycarbonyl group, 3-methylbutenyloxycarbonyl group, 2-methylbutenyloxycarbonyl group, 1-ethylpropenyloxycarbonyl group, hexenyloxycarbonyl group, 4-methylpentenyloxycarbonyl group, 3-methylpentenyloxycarbonyl group, 2-methylpentenyloxycarbonyl group, 1-methylpentenyloxycarbonyl group, 3,3-dimethylbutenyloxycarbonyl group, 1,2-dimethylbutenyloxycarbonyl group, heptenyloxycarbonyl group, 1-methylhexenyloxycarbonyl group, 1-ethylpentenyloxycarbonyl group, octenyloxycarbonyl group, 1-methylheptenyloxycarbonyl group, 2-ethylhexenyloxycarbonyl group, nonenyloxycarbonyl group, decenyloxycarbonyl group, etc., preferably C₃₋₆, and more preferably C₃₋₅ alkenyloxycarbonyl group.

"Alkynyl moiety" in "alkynyloxy group", "alkynylcarbonyl group", "alkynylcarbonyloxy group", "alkynylsulfonyl group", "alkynylsulfinyl group" and "alkynyloxycarbonyl group" is the same as the alkynyl group as mentioned above.

"Alkynyloxy group" includes such as ethynyloxy group, propynyloxy group, butynyloxy group, pentynyloxy group, 3-methylbutynyloxy group, hexynyloxy group, 4-methylpentynyloxy group, 3-methylpentynyloxy group, 3,3-dimethylbutynyloxy group, heptynyloxy group, octynyloxy group, 3-methylheptynyloxy group, 3-ethylhexynyloxy group, nonynyloxy group, decynyloxy group, etc., preferably C₂₋₆ and more preferably C₂₋₅ alkynyloxy group.

"Alkynylcarbonyl group" includes such as ethynylcarbonyl group, propynylcarbonyl group, butynylcarbonyl group, pentynylcarbonyl group, 3-methylbutynylcarbonyl group, hexynylcarbonyl group, 4-methylpentynylcarbonyl group, 3-methylpentynylcarbonyl group, 3,3-dimethylbutynylcarbonyl group, heptynylcarbonyl group, octynylcarbonyl group, 3-methylheptynylcarbonyl group, 3-ethylhexynylcarbonyl group, nonynylcarbonyl group, decynylcarbonyl group, etc., preferably C₃₋₆, more preferably C₃₋₅ alkynylcarbonyl group.

"Alkynylcarbonyloxy group" includes for example, one constituted by combining an oxygen atom to carbonyl moiety of the above "alkynylcarbonyl group". Preferably C₃₋₆, and more preferably C₃₋₅ alkynylcarbonyloxy groups are illustrated.

"Alkynylsulfonyl group", includes, for example ethynylsulfonyl group, propynylsulfonyl group, butynylsulfonyl group, pentynylsulfonyl group, 3-methylbutynylsulfonyl group, hexynylsulfonyl group, 4-methylpentynylsulfonyl group, 3-methylpentynylsulfonyl group, 3,3-dimethylbutynylsulfonyl group, heptynylsulfonyl group, octynylsulfonyl group, 3-methylheptynylsulfonyl group, 3-ethylhexynylsulfonyl group, nonynylsulfonyl group, or decynylsulfonyl group, preferably C₂₋₆, more preferably C₂₋₅ alkynylsulfonyl group.

"Alkynylsulfinyl group", the following groups includes, for example ethynylsulfinyl group, propynylsulfinyl group, butynylsulfinyl group, pentynylsulfinyl group, 3-methylbutynylsulfinyl group, hexynylsulfinyl group, 4-methylpentynylsulfinyl group, 3-methylpentynylsulfinyl group, 3,3-dimethylbutynylsulfinyl group, heptynylsulfinyl group, octynylsulfinyl group, 3-methylheptynylsulfinyl group, 3-ethylhexynylsulfinyl group, nonylsulfinyl group, or decynylsulfinyl group, preferably C₂₋₆, more preferably C₂₋₅ alkynylsulfinyl group.

As "alkynyloxycarbonyl group ", the following groups are illustrated; ethynyloxycarbonyl group, propynyloxycarbonyl group, butynyloxycarbonyl group, pentynyloxycarbonyl group, 3-methylbutynyloxycarbonyl group, hexynyloxycarbonyl group, 4-methylpentynyloxycarbonyl group, 3-methylpentynyloxycarbonyl group, 3,3-dimethylbutynyloxycarbonyl group, heptynyloxycarbonyl group, octynyloxycarbonyl group, 3-methylheptynyloxycarbonyl group, 3-ethylhexynyloxycarbonyl group, nonynyloxycarbonyl group, or decynyloxycarbonyl group, preferably C₃₋₆, more preferably C₃₋₅ alkynyloxycarbonyl group.

As "cycloalkyl" in "cycloalkylcarbonyl group", "cycloalkylcarbonyloxy group", "cycloalkylsulfonyl group" and "cycloalkylsulfinyl group", the same groups as the above cycloalkyl groups are illustrated.

As "cycloalkylcarbonyl group", the following groups are illustrated; cyclopropylcarbonyl group, cyclobutylcarbonyl group, cyclopentylcarbonyl group, cyclohexylcarbonyl group, cycloheptylcarbonyl group, or cyclooctylcarbonyl group.

As "cycloalkylcarbonyloxy group", one constituted by binding an oxygen atom to carbonyl moiety of "cycloalkylcarbonyl group" are illustrated. For example cyclopropylcarbonyloxy group, cyclobutylcarbonyloxy group, cyclopentylcarbonyloxy group, cyclohexylcarbonyloxy group, cycloheptylcarbonyloxy group, or cyclooctylcarbonyloxy group are illustrated.

As "cycloalkylsulfonyl group", the following groups are illustrated; cyclopropylsulfonyl group, cyclobutylsulfonyl group, cyclopentylsulfonyl group, cyclohexylsulfonyl group, cycloheptylsulfonyl group, or cyclooctylsulfonyl group.

As "cycloalkylsulfinyl group", the following groups are illustrated; cyclopropylsulfinyl group, cyclobutylsulfinyl group, cyclopentylsulfinyl group, cyclohexylsulfinyl group, cycloheptylsulfinyl group or cyclooctylsulfinyl group.

As "cycloalkoxy" in "cycloalkoxycarbonyl group", the same as the above cycloalkoxy group is illustrated. For example, cyclopropyloxycarbonyl group, cyclobutyloxycarbonyl group, cyclopentyloxycarbonyl group, cyclohexyloxycarbonyl group, cycloheptyloxycarbonyl group, or cyclooctyloxycarbonyl group is illustrated.

As aryl in "aryloxy group", "arylcarbonyl group", "aryloxycarbonyl group", "arylcarbonyloxy group", "arylsulfonyl group" and "arylsulfinyl group", the same as the above aryl group are illustrated. As "aryloxy group", phenoxy group, 1-naphthoxy group or 2-naphthoxy group is illustrated. As "arylcarbonyl group", benzoyl group, 1-naphthoyl group or 2-naphthoyl group is illustrated. As "aryloxycarbonyl group", phenoxycarbonyl group, 1-naphthoxycarbonyl group or 2-naphthoxycarbonyl group is illustrated. As "arylcarbonyloxy group", benzoyloxy group, 1-naphthoyloxy group or 2-naphthoyloxy group is illustrated. As "arylsulfonyl group", phenylsulfonyl group, 1-naphthylsulfonyl group, or 2-naphthylsulfonyl group is illustrated. As "arylsulfinyl group ", phenylsulfinyl group, 1-naphthylsulfinyl group, or 2-naphthylsulfinyl group is illustrated.

As heteroaryl group in "heteroaryloxy group", "heteroarylcarbonyl group", "heteroaryloxycarbonyl group", "heteroarylcarbonyloxy group", "heteroarylsulfonyl group" and "heteroarylsulfinyl group", the same as the above heteroaryl groups are illustrated. As "heteroaryloxy group", pyrrolyloxy group, pyridyloxy group, pyrazinyloxy group, pyrimidinyloxy group, pyridazynyloxy group, furyloxy group, or thienyloxy group is illustrated. As "heteroarylcarbonyl group", pyrrolylcarbonyl group, pyridylcarbonyl group, pyrazinylcarbonyl group, pyrimidinylcarbonyl group, pyridazinylcarbonyl group, furylcarbonyl group, thienylcarbonyl group, etc. is illustrated. As "heteroaryloxycarbonyl group", pyrrolyloxycarbonyl group, pyridyloxycarbonyl group, pyrazinyloxycarbonyl group, pyrimidinyloxycarbonyl group, pyridazinyloxycarbonyl group, furyloxycarbonyl group, or thienyloxycarbonyl group is illustrated. As "heteroarylcarbonyloxy group", pyrrolylcarbonyloxy group, pyridylcarbonyloxy group, pyrazinylcarbonyloxy group, pyrimidinylcarbonyloxy group, pyridazinylcarbonyloxy group, furylcarbonyloxy group, or thienylcarbonyloxy group is illustrated. As "heteroarylsulfonyl group", pyrrolylsulfonyl group, pyridylsulfonyl group, pyrazinylsulfonyl group, pyrimidinylsulfonyl group, pyridazinylsulfonyl group, furylsulfonyl group, or thienylsulfonyl group is illustrated. As "heteroarylsulfinyl group", pyrrolylsulfinyl group, pyridylsulfinyl group, pyrazinylsulfinyl group, pyrimidinylsulfinyl group, pyridazinylsulfinyl group, furylsulfinyl group, or thienylsulfinyl group is illustrated.

Aromatic hydrocarbon group in A¹ and A² includes benzene ring and naphthalene ring and its binding position is not limited.

Heterocyclic aromatic group includes 5 to 10 membered monocyclic or bicyclic heteroaromatic ring containing 1 to 3 hetero atoms selected from 0 to 3 nitrogen atoms, 0 to 1 oxygen atom and 0 to 1 sulfur atom, such as furan, thiophene, pyrrol, pyridine, indole, isoindole, quinoline, isoquinoline, pyrazole, imidazole, pyrimidine, pyrazine, pyridazine, thiazole, oxazole, etc. The binding position in the heterocyclic aromatic group is not specifically limited if chemically stable.

As "4 to 7 membered saturated heterocyclic ring" in the present specification is illustrated 4 to 7 membered saturated heterocyclic ring containing 1 to 3 hetero atoms selected from 1 to 3 nitrogen atoms, 0 to 1 oxygen atom and 0 to 1 sulfur atom and its binding position is not limited if chemically stable. It includes azetidine, pyrrolidine, piperidine, piperazine, morpholine, thiomorpholine, thiomorpholine-1-oxide, thiomorpholine-1,1-dioxide, perhydroazepine, imidazolidine, oxazolizine, etc.

In the present specification, when alkyl group, alkenyl group, alkynyl group are substituted, "said substituent" is selected from the group consisting of the following groups (a) to (c):
(a) halogen atom, hydroxy group, carboxy group, haloalkoxy group, and mercapto group;
(b) alkoxy group, alkylthio group, alkylcarbonyl group, alkylcarbonyloxy group, alkylsulfonyl group, alkylsulfinyl group, alkoxycarbonyl group, alkenyloxy group, alkenylcarbonyl group, alkenylcarbonyloxy group, alkenylsulfonyl group, alkenylsulfinyl group, alkenyloxycarbonyl group, alkynyloxy group, alkynylcarbonyl group, alkynylcarbonyloxy group, alkynyloxycarbonyl group, alkynylsulfonyl group, and alkynylsulfinyl group (the group of this group may be substituted by one or two groups independently selected from the group consisting of halogen atom, hydroxy group, carboxyl group, alkoxy group, alkoxycarbonyl group, amino group optionally substituted by the same or different and one or two alkyl groups, carbamoyl group optionally substituted by the same or different and one or two alkyl groups, a sulfamoyl group optionally substituted by the same or different and one or two alkyl groups, and alkylsulfonyl group.);
(c) optionally substituted amino group, optionally substituted carbamoyl group and optionally substituted sulfamoyl group (the group of this group may be substituted by one or two groups independently selected from the group consisting of the following groups (j), (k) and (1).), optionally substituted aryl group, optionally substituted aryloxy group, optionally substituted arylcarbonyl group, optionally substituted arylcarbonyloxy group, optionally substituted arylsulfonyl group, optionally substituted arylsulfinyl group, optionally substituted aryloxycarbonyl group, optionally substituted heteroaryl group, optionally substituted heteroaryloxy group, optionally substituted heteroarylcarbonyl group, optionally substituted heteroarylcarbonyloxy group, optionally substituted heteroarylsulfonyl group, optionally substituted heteroarylsulfinyl group and optionally substituted heteroaryloxycarbonyl group (the group of this group may be substituted by one or more groups independently selected from the group consisting of the following groups (g), (h) and (i).), and optionally substituted cycloalkyl group, optionally substituted cycloalkoxy group, optionally substituted cycloalkylcarbonyl group, optionally substituted cycloalkylcarbonyloxy group, optionally substituted cycloalkylsulfonyl group, optionally substituted cycloalkylsulfinyl group, optionally substituted cycloalkoxycarbonyl group and optionally substituted saturated heterocyclic group (this group may be substituted by one or more groups independently selected from the group consisting of the following groups (d), (e) and (f).);
   and this group can be substituted by one or more, and the same or different groups, preferably 1 to 5, more preferably 1 to 3 groups.
   In the present specification, when cycloalkyl group, cycloalkoxy group, cycloalkylcarbonyl group, cycloalkylsulfonyl group, cycloalkylsulfinyl group, cycloalkylcarbonyloxy group, cycloalkoxycarbonyl group and saturated heterocyclic group are substituted, "said substituent" is selected from the group consisting of the following groups (d) to (f):
(d) halogen atom, hydroxy group, carboxy group, mercapto group, haloalkyl group, and haloalkoxy group;
(e) alkyl group, alkenyl group, alkynyl group, alkoxy group, alkylcarbonyl group, alkylcarbonyloxy group, alkoxycarbonyl group, alkylthio group, alkylsulfonyl group, and alkylsulfinyl group (the group of this group may be substituted by halogen atom, hydroxy group, carboxyl, alkoxy group group, alkoxycarbonyl group, amino group optionally substituted by the same or different and one or two alkyl groups, carbamoyl group optionally substituted by the same or different and one or two alkyl groups, a sulfamoyl group optionally substituted by the same or different and one or two alkyl groups, and alkylsulfonyl group.);
(f) optionally substituted aryl group and optionally substituted heteroaryl group (the group of this group may be substituted by the same or different and one or more groups selected from the following groups (g), (h) and (i).), or optionally substituted amino group, optionally substituted carbamoyl group or optionally substituted sulfamoyl group (the group of this group may be substituted by one or two groups selected from the following (j), (k) and (1).);
   and this group can be substituted by one or more, and the same or different groups, preferably 1 to 5, more preferably 1 to 3 groups.
   In the present specification, when aryl group, heteroaryl group, aromatic carbocyclic group and aromatic hetero cyclic group are substituted, said substituent is selected from the group consisting of the following groups (g) to (i):
(g) halogen atom, hydroxy group, carboxy group, mercapto group, cyano group, nitro group, haloalkyl group, and haloalkoxy group;
(h) alkyl group, alkenyl group, alkynyl group, alkoxy group, alkylcarbonyl group, alkylcarbonyloxy group, alkylthio group, alkoxycarbonyl group, alkylsulfonyl group, alkylsulfinyl group, cycloalkyl group, and saturated heterocyclic group (the group of this group may be substituted by halogen atom, hydroxy group, alkyl group, alkoxy group, carboxyl group, alkoxycarbonyl group, amino group optionally substituted by the same or different and one or two alkyl groups, carbamoyl group optionally substituted by the same or different and one or two alkyl groups, or a sulfamoyl group optionally substituted by the same or different and one or two alkyl groups, and alkylsulfonyl group.);
(i) optionally substituted amino group, optionally substituted carbamoyl group and optionally substituted sulfamoyl group (the group of this group may be substituted by the same or different and one or two groups described in the following (j), (k) and (l).);
   and this group can be substituted by one or more, and the same or different groups, preferably 1 to 5, more preferably 1 to 3 groups.
   "Substituent" in "optionally substituted amino group", "optionally substituted carbamoyl group" and "optionally substituted sulfamoyl group" is selected from the group consisting of the following groups (j), (k) and (l):
(j) alkyl group, alkenyl group, alkynyl group, alkylcarbonyl group, alkoxycarbonyl group, alkylsulfonyl group, alkylsulfinyl group, alkenylcarbonyl group, alkenyloxycarbonyl group, alkenylsulfonyl group, alkenylsulfinyl group, alkynylcarbonyl group, alkynyloxycarbonyl group, alkynylsulfonyl group, alkynylsulfinyl group, cycloalkyl group, cycloalkylcarbonyl group, cycloalkoxycarbonyl group, cycloalkylsulfonyl group, cycloalkylsulfinyl group, and saturated heterocyclic group (the group of this group may be substituted by one or two groups independently selected from the group consisting of halogen atom, hydroxy group, carboxyl group, alkoxy group, alkyl group, alkoxy group, alkoxycarbonyl group, amino group optionally substituted by the same or different and one or two alkyl groups, carbamoyl group optionally substituted by the same or different and one or two alkyl groups, a sulfamoyl group optionally substituted by the same or different and one or two alkyl groups, and alkylsulfonyl group.);
(k) aryl group, arylcarbonyl group, aryloxycarbonyl group, arylsulfonyl group, arylsulfinyl group, heteroaryl group, heteroarylcarbonyl group, heteroaryloxycarbonyl group, heteroarylsulfonyl group, and heteroarylsulfinyl group (the group of this group may be substituted by one or two groups independently selected from the group consisting of halogen atom, hydroxy group, alkyl group, alkoxy group, carboxy group, alkoxycarbonyl group, amino group optionally substituted by the same or different and one or two alkyl groups, carbamoyl group optionally substituted by the same or different and one or two alkyl groups, a sulfamoyl group optionally substituted by the same or different and one or two alkyl groups, and alkylsulfonyl group.);
   (1) 4 to 7 membered saturated heterocyclic group containing 1 to 4 hetero atoms selected from 1 to 2 nitrogen atoms, 0 to 1 oxygen atom and 0 to 1 sulfur atom, which is formed by combining two substituents on amino group, carbamoyl group or sulfamoyl group with the nitrogen atom (this saturated heterocyclic group may be substituted, if chemically stable, on optional its carbon atom or nitrogen atom, by one or two groups independently selected from the group consisting of halogen atom, hydroxy group, carboxyl group, alkyl group, alkoxy group, alkoxycarbonyl group, alkylcarbonyl group, amino group optionally substituted by the same or different and one or two alkyl groups, carbamoyl group optionally substituted by the same or different and one or two alkyl groups, a sulfamoyl group optionally substituted by the same or different and one or two alkyl groups, and alkylsulfonyl group.);

And said group may be substituted by one or two substituents, if chemically stable.

R² of the formula (1) is preferably C₁₋₄ alkyl group, C₃₋₈ alkylcarbonyloxyalkyl group, 6 to 10 membered arylcarbonyloxyalkyl group, 5 to 10 membered heteroarylcarbonyloxyalkyl group or alkyl group substituted by optionally substituted amino group. The alkyl group substituted by optionally substituted amino group preferably includes dialkylaminoalkyl group, or alkyl group substituted by morpholino group, 1-piperidinyl group, piperazino group or 1-pyrrolidinyl, for example 4-dimethylaminobutyl group, 4-morpholinobutyl group, etc.
As the above alkylcarbonyloxyalkyl group, acetoxymethyl group, 1-acetoxyethyl group, etc. are illustrated. As the above arylcarbonyloxyalkyl group, benzoyloxymethyl group is illustrated. R² is further preferably methyl group.

When optionally substituted alkylene in L⁴ of the formula (1) is substituted, the substituent is halogen atom, alkoxy group, etc.

A2 of the formula (1) is preferably benzene ring or 5 to 6 membered heteroaromatic ring containing at least one hetero atom selected from 0 to 2 nitrogen atoms, 0 to 1 oxygen atom and 0 to 1 sulfur atom, more preferably benzene, pyridine and furan, and its binding position is not limited, if chemically stable. L⁴ of the formula (1) is preferably a single bond or C₁₋₄, preferably C₁₋₃ straight or branched alkylene, more preferably a single bond, methylene, ethylene, 1-methylmethylene, or 1,1-dimethylmethylene.

Preferable mode of "-A²-L⁴-CO₂R²" of the formula (1) is selected from following formulas (9)~(20): 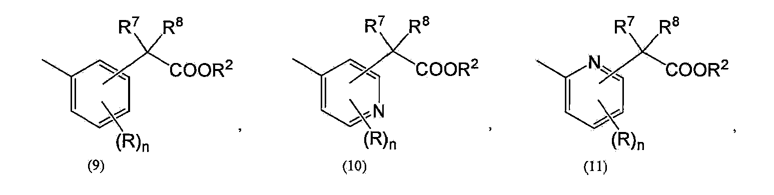 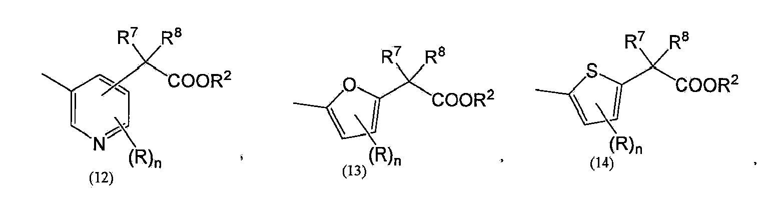 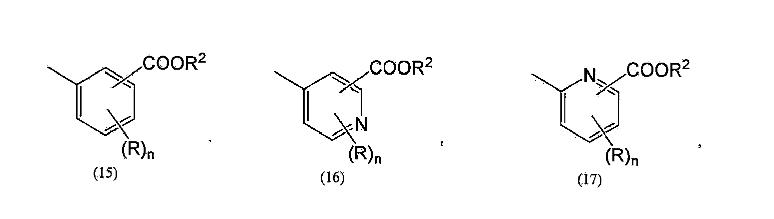 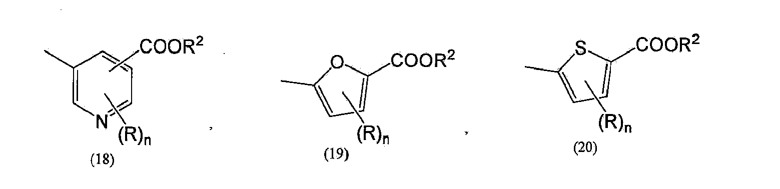 (wherein R² is the same as defined above, R⁷ and R⁸ are independently, hydrogen atom, C₁₋₄ alkyl group, R is hydrogen atom, halogen atom, haloalkyl group, alkyl group, alkoxy group, haloalkoxy group, amino group, alkylamino group or dialkylamino group, n is an integer 0-2 and when n is 2, R may be the same or different. The binding position thereof is not limited, if chemically stable.)

More preferable R⁷ and R⁸ in the formulas (9) to (14) are hydrogen atom.

L¹ in the formula (1) is preferably methylene.

L² in the formula (1) is preferably C₁₋₆ alkylene, and any methylene not adjacent to nitrogen atom in said alkylene may be substituted by oxygen atom.

L³ in the formula (1) is preferably C₁₋₆ alkylene, and any methylene not adjacent to nitrogen atom in said alkylene may be substituted by oxygen atom.

R³ in the formula (1) is preferably hydrogen atom, C₁₋₆ alkyl group, halogen atom, hydroxy group, optionally substituted amino group, C₁₋₆ alkyl group substituted by optionally substituted aryl group or optionally substituted heteroaryl group, and when said optionally substituted amino group is substituted, the substituted amino group includes morpholino group, dimethylamino group, diethylamino group, pyrrolidinyl group, piperidino group, etc. The "aryl group" includes phenyl group, the "heteroaryl group" includes imidazolyl group, pyridyl group, etc., and the above aryl group and heteroaryl group may be substituted by one or more substituents selected from halogen atom, hydroxy group, alkyl group, alkoxy group, haloalkyl group and haloalkoxy group.

When X in the formula (1) is NR⁵, R⁵ is preferably hydrogen atom, or C₁₋₃ alkyl group, more preferably hydrogen atom or methyl group. X is preferably oxygen atom, or a single bond.

"-L²-NR³-L³-" in the formula (1), a divalent group preferably includes the following formulas (2)~(7):

formula (2): -(O)ₚ-(CH₂)ₘ-NR³'-(CH₂)ₙ-(O)_{q}-

(wherein R³' is hydrogen atom; C₁₋₆ alkyl group; or C₂₋₆ alkyl group substituted by halogen atom, optionally substituted amino group, optionally substituted 6 to 10 membered aryl group, optionally substituted 5 to 10 membered heteroaryl group or hydroxy group, p and q are independently 0 or 1, and m and n are independently an integer of 1~4, provided that when p is 1, m is 2 or more, and when q is 1, n is 2 or more.); 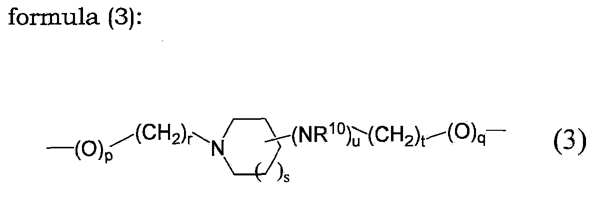 (wherein R¹⁰ is hydrogen atom or C₁₋₆ alkyl group, p and q are the same as defined above, r and t are independently an integer of 0-4, s is an integer of 0-2, and u is 0 or 1, provided that when p is 1, r is an integer of 2 or more, and when both u and q are 1, t is an integer of 2 or more.); 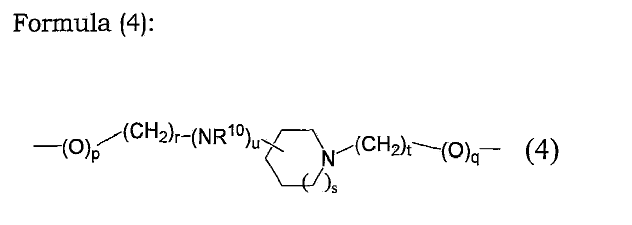 (wherein R¹⁰, p and q are the same as defined above, r and t are independently an integer of 0~4, s is an integer of 0~2, and u is 0 or 1, provided that when both p and u are 1, r is an integer of 2 or more, and when q is 1, t is an integer of 2 or more.); 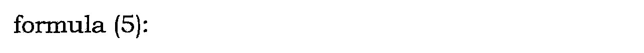 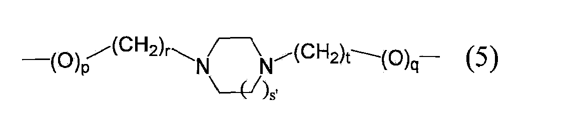 (wherein p and q are the same as defined above, r and t are independently an integer of 0~4, s' is 1 or 2, provided that when p is 1, r is an integer of 2 or more, and when q is 1, t is an integer of 2 or more.);

formula (6): -≡-(CH₂)ᵥ-NR³-(CH₂)ₙ-(O)_{q}- (6)

(wherein R^{3'}, q and n are the same as defined above, v is an integer of 0~3, provided that when q is 1, n is an integer of 2 or more.);

formula (7): -CO-NR³'-(CH₂)ₙ-(O)_{q}-

(wherein R³', q and n are the same as defined above, provided that when q is 1, n is an integer of 2 or more.);

In the formula (2), when optionally substituted amino group is substituted, said substituent includes a group selected from the group consisting of the above groups (j), (k) and (1), and when optionally substituted aryl group and optionally substituted heteroaryl group are substituted, said substituent includes a group selected from the group consisting of the above groups (g), (h) and (i).

Furthermore, when methylene in L³ is substituted by NR⁴, optionally substituted alkyl group in said R⁴ is combined together with carbon atom in L³ to form 4 to 7 membered saturated nitrogen containing hetero cycle, and that said carbon atom may be combined together with optionally substituted alkyl group in R³ on nitrogen atom adjacent to L³ to form 4 to 7 membered saturated nitrogen containing heterocycle. Namely "L2-NR3-L3" may form a spiro ring of the following formula (21): 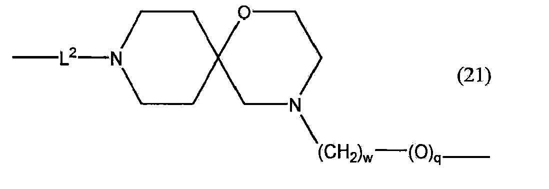 (wherein L² and q are the same as defined above, w is an integer of 0-6, and when q is 1, w is an integer of 2 or more.)

In the formula (1), R¹ is preferably, optionally substituted C₁₋₆ straight or branched alkyl group such as methyl group, ethyl group, propyl group, butyl group, pentyl group, 1-methylethyl group, 1-methylpropyl group, 2-methylbutyl group respectively optionally substituted, more preferably straight chained C₁₋₄ alkyl group.

The substituent wherein R¹ is substituted alkyl group includes the above substituent of alkyl group, preferably fluorine atom, hydroxy group, C₁₋₄ straight or branched alkoxy group, or C₁₋₄ branched alkylthio group, more preferably hydroxy group, or C₁₋₃ straight or branched alkoxy group, which may be substituted by one to three substituents.

The adenine compound of the present invention includes all tautomers, geometrical isomers and stereoisomers which are formed in accordance with the kind of the substituent, and a mixture thereof.

Namely, in a case where there are one or more asymmetrical carbon atoms in the compound of the formula (1), there exist diastereomers and optical isomers, and mixtures of those diastereomers and optical isomers and separated ones are also included in the present invention.

Additionally, the adenine compound shown by the formula (1) and its tautomer is chemically equivalent, and the adenine compound of the present invention includes such a tautomer. The tautomer is specifically a hydroxy compound shown by the formula (1'): 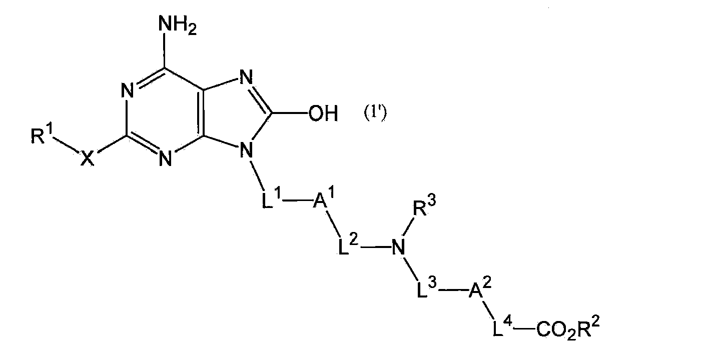 (wherein R¹, R², R³, A¹, A², X, L¹, L², L³ and L⁴ are the same as defined above.)

The pharmaceutically acceptable salt is exemplified by an acid salt and a base addition salt. The acid salt is, for example, an inorganic acid salt such as hydrochloride, hydrobromide, sulfate, hydroiodide, nitrate and phosphate, and an organic acid salt such as citrate, oxalate, acetate, formate, propionate, benzoate, trifluoroacetate, fumarate, maleate, succinate, tartrate, lactate, pyruvate, methanesulfonate, benzenesulfonate and p-toluenesulfonate, and the base salt is exemplified by an inorganic base salt such as sodium salt, potassium salt, calcium salt, magnesium salt and ammonium salt, and an organic base salt such as triethylammonium salt, triethanolammonium salt, pyridinium salt and diisopropylammonium salt, and further a basic or acidic amino acid salt such as arginine salt, aspartic acid salt and glutamic acid salt. The compound shown by the formula (1) may be hydrate and a solvate such as ethanolate.

The compound of the generic formula (1) can be prepared by the following method. The starting materials which are not described can be prepared in accordance with the following method or by known methods or in accordance with the known methods.

### Preparation method 1

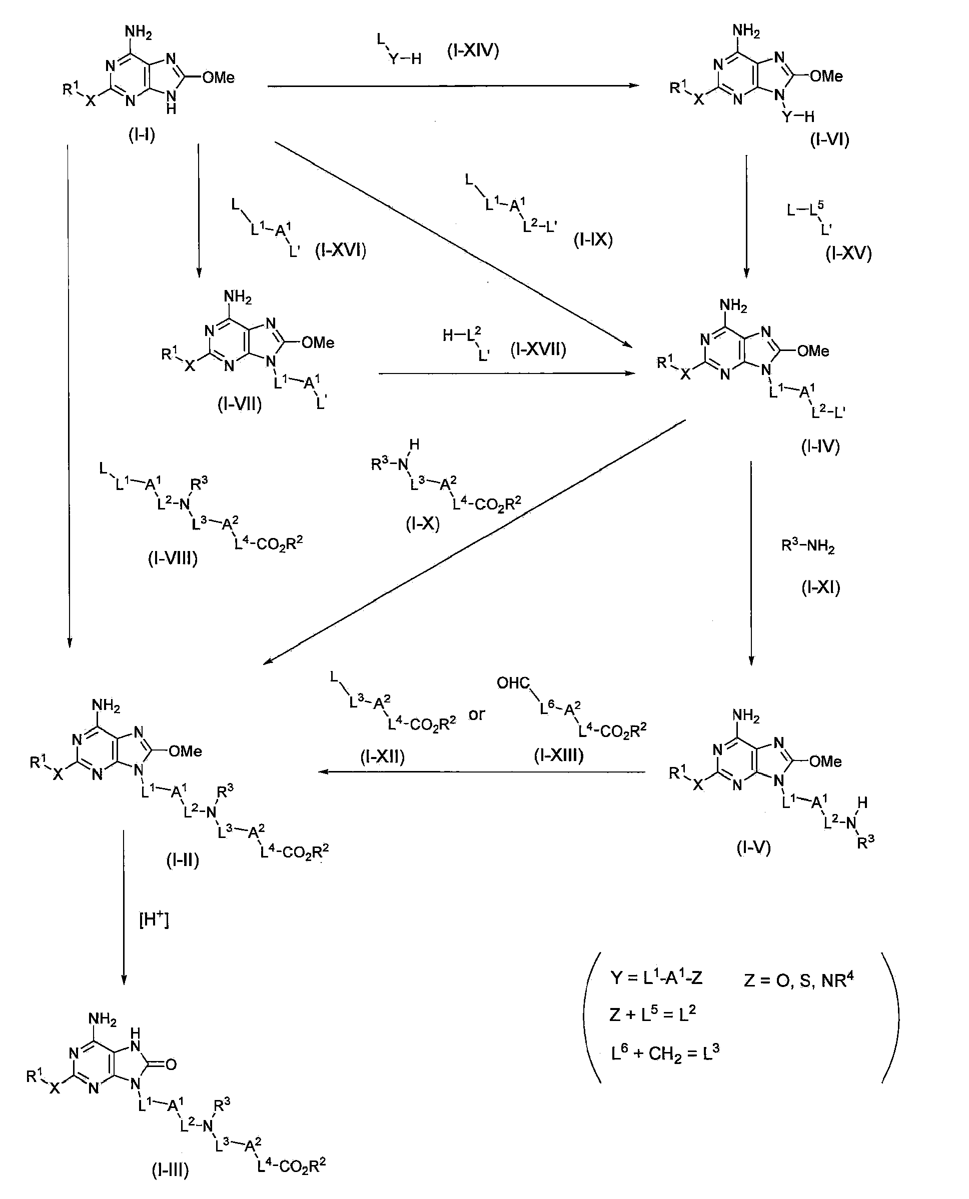

[wherein L and L' are the same or different and a leaving group, A¹, A², R¹, R², R³, X, F, L², L³ and L⁴ are the same as defined above. "-Y-" is a group represented by the following formulas: 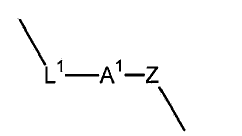 [wherein Z is oxygen atom, sulfur atom or NR⁴ (wherein R⁴ is the same as defined above.). L⁵ is a divalent group taken together Z to represent L². L⁶ is a divalent group taken together methylene to represent L³.]]

The leaving group herein includes halogen atom, sulfonyl group such as p-toluenesulfonyl group or methanesulfonyl group, etc., in alkylation reaction or acylation reaction.

When the compound of the present invention or its intermediate has a functional group such as amino group, carboxy group, hydroxy group, or oxo group etc., the compound can be protected or deprotected, if necessary. The preferable protecting group and the protecting method and deprotecting method are described in detail in "Protective Groups in Organic Synthesis 2nd Edition (John Wiley & Sons, Inc.; 1990)" and so on.

### [Step 1]

Compound (I-II) is prepared by reacting compound (I-I) and compound (I-VIII) in the presence of a base. The base includes, for example alkali metal carbonate such as sodium carbonate, potassium carbonate, etc., alkaline earth metal carbonate such as calcium carbonate, etc., metal hydroxide such as sodium hydroxide, potassium hydroxide, etc., metal hydride such as sodium hydride, etc, or metal alkoxide such as potassium t-butoxide, etc. The solvent includes an aprotic solvent such as dimethylformamide, dimethyl sulfoxide, acetonitrile, etc., a halogenated hydrocarbon such as carbon tetrachloride, chloroform, methylene chloride, etc., an ether such as diethyl ether, tetrahydrofuran, 1,4-dioxne, etc. The reaction temperature is selected from the range of about 0°C to around boiling point of the solvent.

### [Step 2]

Compound (I-III) can be prepared by treating compound (I-II) under acidic condition.

The acid includes an inorganic acid such as hydrochloric acid, hydrobromic acid, sulfuric acid, etc., or an organic acid such as trifluoroacetic acid, etc. The solvent includes water or a mixture of water and an organic solvent. The above organic solvent includes an ether such as diethyl ether, tetrahydrofuran etc., an aprotic solvent such as dimethylformamide, acetonitrile, etc., and an alcohol such as methanol, ethanol, etc. The reaction temperature is selected from the range of room temperature to around boiling point of the solvent. The modification of methoxy group at position 8 of adenine ring to oxo group may be carried out in any step as well as the last step.

### [Step 3]

Compound (I-VIII) which is a starting material of step 1 can be prepared by the following methods. 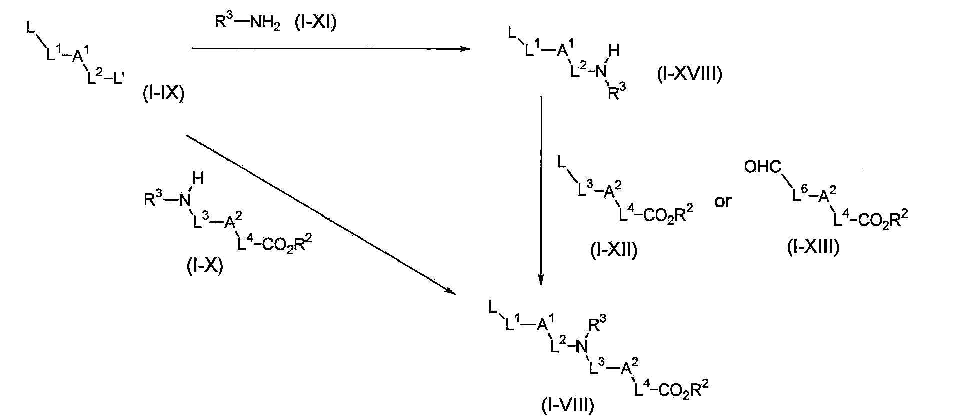 (wherein L and L' are the same or different and a leaving group, A¹, A², R², R³, L¹, L², L³, L⁴ and L⁶ are the same as defined above.)
Compound (I-VIII) can be prepared by reacting compound (I-IX) and compound (I-X) in the same manner as the above method.

Compound (I-VIII) can be also prepared by reacting compound (I-IX) and compound (I-XI) in the same manner as the above method to obtain compound (I-XVIII) and then by reacting compound (I-XVIII) and compound (I-XII) in the same manner as the above step 1.

Compound (I-VIII) can be also prepared by reacting compound (I-XVIII) and an aldehyde compound of compound (I-XIII) in a solvent of methanol etc., in the presence of a reducing agent such as NaBH₄, etc. Furthermore, in step 1 to compound (I-II) from compound (I-I), compound (I-II) can be also prepared by reacting compound (I-I) and compound (I-IX) in the same manner as the above step 1 to obtain compound (I-IV), and then reacting compound (I-IV) and compound (I-X) in the same manner as the above step 1.

In step to compound (I-II) from compound (I-IV), compound (I-II) can be prepared by reacting compound (I-IV) and compound (I-XI) in the same manner as the above step 1 to obtain compound (I-V) and then by reacting compound (I-V) and compound (I-XIII) in the same manner as the above step 1. Compound (I-II) can be also prepared by condensing compound (I-V) with compound (I-XII) by the reductive amination as described in step 3.

Furthermore, in step to compound (I-IV) from compound (I-I), compound (I-II) can be also prepared by reacting compound (I-I) and compound (I-XIV) in the same manner as the above step 1 to obtain compound (I-VI), and then by reacting compound (I-VI) and compound (I-XV) in the same manner as the above step 1.

Compound (I-IV) can be also prepared by reacting compound (I-I) and compound (I-XVI) in the same manner as the above step 1 to obtain compound (I-VII), and then by reacting compound (I-VII) and compound (I-XVII) in the same manner as the above step 1.

In each step, according to the structure of each intermediate, the compound can be prepared by the well known method in the art (for example, alkylation, dehydrative condensation of a carboxylic acid and an amine compound or reductive alkylation of an amine compound, and so on).

### [Step 4]

Compound (I-I) can be prepared by the following methods. 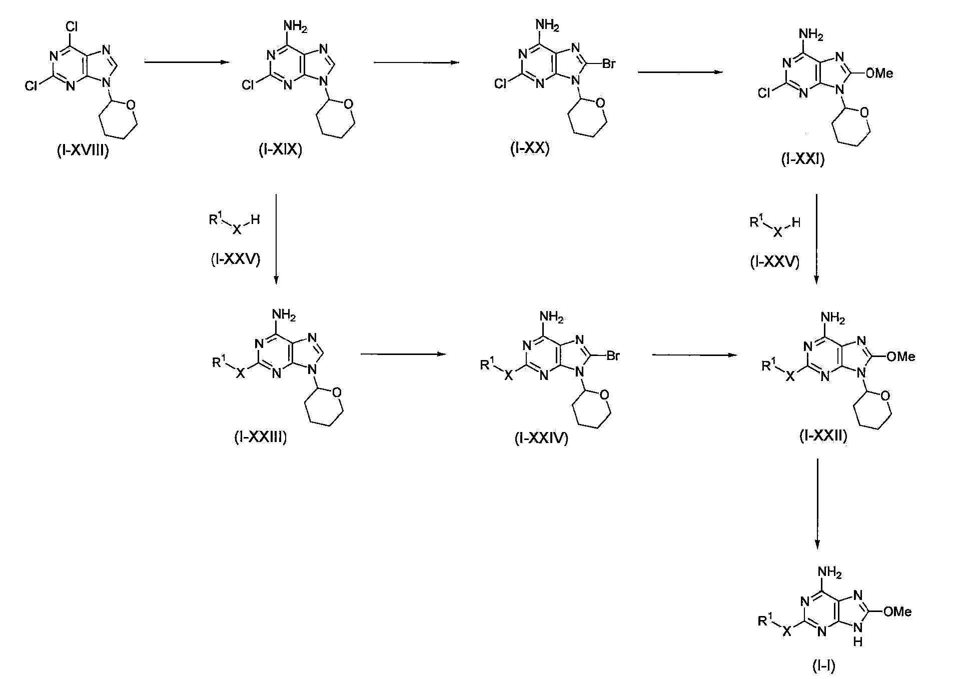 (wherein R¹ and X are the same as defined above.)

Compound (I-XIX) can be prepared by reacting compound (I-XVIII) and ammonia in an aqueous solution, an organic solvent or a mixture thereof.

The organic solvent includes an alcohol such as methanol, ethanol, propanol, butanol, etc., an ether such as tetrahydrofuran, 1,4-dioxane, diglyme, etc., and an aprotic solvent such as acetonitrile, etc. The reaction temperature is selected from the range of room temperature to 200°C. The reaction may be carried out in a reaction vessel such as an autoclave, etc.

Compound (I-XX) can be prepared by brominating compound (I-XIX). The brominating agent includes for example, bromine, hydrobromic acid perbromide, N-bromosuccinimide, etc. In this reaction, a reaction auxiliary such as sodium acetate, etc., may be added. The solvent includes a halogenated hydrocarbon such as carbon tetrachloride, methylene chloride, dichloroethane, etc., an ether such as diethyl ether, etc., acetic acid, and carbon disulfide. The reaction temperature is selected from the range of about 0°C to around boiling point of the solvent.

Compound (I-XXI) can be obtained by reacting compound (I-XX) and sodium methoxide.

An organic solvent includes an ether such as diethyl ether, tetrahydrofuran, 1,4-dioxane, etc., an aprotic solvent such as dimethylformamide, etc., and an alcohol such as methanol, etc. The reaction temperature is selected from the range of room temperature to around boiling point of the solvent.

Compound (I-XXI) can be prepared by treating compound (I-XX) with an aqueous alkaline solution containing methanol.

The aqueous alkaline solution includes an aqueous solution of alkali metal hydroxide such as sodium hydroxide, potassium hydroxide, etc. The reaction temperature is selected from the range of room temperature to around boiling point of the solvent.

Compound (I-XXII) can be prepared by reacting compound (I-XXI) and compound (I-XXV).

When X is NR⁹ (wherein R⁹ is the same as define above.), the reaction is carried out in the presence or absence of a base. The base includes an alkali metal carbonate such as sodium carbonate, potassium carbonate, etc., an alkaline earth metal carbonate such as calcium carbonate, etc., a metal hydroxide such as sodium hydroxide, potassium hydroxide, etc., and an organic base such as triethylamine, diisopropylethylamine, 4-dimethylaminopyridine, etc. The solvent includes an ether such as tetrahydrofuran, 1,4-dioxane, diglyme, etc., an alcohol such as propanol, butanol, etc., and an aprotic solvent such as dimethylformamide, etc. The reaction may be carried out in the absence of the solvent. The reaction temperature is selected from the range of about 50°C to 200°C.

When X is oxygen atom or sulfur atom, the reaction is carried out in the presence of a base. The base includes an alkali metal such as sodium, potassium, etc., and an alkali metal hydride such as sodium hydride. The solvent includes an ether such as tetrahydrofuran, 1,4-dioxane, diglyme, etc., an aprotic solvent such as dimethylformamide, dimethyl sulfoxide, etc. The reaction may be carried out in the absence of the solvent. The reaction temperature is selected from the range of about 50°C to 200°C.

In step to compound (I-XXII) from compound (I-XIX), compound (I-XXII) can be prepared by reacting compound (I-XXV) in the same manner as described above to obtain compound (I-XXIII) and then by converting compound (I-XXIII) to compound (I-XXIV) by bromination, followed by methoxylation at 8-position.

Compound (I-I) can be prepared by treating compound (I-XXII) with trifluoroacetic acid in an organic solvent such as methanol, etc.

The acid includes an inorganic acid such as hydrochloric acid, hydrobromic acid, sulfuric acid, etc., and an organic acid such as trifluoroacetic acid, etc. The solvent includes water, and a mixture of water and an organic solvent. The organic solvent includes an ether such as diethyl ether, tetrahydrofuran, etc., an aprotic solvent such as dimethylformamide, acetonitrile, etc., and an alcohol such as methanol, ethanol, etc. The reaction temperature is selected from the range of at room temperature to around boiling point of the solvent.

The compound of the generic formula (1) can be prepared by the following methods starting from compound (II-I). The process for preparing starting compound (II-I) is described in WO 02/85905, and WO 2004/29054 in detail and if necessary can be prepared referring to these documents. The starting materials not described in below can be prepared accordance with the description of the present invention or a known method or the similar method.

### Preparation method 2

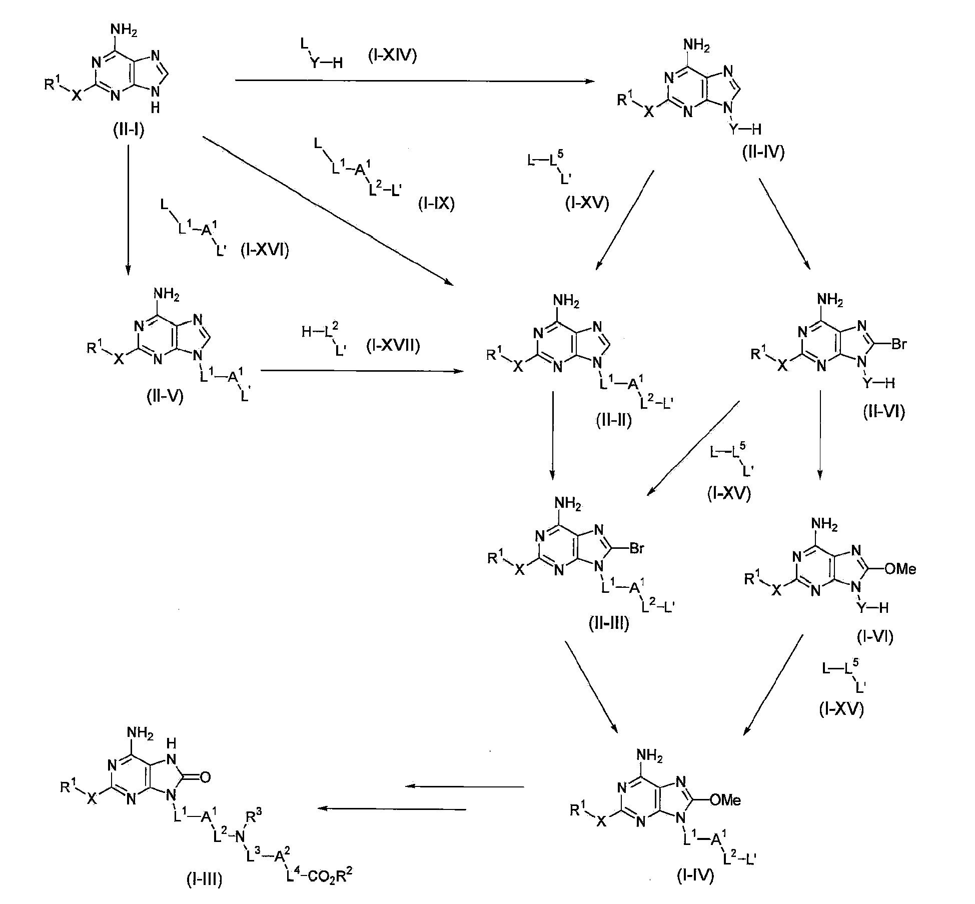

(wherein L and L' are the same or different and are a leaving group, A¹, A², R¹, R², R³, L¹, L², L³, L⁴, L⁵, L⁶, X and Y are the same as defined above.)

### [Step 5]

Compound (II-II) can be prepared by reacting compound (II-I) and compound (I-IX) in the presence of a base. The base includes, for example alkali metal carbonate such as sodium carbonate, potassium carbonate, etc., alkaline earth metal carbonate such as calcium carbonate, etc., metal hydroxide such as sodium hydroxide, potassium hydroxide, etc., metal hydride such as sodium hydride, etc, or metal alkoxide such as potassium t-butoxide, etc. The solvent includes an aprotic solvent such as dimethylformamide, dimethyl sulfoxide, acetonitrile, etc., a halogenated hydrocarbon such as carbon tetrachloride, chloroform, methylene chloride, etc., and an ether such as diethyl ether, tetrahydrofuran, 1,4-dioxane, etc. The reaction temperature is selected from the range of about 0°C to around boiling point of the solvent.

In step to compound (II-II) from compound (II-I), compound (II-II) can be also prepared via compound (II-IV) or compound (II-V) in the same manner as step 5.

### [Step 6]

Compound (II-III) can be prepared by brominating compound (II-II). The brominating agent includes for example, bromine, hydrobromic acid perbromide, N-bromosuccinimide, etc. In this reaction, a reaction auxiliary such as sodium acetate, etc., may be added. The solvent includes a halogenated hydrocarbon such as carbon tetrachloride, methylene chloride, dichloroethane, etc., an ether such as diethyl ether, etc., acetic acid, and carbon disulfide. The reaction temperature is selected from the range of about 0°C to around boiling point of the solvent.

### [Step 7]

Compound (I-IV) can be obtained by reacting compound (II-III) and a metal alkoxide such as sodium methoxide, etc.

An organic solvent used in this reaction includes an ether such as diethyl ether, tetrahydrofuran, 1,4-dioxane, etc., an aprotic solvent such as dimethylformamide, etc., and an alcohol corresponding to the metal alkoxide such as methanol, etc. The reaction temperature is selected from the range of room temperature to around boiling point of the solvent.

Compound (I-IV) can be also prepared via compound (II-III) or compound (I-VI) in the same manner as the above step 5, after obtaining compound (II-VI) by bromination of compound (II-IV) in the same manner as the above step 6.

Compound (I-III) can be prepared using compound (I-IV) in the similar manner as preparation method 1.

Even in the compound wherein methylene group in L¹, L² or L³ of the formula (1) is substituted by oxygen atom, etc., the compound can be prepared by the above preparation method 1 or 2, or a known method in the art. There are illustrated for example, the process for preparing an amide compound by condensing an amine compound and a carboxylic acid compound in the presence of dicyclohexylcarbodiimide, a process for preparing a sulfonamide compound by condensing an amine compound and chlorosulfonyl compound in the presence of a base, and a process for preparing a sulfoxide compound or sulfone compound by oxidizing a thioether compound using m-chloroperbenzoic acid or hydrogen peroxide and so on.

In a case where the compound of the present invention, its intermediate or the starting compound has a functional group, a reaction for increasing a carbon atom, a reaction for introducing a substituent or a reaction for conversion of the functional group can be conducted optionally according to a manner conventional to the skilled artisan in an appropriate step, namely in an intermittent step in each of the preparation methods described in the preparation method 1 or 2. For this purpose, the methods described in "JIKKEN KAGAKU-KOZA (edited by NIHON KAGAKU-KAI, MARUZEN)", or "Comprehensive Organic Transformation, R. C. Larock (VCH Publishers, Inc. 1989)" can be used. The reaction for increasing a carbon atom includes a method comprising converting an ester group to hydroxymethyl group using a reducing agent such as lithium aluminum hydride, introducing a leaving group and then introducing a cyano group. The reacting for conversion of a functional group includes a reaction for conducting acylation or sulfonylation using an acid halide, a sulfonyl halide, etc., a reaction for reacting an alkylation agent such as an alkyl halide, a hydrolysis reaction, a reaction for C-C bond formation such as Friedel-Crafts reaction and Wittig reaction, and oxidizing or reducing reaction, etc.

In a case where the compound of the present invention or its intermediate contains a functional group such as amino group, carboxy group, hydroxy group and oxo group, a technology of protection and deprotection can optionally be used. A preferable protective group, a protection method and a deprotection method are described in details in "Protective Groups in Organic Synthesis 2nd Edition (John Wiley & Sons, Inc.; 1990)", etc.

The compound of the formula (1) of the present invention and the intermediate compound for production thereof can be purified by a method known to the skilled artisan. For instance, purification can be conducted by column chromatography (e.g. silica gel column chromatography or ion exchange chromatography) or recrystallization. As a recrystallization solvent, for instance, can be used an alcohol such as methanol, ethanol and 2-propanol, an ether such as diethyl ether, an ester such as ethyl acetate, an aromatic hydrocarbon such as benzene and toluene, a ketone such as acetone, a hydrocarbon such as hexane, an aprotic solvent such as dimethylformamide and acetonitrile, water and a mixture of two or more thereof. As other purification method, can be used those described in "JIKKEN KAGAKU-KOZA (edited by NIHON KAGAKU-KAI, MARUZEN) Vol. 1", etc.

In a case where the compound of the formula (1) of the present invention contains one or more asymmetric carbon, its production can be conducted by using the starting material containing those asymmetric carbons or by asymmetric induction during the production steps. For instance, in a case of an optical isomer, the object can be obtained by using an optically active starting material or by conducting an optical resolution at a suitable stage of the production steps. The optical resolution method can be conducted by a diastereomer method comprising allowing the compound of the formula (1) or its intermediate to form a salt with an optically active acid (e.g. a monocarboxylic acid such as mandelic acid, N-benzyloxyalanine and lactic acid, a dicarboxylic acid such as tartaric acid, o-diisopropylidene tartrate and malic acid, a sulfonic acid such as camphor sulfonic acid and bromocamphor sulfonic acid) in an inert solvent (e.g. an alcohol such as methanol, ethanol, and 2-propanol, an ether such as diethyl ether, an ester such as ethyl acetate, a hydrocarbon such as toluene, an aprotic solvent such as acetonitrile and a mixture thereof).

In a case where the compound of the formula (1) or its intermediate contains an acidic functional group such as carboxylic group, the object can be attained also by forming a salt with an optically active amine (e.g. an organic amine such as α-phenethylamine, quinine, quinidine, cinchonidine, cinchonine and strychnine).

The temperature for salt formation is selected from room temperature to the boiling point of the solvent. In order to increase optical purity, the temperature is preferably once increased up to the boiling point of the solvent. Upon recovering the salt formed by filtration, the yield can be increased optionally by cooling. An amount of the optical active acid or amine is about 0.5 to about 2.0 equivalent, preferably around 1 equivalent, relative to the substrate. An optically active salt with highly optical purity can be obtained optionally by recrystallization from an inert solvent (e.g. an alcohol such as methanol, ethanol and 2-propanol, an ether such as diethyl ether, an ester such as ethyl acetate, a hydrocarbon such as toluene, an aprotic solvent such as acetonitrile and a mixture thereof). If necessary, the optically resoluted salt can be converted into a free form by treating with an acid or a base by the conventional method.

The adenine compound or its pharmaceutically acceptable salt of the present invention activates Toll-like receptor (TLR), concretely TLR7 and is useful as an immuno-modulator and thus useful as a therapeutic and prophylactic agent for diseases associated with an abnormal immune response (e.g. autoimmune diseases and allergic diseases) and various infectious diseases and cancers which are required for activation of an immune response. For instance, the adenine compound or its pharmaceutically acceptable salt of the present invention is useful as a therapeutic and prophylactic agent for the diseases mentioned in the following (1)-(8).
(1) (Respiratory diseases) asthma, including bronchial, allergic, intrinsic, extrinsic, exercise-induced, drug-induced (including NSAID such as aspirin and indomethacin) and dust-induced asthma both intermittent and persistent and of all severities, and other causes of airway hyper-responsiveness; chronic obstructive pulmonary disease (COPD); bronchitis, including infectious and eosinophilic bronchitis; emphysema; bronchiectasis; cystic fibrosis; sarcoidosis; farmer's lung and related diseases; hypersensitivity pneumonitis; lung fibrosis, including cryptogenic fibrosing alveolitis, idiopathic interstitial pneumonias, fibrosis complicating anti-neoplastic therapy and chronic infection, including tuberculosis and aspergillosis and other fungal infections; complications of lung transplantation; vasculitic and thrombotic disorders of the lung vasculature, and pulmonary hypertension; antitussive activity including treatment of chronic cough associated with inflammatory and secretory conditions of the airways, and iatrogenic cough; acute and chronic rhinitis including rhinitis medicamentosa, and vasomotor rhinitis; perennial and seasonal allergic rhinitis including rhinitis nervosa (hay fever); nasal polyposis; acute viral infection including the common cold, and infection due to respiratory syncytial virus, influenza, coronavirus (including SARS) and adenovirus;
(2) (Skin) psoriasis, atopic dermatitis, contact dermatitis or other eczematous dermatoses, and delayed-type hypersensitivity reactions; phyto-and photodermatitis; seborrheic dermatitis, dermatitis herpetiformis, lichen planus, lichen sclerosus, lichen sclerosus et atrophicus, pyoderma gangrenosum, skin sarcoidosis, discoid lupus erythematosus, pemphigus, pemphigoid, epidermolysis bullosa, urticaria, angioedema, vasculitides, toxic erythemas, cutaneous eosinophilias, alopecia areata, male-pattern baldness, Sweet's syndrome, Weber-Christian syndrome, erythema multiforme; cellulitis, both infective and non-infective; panniculitis; cutaneous lymphomas, non-melanoma skin cancer and other dysplastic lesions; drug-induced disorders including fixed drug eruptions;
(3) (Eyes) blepharitis; conjunctivitis, including perennial and vernal allergic conjunctivitis; iritis; anterior and posterior uveitis; choroiditis; autoimmune, degenerative or inflammatory disorders affecting the retina; ophthalmitis including sympathetic ophthalmitis; sarcoidosis; infections including viral, fungal, and bacterial;
(4) (Genitourinary) nephritis including interstitial and glomerulonephritis; nephrotic syndrome; cystitis including acute and chronic (interstitial) cystitis and Hunner's ulcer; acute and chronic urethritis, prostatitis, epididymitis, oophoritis and salpingitis; vulvo-vaginitis; Peyronie's disease; erectile dysfunction (both male and female);
(5) (Allograft rejection) acute and chronic following, for example, transplantation of kidney, heart, liver, lung, bone marrow, skin or cornea or following blood transfusion; or chronic graft versus host disease;
(6) (Auto-immune diseases) other auto-immune and allergic disorders including rheumatoid arthritis, irritable bowel syndrome, systemic lupus erythematosus, multiple sclerosis, Hashimoto's thyroiditis, Graves' disease, Addison's disease, diabetes, idiopathic thrombocytopaenic purpura, eosinophilic fasciitis, hyper-IgE syndrome, antiphospholipid syndrome;
(7) (Oncology) treatment of common cancers including prostate, breast, lung, ovarian, pancreatic, living bowel and colon, stomach, skin and brain tumors and malignant bone marrow neoplasm (including the leukaemias) and lymphoproliferative systems neoplasm, such as Hodgkin's and non-Hodgkin's lymphoma; including the prevention and treatment of metastasis and tumor recurrences, and paraneoplastic syndromes; and
(8) (Infectious diseases) viral diseases such as genital warts, common warts, plantar warts, hepatitis B, hepatitis C, herpes simplex virus, molluscum contagiosum, variola, or acquired immunodeficiency syndrome (HIV), human papilloma virus (HPV), cytomegalo virus (CMV), varicella zoster virus (VZV), rhinovirus, adenovirus, coronavirus, influenza, or para-influenza; bacterial diseases such as tuberculosis, mycobacterium avium, or leprosy; other infectious diseases, such as fungal diseases, candida, chlamydia, or aspergillus, cryptococcal meningitis, pneumocystis carnii, cryptosporidiosis, histoplasmosis, toxoplasmosis, trypanosome infection, or leishmaniasis.

The adenine compounds or pharmaceutically acceptable salt thereof can also be used as vaccine adjuvant.

The adenine compound of the present invention, or its pharmaceutically acceptable salt has an activating effect of TLR, concretely TLR7. The adenine compound of the present invention, or its pharmaceutically acceptable salt shows an interferon-α or interferon-y inducing activity and a suppressing activity of the production of IL-4 or IL-5, and thus shows an effect as a medicament having an immunomodulating activity specific against type 1 helper T-cell (Th1 cell)/type 2 helper T-cell (Th2 cell), namely, preferably useful as a prophylactic or therapeutic agent for allergic diseases such as asthma, COPD, allergic rhinitis, allergic conjunctivitis and atopic dermatosis due to the cell selective immuno-suppressive action. On the other hand, due to its immune activating effect, it is useful as a prophylactic or therapeutic agent for cancer, hepatitis B, hepatitis C, acqured immnodeficiency syndrome (HIV) and human papilloma virus (HPV), a bacterial infectious disease and dermatosis such psoriasis.

The adenine compound of the present invention, or its pharmaceutically acceptable salt is useful as a prophylactic or therapeutic agent for airway obstruction such as asthma or COPD, or for reduction of the risk thereof.

The adenine compound of the present invention or its pharmaceutically acceptable salt has no limitation as to its administration formulation and is administered orally or parenterally. The preparation for oral administration can be exemplified by capsules, powders, tablets, granules, fine-grain, syrups, solutions, suspensions, etc., and the preparation for parenteral administration can be exemplified by injections, drips, eye-drops, intrarectal preparations, inhalations, sprays (e.g., liquids/suspensions for sprays, aerosols, or cartridge spray for inhalators or insufflators), lotions, gels, ointments, creams, transdermal preparations, transmucosa preparations, nasal drops, ear drops, tapes, transdermal patches, cataplasms, powders for external application, and the like. Those preparations can be prepared by known manners, and acceptable conventional carriers, fillers, binders, lubricants, stabilizers, disintegrants, buffering agents, solubilizing agents, isotonic agents, surfactants, antiseptics, perfumes, and so on can be used. Two or more pharmaceutical carriers can be appropriately used.

The compound of the present invention, or its pharmaceutically acceptable salt is admixed with a pharmaceutically acceptable carrier by the conventional method for the person in the art to prepare the pharmaceutical composition suitable for administration. For example, the pharmaceutical composition containing the compound of the present invention or its pharmaceutically acceptable salt 0.05-99 weight %, preferably 0.05 - 80 weight %, more preferably 0.1 - 70 weight %, and further more preferably 0.1 - 50 weight % as an active ingredient can be prepared.

The liquid preparation such as emulsions and syrups, among the preparations for oral administration, can be prepared by using additives for a pharmaceutical preparation including water; a sugar such as sucrose, sorbitol and fructose; ehanol; a glycol such as polyethylene glycol and propylene glycol; an oil such as sesame oil, olive oil and soybean oil; an preservative such as p-hydroxybenzoate; a sweetening such as saccharin, a thickening agent such as carboxymethyl cellulose, a flavor such as strawberry flavor and peppermint flavor, a coloring agent and so on.

The solid preparation such as capsules, tablets, powders and granules can be prepared by appropriatelly using following fillers: a carrier such as lactose, glucose, sucrose sorbitol, mannitol, mannite and a cellulose derivative; a disintegrant such as starch (potato starch, corn starch, amylopectin, etc.), and sodium alginate; a lubricant such as magnesium stearate, calcium stearate, polyethylene glycol, wax, paraffin and talc; a binder such as polyvinyl alcohol, polyvinyl pyrrolidone, hydroxypropyl cellulose and gelatin; a surfactant such as a fatty acid ester; or a plasticizer such as glycerin.

In case of preparation of sugar coated tablets, a condensed sugar solution, which may contain gum arabic, gelatin, talc, or titanium oxide is coated on the core of tables prepared by using fillers as described above. There can be also prepared a film coated tablet, which is coated by a suitable polymer dissolved in an easily removable organic solvent.

In case of preparation of soft gelatin capsules, the capsules can be prepared by mixing the compound of the present invention with for example, vegetable oil or polyethylene glycol. In case of preparation of hard gelatin capsules, the capsules can be prepared by using granules of the compound of the present invention which are prepared by mixing it with suitable fillers as described above.

The liquid preparation such as injections, drips, eye-drops and ear drops, among the preparations for parenteral administration, can be prepared preferably as a sterilized isotonic liquid preparation. For instance, injections can be prepared by using an aqueous medium such as a salt solution, a glucose solution or a mixture of a salt solution and a glucose solution. The preparation for intrarectal administration can be prepared by using a carrier such as cacao butter usually in the form of suppository.

The ointments, creams and gels contain the compound of the present invention usually in an amount of 0.01-10 w/w%, and there may be incorporated a thickener suitable to an aqueous or oily base and/or a gelling agent and/or a solvent. The base is exemplified by water and/or an oil such as liquid paraffin, a vegetable oil such as arachis oil and castor oil, a solvent such as polyethylene glycol, and so on. The thickener and gelling agent are exemplified by soft paraffin, aluminum stearate, cetostearic alcohol, polyethylene glycol, sheep fat, beeswax, carboxypolymethylene and cellulose derivatives and/or glyceryl monostearate and/or nonionic emulsifiers.

The lotions contain the compound of the present invention usually in an amount of 0.01-10 w/w%, and it may be prepared with the use of an aqueous or oily base, it may contain generally emulsifiers, stabilizers, dispersing agents, precipitation inhibitors and also thickeners.

Powders for external use contain the compound of the present invention usually an amount of 0.01-10 w/w%, and it may be formulated using a suitable powdery base such as talc, lactose and starch.

The drips may be formulated by using an aqueous or non-aqueous base, and may contain dispersing agents, solubilizing agents, precipitation inhibitors or preservatives.

The sprays (sprays, aerosols, dry-powders, etc.) may be formulated into an aqueous solution or suspension using a suitable liquid propellant, or into an aerosol distributed from a pressured package such as a metered-dose inhaler. Dry-powders preparations can be used.

The aerosols suitable to inhalation may be a suspension or aqueous solution, and they contain generally the compound of the present invention and a suitable propellant such as fluorocarbon, hydrogencontaining chlorofluorocarbon and a mixture thereof, particularly hydrofluoroalkane, specifically 1,1,1,2-tetrafluoroethane, heptafluoroalkane (HFA) such as 1,1,1,2,3,3,3-heptafluoro-n-propane or a mixture thereof. The aerosols may contain optionally additional excipients well known in the art such as a surfactant, (e.g., oleic acid or lecithin) and a co-solvent such as ethanol. For example, an inhaler known as Turubuhaler® is illustrated.

The gelatin capsules or cartridges used for inhalator or insufflator may be formulated by using a powdery mixture of the compounds used in the present invention and a powdery base such as lactose and starch. They contain the compound of the present invention usually in an amount of 20µg-10mg. The compound of the present invention may be administered without using excipients such as lactose as an alternative method.

In case of being orally or nasally inhalated in the form of pressured HFA aerosols or dry-powders preparations, the adenine compound of the present invention, or its pharmaceutically acceptable salt is pulverized in a size of less than 10µm and it is dispersed in a dispersing agent such as C₈₋₂₀ fatty acid or its salt (e.g., oleic acid), bile salt, phospholipid, an alkyl saccharide, a completely fluorinated or polyethoxylated surfactant, or a pharmaceutically acceptable dispersing agent.

The adenine compound of the present invention is preferably parenterally administered as a preparation for topical administration. The suitable preparation is exemplified by ointments, lotions (solutions or suspensions), creams, gels, tapes, transdermal patches, cataplasms, sprays, aerosols, dry-powders, aqueous solutions/suspensions for cartridge spray for inhalators or insufflators, eye-drops, ear drops, nasal drops, transdermal agents, pulmonary absorbent, air-way absorbent, powders for external administrations and so on.

A ratio of the active compound of the present invention in the preparation for topical administration of the present invention is, though depending upon the formulation, generally 0.001-10 wt%, preferably 0.005-1%. The ratio used in powders for inhalation or insufflation is 0.1-5%.

In a case of aerosols, the compound of the present invention is preferably contained in an amount of 20-2000µg, more preferably about 20µg-500µg per each a measured amount or one sprayed amount. The dosage is once or several times per day, for instance, 2,3, 4 or 8 times, and one to three units are administered per each time.

The pharmacological activity can be measured by any of conventional evaluation methods, preferably by an in vitro evaluation method. An example of the methods is a method described in examples of the present specification.

The invention further relates to combination therapies wherein a compound of the formula (1) or its pharmaceutically acceptable salt or a pharmaceutical composition comprising a compound of the formula (1) or its pharmaceutically acceptable salt is administered concurrently or sequentially or as a combined preparation with other therapeutic agent(s), for the treatment of one or more of the conditions listed in the specification.

In particular, for the treatment of the inflammatory diseases, COPD, asthma and allergic rhinitis, the compounds of the invention may be combined with agents such as tumour necrosis factor alpha (TNF-α) inhibitors such as anti-TNF monoclonal antibodies (for example Remicade, CDP-870 and adalimumab) and TNF receptor immunoglobulin molecules (such as Enbrel); non-selective cyclo-oxygenase COX-1/COX-2 inhibitors whether applied topically or systemically (such as piroxicam, diclofenac, propionic acids such as naproxen, flubiprofen, fenoprofen, ketoprofen and ibuprofen, fenamates such as mefenamic acid, indomethacin, sulindac, azapropazone, pyrazolones such as phenylbutazone, salicylates such as aspirin), COX-2 inhibitors (such as meloxicam, celecoxib, rofecoxib, valdecoxib, lumarocoxib, parecoxib and etoricoxib); glucocorticosteroids (whether administered by topical, oral, intramuscular, intravenous, or intra-articular routes); methotrexate, leflunomide; hydroxychloroquine, d-penicillamine, auranofin or other parenteral or oral gold preparations.

The present invention still further relates to combination therapies of a compound of the invention together with a leukotriene biosynthesis inhibitor, 5-lipoxygenase (5-LO) inhibitor or 5-lipoxygenase activating protein (FLAP) antagonist such as; zileuton; ABT-761; fenleuton; tepoxalin; Abbott-79175; Abbott-85761; N-(5-substituted)-thiophene-2-alkylsulfonamides; 2,6-di-tert-butylphenol hydrazones; methoxytetrahydropyrans such as Zeneca ZD-2138; SB-210661; pyridinyl-substituted 2-cyanonaphthalene compounds such as L-739,010; 2-cyanoquinoline compounds such as L-746,530; MK-591, MK-886, and BAY-x-1005.

The present invention still further relates to combination therapies of a compound of the invention together with a receptor antagonist for leukotrienes (LT)B4, LTC4, LTD4 and LTE4 selected from the group consisting of phenothiazin compound such as L-651,392; amidino compounds such as CGS-25019c; benzoxalamines such as ontazolast; benzenecarboximidamides such as BIIL 284/260; and compounds such as zafirlukast, ablukast, montelukast, pranlukast, verlukast (MK-679), RG-12525, Ro-245913, iralukast (CGP 45715A), and BAY-x-7195.

The present invention still further relates to combination therapies of a compound of the invention together with a phosphodiesterase (PDE) inhibitor such as the methylxanthanines including theophylline and aminophylline; and selective PDE isoenzyme inhibitors including PDE4 inhibitors and inhibitors of isoform PDE4D, and inhibitors of PDE5.

The present invention still further relates to combination therapies of a compound of the invention together with histamine type 1 receptor antagonists such as cetirizine, loratadine, desloratadine, fexofenadine, acrivastine, terfenadine, astemizole, azelastine, levocabastine, chlorpheniramine, promethazine, cyclizine, or mizolastine, which is applied orally, topically or parenterally.

The present invention still further relates to combination therapies of a compound of the invention together with a gastroprotective histamine type 2 receptor antagonist.

The present invention still further relates to combination therapies of a compound of the invention with an antagonist of the histamine type 4 receptor.

The present invention still further relates to combination therapies of a compound of the invention together with an alpha-1 /alpha 2 adrenoceptor agonist, vasoconstrictor sympathomimetic agent, such as propylhexedrine, phenylephrine, phenylpropanolamine, ephedrine, pseudoephedrine, naphazoline hydrochloride, oxymetazoline hydrochloride, tetrahydrozoline hydrochloride, xylometazoline hydrochloride, tramazoline hydrochloride, or ethylnorepinephrine hydrochloride.

The present invention still further relates to combination therapies of a compound of the invention together with an anticholinergic agent including muscarinic receptor (M1, M2 and M3) antagonists such as atropine, hyoscine, glycopyrrolate, ipratropium bromide; tiotropium bromide; oxitropium bromide; pirenzepine; or telenzepine.

The present invention still further relates to combination therapies of a compound of the invention together with a beta-adrenoceptor agonist (including beta receptor subtypes 1-4) such as isoprenaline, salbutamol, formoterol, salmeterol, terbutaline, orciprenaline, bitolterol mesylate, or pirbuterol.

The present invention still further relates to combination therapies of a compound of the invention together with a chromone, such as sodium cromoglycate or nedocromil sodium.

The present invention still further relates to combination therapies of a compound of the invention together with an insulin-like growth factor type I (IGF-1) mimetic.

The present invention still further relates to combination therapies of a compound of the invention together with an inhaled glucocorticoid, such as flunisolide, triamcinolone acetonide, beclomethasone dipropionate, budesonide, fluticasone propionate, ciclesonide, or mometasone furoate.

The present invention still further relates to combination therapies of a compound of the invention together with an inhibitor of matrix metalloproteases, i.e., an inhibitor of stromelysin, collagenase, gelatinase, aggrecanase; especially collagenase-1 (MMP-1), collagenase-2 (MMP-8), collagenase-3 (MMP-13), stromelysin-1 (MMP-3), stromelysin-2 (MMP-10), stromelysin-3 (MMP-11), MMP-9 or MMP-12.

The present invention still further relates to combination therapies of a compound of the invention together with modulators of chemokine receptor function such as antagonists of CCR1, CCR2, CCR2A, CCR2B, CCR3, CCR4, CCR5, CCR6, CCR7, CCR8, CCR9, CCR10 and CCR11 (for the C-C family); CXCR1, CXCR2, CXCR3, CXCR4 and CXCR5 (for the C-X-C family) and CX3CR1 (for the C-X3-C family).

The present invention still further relates to combination therapies of a compound of the invention together with a cytokine or a modulator of cytokine function including agents which act on cytokine signalling pathways, such as alpha-, beta-, and gamma-interferon; interleukins (IL) including IL-1 to IL-15, and interleukin antagonists or inhibitors.

The present invention still further relates to combination therapies of a compound of the invention together with an immunoglobulin (Ig), an Ig preparation, or an antagonist or antibody modulating Ig function such as anti-IgE (omalizumab).

The present invention still further relates to combination therapies of a compound of the invention together with systemic or topically-applied anti-inflammatory agents such as thalidomide or its derivatives, retinoids, dithranol, or calcipotriol.

The present invention still further relates to combination therapies of a compound of the invention together with an antibacterial agent including penicillin derivatives, tetracyclines, macrolides, beta-lactams, flouroquinolones, metronidazole and inhaled aminoglycosides; antiviral agent including acyclovir, famciclovir, valaciclovir, ganciclovir, cidofovir, amantadine, rimantadine, ribavirin; zanamavir and oseltamavir; enzyme inhibitors such as indinavir, nelfinavir, ritonavir, and saquinavir; nucleoside reverse transcriptase inhibitors such as didanosine, lamivudine, stavudine, zalcitabine and zidovudine; or non-nucleoside reverse transcriptase inhibitors such as nevirapine or efavirenz.

The present invention still further relates to combination therapies of a compound of the invention together with agents used for treatment of cancers. Suitable agents to be used in the combination therapies include:
(i) antiproliferative/antineoplastic drugs and combinations thereof, which are used as an anticancer agent, such as alkylating agents (for example cisplatin, carboplatin, cyclophosphamide, nitrogen mustard, melphalan, chlorambucil, busulphan or nitrosoureas); antimetabolites (for example fluoropyrimidines, like 5-fluorouracil and tegafur, antifolates such as raltitrexed, methotrexate, cytosine arabinoside, hydroxyurea, gemcitabine or paclitaxel); antitumour antibiotics (for example anthracyclines, such as adriamycin, bleomycin, doxorubicin, daunomycin, epirubicin, idarubicin, mitomycin-C, dactinomycin or mithramycin); antimitotic agents (for example vinca alkaloids, like vincristine, vinblastine, vindesine and vinorelbine and taxoids, such as taxol and taxotere); or topoisomerase inhibitors (for example epipodophyllotoxins, such as etoposide, teniposide, amsacrine, topotecan or camptothecins);
(ii) cytostatic agents such as antiestrogens (for example tamoxifen, toremifene, raloxifene, droloxifene or iodoxyfene), estrogen receptor down regulators (for example fulvestrant), antiandrogens (for example bicalutamide, flutamide, nilutamide and cyproterone acetate), LHRH antagonists or LHRH agonists (for example goserelin, leuprorelin or buserelin), progestogens (for example megestrol acetate), aromatase inhibitors (for example as anastrozole, letrozole, vorazole or exemestane) and inhibitors of 5α-reductase such as finasteride;
(iii) agents which inhibit cancer cell invasion (for example metalloproteinase inhibitors, such as marimastat or inhibitors of urokinase plasminogen activator receptor function);
(iv) inhibitors of growth factor function, for example such inhibitors include growth factor antibodies, growth factor receptor antibodies (for example the anti erbb2 antibody trastuzumab or the anti erbb1 antibody cetuximab [C225]), farnesyl transferase inhibitors, tyrosine kinase inhibitors or serine/threonine kinase inhibitors; for example inhibitors of the epidermal growth factor family (for example EGFR family tyrosine kinase inhibitors such as N-(3-chloro-4-fluorophenyl)-7-methoxy-6-(3-morpholinopropoxy)quinazolin-4-amine (gefitinib, AZD1839), N-(3-ethynylphenyl)-6,7-bis(2-methoxyethoxy)quinazolin-4-amine (erlotinib, OSI 774) or 6-acrylamido-N-(3-chloro-4-fluorophenyl)-7-(3-morpholinopropoxy)quinazolin-4-amine (CI 1033)); for example inhibitors of the platelet-derived growth factor family; or for example inhibitors of the hepatocyte growth factor family;
(v) antiangiogenic agents such as those which inhibit the effects of vascular endothelial growth factor, (for example the anti vascular endothelial cell growth factor antibody bevacizumab, compounds disclosed in WO 97/22596, WO 97/30035, WO 97/32856 or WO 98/13354), or compounds that work by other mechanisms (for example linomide, inhibitors of integrin αvβ3 function or angiostatin);
(vi) vascular damaging agents such as combretastatin A4 or compounds disclosed in WO 99/02166, WO00/40529, WO 00/41559, WO 01/92224, WO 02/04434 or WO 02/08213;
(vii) antisense therapies, for example those which are directed to the targets listed above, such as ISIS 2503, an anti-ras antisense;
(viii) gene therapy approaches, including for example approaches to replace aberrant genes such as aberrant p53 or aberrant BRCA1 or BRCA2, GDEPT (gene directed enzyme pro-drug therapy) approaches such as those using cytosine deaminase, thymidine kinase or a bacterial nitroreductase enzyme and approaches to increase patient tolerance to chemotherapy or radiotherapy such as multi drug resistance gene therapy; or
(ix) immunotherapy approaches, including for example ex vivo and in vivo approaches to increase the immunogenicity of patient tumour cells, such as exposure with cytokines such as interleukin 2, interleukin 4 or granulocyte macrophage colony stimulating factor (GM-CSF), approaches to decrease T cell anergy, approaches using transplanted immune cells such as cytokine exposed dendritic cells, approaches using cytokine exposed tumour cell lines and approaches using anti idiotypic antibodies.

### Example

The present invention is illustratively described in the following examples, but should be not limited by these examples.

### Example 1

2-Butoxy-7,8-dihydro-9-[(3-[{N-methyl-N-[3-(methoxycarbonylmethyl)benzyl]}amino]propoxy)benzyl]-8-oxoadenine

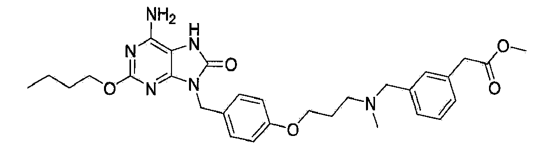

### Step (i)

9-(4-Benzyloxybenzyl)-2-butoxyadenine

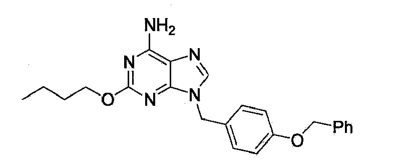

To a suspension of 2-butoxyadenine 7.2g (34.8mmol) in DMF 150ml was added potassium carbonate 4.8g (34.8mmol) and the mixture was stirred at 60°C for 2 hours. After being cooled, thereto was added 4-benzyloxybenzyl chloride 9.7g (41.8mmol) and the mixture was stirred for 20 hours. After removal of the solvent, to the residue was added water and the mixture was extracted with chloroform. The organic layer was dried over anhydrous magnesium sulfate, and concentrated in vacuo. The residue was purified by silica gel column chromatography to give the object compound 10.9g as a pale yellow solid. Yield 78%
¹H NMR (CDCl₃) δ7.58 (1H, s), 7.43-7.32 (5H, m), 7.25 (2H, d, J = 8.6 Hz), 6.94 (2H, d, J = 8.6 Hz), 5.93 (2H, brs), 5.20 (2H, s), 5.05 (2H, s), 4.35 (2H, t, J = 6.6 Hz), 1.79 (2H, tt, J = 7.6 Hz, 6.6 Hz), 1.50 (2H, tq, J = 7.6 Hz, 7.4 Hz), 0.97 (3H, t, J = 7.4 Hz).

### Step (ii)

9-(4-Benzyloxybenzyl)-8-bromo-2-butoxyadenine

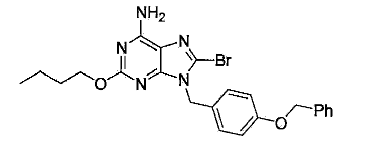

The compound 10.9g (27.0mmol) obtained in step (i) was dissolved in chloroform 250ml and thereto was added sodium acetate 4.0g (48.6mmol). After being cooled to 0°C, thereto was dropped bromine 6.47g (40.5mmol) and the mixture was stirred at room temperature for 1 hour. After being cooled to 0°C, thereto were added saturated aqueous sodium hydrogencarbonate, saturated aqueous sodium thiosulfate and the mixture was extracted with chloroform. The organic layer was dried over anhydrous magnesium sulfate and concentrated in vacuo to give the object compound 13.0g as a pale yellow solid. Yield 100%
¹H NMR (CDCl₃) δ7.42-7.31 (7H, m), 6.91 (2H, d, J = 8.7 Hz), 6.08 (2H, brs), 5.23 (2H, s), 5.03 (2H, s), 4.35 (2H, t, J = 6.7 Hz), 1.79 (2H, tt, J = 7.5 Hz, 6.7 Hz), 1.51 (2H, tq, J = 7.5 Hz, 7.3 Hz), 0.97 (3H, t, J = 7.3 Hz).

### Step (iii)

9-(4-Benzyloxybenzyl)-2-butoxy-8-methoxyadenine

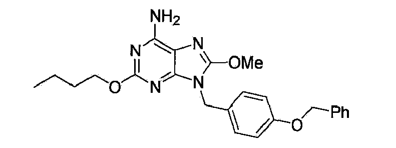

To a suspension of the compound 13.0g (27.0mmol) obtained in step (ii) in methanol 400ml was added 28% sodium methoxide/methanol 100ml, and the mixture was refluxed under stirring for 3 hours. After being cooled to 0°C, the mixture was neutralized with acetic acid. After removal of the solvent, to the residue was added water and the resulting solid was filtered. The solid was dried and purified by silica gel column chromatography to give the object compound 9.05g as a white solid. Yield 77%
¹H NMR (CDCl₃) δ7.42-7.28 (7H, m), 6.91 (2H, d, J = 8.6 Hz), 5.29 (2H, brs), 5.03 (2H, s), 4.32 (2H, t, J = 6.7 Hz), 4.10 (3H, s), 1.78 (2H, tt, J = 7.5 Hz, 6.7 Hz), 1.50 (2H, tq, J = 7.5 Hz, 7.4 Hz), 0.97 (3H, t, J = 7.4 Hz).

### Step (iv)

2-Butoxy-9-(4-hydroxybenzyl)-8-methoxyadenine

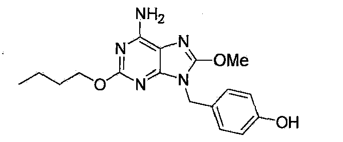

To a solution of the compound 9.04g (20.9mmol) obtained in step (iii) in THF 150ml was added 20% Pd(OH)₂/C 2.0g, and the mixture was stirred under an atmosphere of hydrogen for 9 hours. After filtering over Celite, the filtrate was concentrated and the resulting solid was washed with hexane to give the object compound 7.18g as a white solid. Yield 100%
¹H NMR (DMSO-d₆) δ9.44 (1H, s), 7.09 (2H, d, J = 8.5 Hz), 6.83 (2H, brs), 6.69 (2H, d, J = 8.5 Hz), 4.89 (2H, s), 4.17 (2H, t, J = 6.6 Hz), 4.03 (3H, s), 1.65 (2H, tt, J = 7.5 Hz, 6.6 Hz), 1.40 (2H, tq, J = 7.5 Hz, 7.3 Hz), 0.92 (3H, t, J = 7.3 Hz).

### Step (v)

9-[4-(3-Bromopropoxy)benzyl]-2-butoxy-8-methoxyadenine

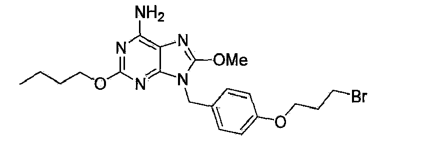

To a solution of the compound 1.50g (4.37mmol) obtained in step (iv) in DMF 50ml were added 1,3-dibromopropane 4.4ml (43.7mmol) and potassium carbonate 0.60g (4.37mmol), and the mixture was stirred at 70°C for 6 hours. After removal of the solvent, thereto was added water and the mixture was extracted with chloroform. The organic layer was dried over anhydrous magnesium sulfate and concentrated in vacuo. The residue was purified by silica gel column chromatography to give the object compound 0.48g as a white solid. Yield 24%
¹H NMR (CDCl₃) δ7.29 (2H, d, J = 8.6 Hz), 6.81 (2H, d, J = 8.6 Hz), 5.26 (2H, brs), 5.03 (2H, s), 4.31 (2H, t, J = 6.6 Hz), 4.09 (3H, s), 4.07 (2H, t, J = 5.8 Hz), 3.58 (2H, t, J = 6.4 Hz), 2.29 (2H, tt, J = 6.4 Hz, 5.8 Hz), 1.78 (2H, tt, J = 7.5 Hz, 6.6 Hz), 1.50 (2H, tq, J = 7.5 Hz, 7.4 Hz), 0.97 (3H, t, J = 7.4 Hz).

### Step (vi)

2-Butoxy-8-methoxy-9-[4-(3-methylaminopropoxy)benzyl]adenine

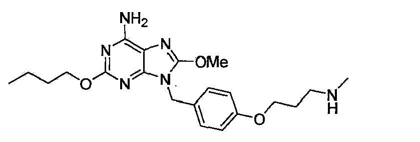

To a solution of the compound 0.15g (0.32mmol) obtained in step (v) in THF 3ml was added 30% methylamine/methanol 3ml and the mixture was stirred at room temperature for 9 hours. After removal of the solvent, the residue was purified by silica gel column chromatography to give the object compound 0.13g as a white solid. Yield 100%
¹H NMR (CDCl₃) δ7.26 (2H, d, J = 8.6 Hz), 6.78 (2H, d, J = 8.6 Hz), 5.51 (2H, brs), 5.00 (2H, s), 4.28 (2H, t, J = 6.8 Hz), 4.09 (3H, s), 4.03 (2H, t, J = 5.8 Hz), 3.20 (2H, t, J = 7.4 Hz), 2.72 (3H, s), 2.37 (2H, tt, J = 7.4 Hz, 5.8 Hz), 1.76 (2H, tt, J = 7.6 Hz, 6.8 Hz), 1.47 (2H, tq, J = 7.6 Hz, 7.4 Hz), 0.96 (3H, t, J = 7.4 Hz).

### Step (vii)

2-Butoxy-9-[(3-[{N-methyl-N-[3-(methoxycarbonylmethyl)benzyl}amino]propoxy)benzyl]-8-methoxyadenine

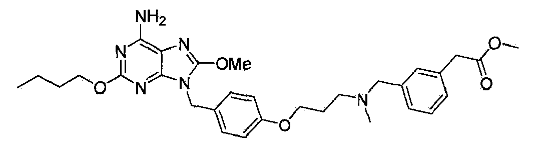

To a solution of the compound 126mg (0.30mmol) obtained in step (vi) in acetonitrile 15ml were added methyl 3-(bromomethyl)phenylacetate 89mg (0.36mmol) and potassium carbonate 62mg (0.45mg), and the mixture was stirred at room temperature for 3 hours. After removal of the solvent, the residue was purified by silica gel column chromatography to give the object compound 79mg as a colorless oil. Yield 45%
¹H NMR (CDCl₃) δ7.29-7.19 (6H, m), 6.79 (2H, d, J = 8.7 Hz), 5.13 (2H, brs), 5.02 (2H, s), 4.31 (2H, t, J = 6.7 Hz), 4.09 (3H, s), 3.98 (2H, t, J = 6.4 Hz), 3.67 (3H, s), 3.57 (2H, s), 3.48 (2H, s), 2.52 (2H, t, J = 7.0 Hz), 2.20 (3H, s), 1.95 (2H, tt, J = 7.0 Hz, 6.4 Hz), 1.82-1.72 (2H, m), 1.58-1.46 (2H, m), 0.97 (3H, t, J = 7.4 Hz).

### Step (viii)

2-Butoxy-7,8-dihydro-9-[(3-[{N-methyl-N-[3-(methoxycarbonylmethyl)benzyl]}amino]propoxy)benzyl]-8-oxoadenine

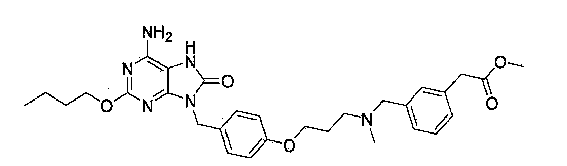

The compound 79mg (0.14mmol) obtained in step (vii) was dissolved in 4M hydrochloric acid/methanol 10ml and the solution was stirred for 12 hours at room temperature. After concentration to the residue was added saturated sodium hydrogencarbonate and the mixture was neutralized. The resulting solid was filtered and washed with water to give the titled compound 47mg as a white solid. Yield 61 %
¹H NMR (DMSO-d₆) δ9.98 (1H, brs), 7.24-7.07 (6H, m), 6.83 (2H, d, J = 8.7 Hz), 6.45 (2H, brs), 4.77 (2H, s), 4.14 (2H, t, J = 6.6 Hz), 3.95 (2H, t, J = 6.3 Hz), 3.59 (2H, s), 3.57 (3H, s), 3.43 (2H, s), 2.43 (2H, t, J = 6.9 Hz), 2.11 (3H, s), 1.86 (2H, tt, J = 6.9 Hz, 6.3 Hz), 1.70-1.59 (2H, m), 1.42-1.33 (2H, m), 0.91 (3H, t, J = 7.4 Hz).

### Example 2

2-Butoxy-7,8-dihydro-9-[(3-[{N-[3-(carboxymethyl)benzyl]-N-methyl}amino]propoxy)benzyl]-8-oxoadenine

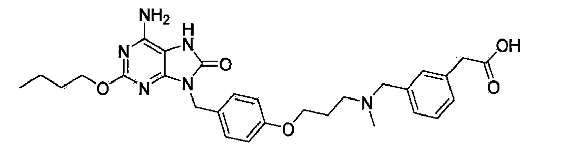

To the compound 29mg (0.053mmol) obtained by example 1 was added aqueous 2M sodium hydroxide 2.5ml and the mixture was refluxed under stirring for 10 minutes. After being cooled to 0°C, the mixture was neutralized with concentrated hydrochloric acid and the resulting solid was filtered and washed with water to give the titled compound 18mg as a white solid. Yield 63%
¹H NMR (DMSO-d₆) δ10.66 (1H, brs), 7.21 (2H, d, J = 8.6 Hz), 7.18-7.12 (4H, m), 6.83 (2H, d, J = 8.6 Hz), 6.64 (2H, brs), 4.76 (2H, s), 4.14 (2H, t, J = 6.6 Hz), 3.95 (2H, t, J = 6.3 Hz), 3.40 (2H, s), 3.39 (2H, s), 2.43 (2H, t, J = 7.0 Hz), 2.09 (3H, s), 1.85 (2H, tt, J = 7.0 Hz, 6.3 Hz), 1.62 (2H, tt, J = 7.5 Hz, 6.6 Hz), 1.36 (2H, tq, J = 7.5 Hz, 7.4 Hz), 0.90 (3H, t, J = 7.4 Hz).

### Example 3

2-Butoxy-7,8-dihydro-9-([3-{N-[3-(methoxycarbonylmethyl)benzyl]amino}propoxy]benzyl)-8-oxoadenine

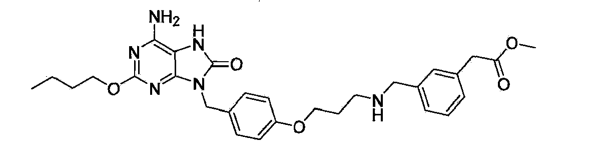

### Step (i)

2-Butoxy-8-methoxy-9-[4-(3-phthalimidopropoxy)benzyl]adenine

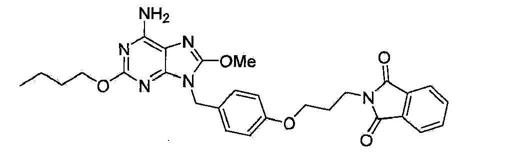

To a solution of the compound 0.35g (1.03mmol) obtained in step (iv) of example 1 in DMF 10ml were added N-(3-bromopropyl)phthalimide 0.55g (2.05mmol), potassium carbonate 0.14g (1.03mmol) and potassium iodide 0.33g (2.00mmol) and the mixture was stirred at 50°C for 10 hours. After removal of the solvent, to the residue was added water and the mixture was extracted with chloroform. The organic layer was dried over anhydrous magnesium sulfate and concentrated in vacuo. The residue was purified by silica gel column chromatography to give the object compound 0.50g as a white solid. Yield 92%
¹H NMR (CDCl₃) 87.84-7.80 (2H, m), 7.73-7.68 (2H, m), 7.24 (2H, d, J = 8.4 Hz), 6.73 (2H, d, J = 8.4 Hz), 5.23 (2H, brs), 5.01 (2H, s), 4.31 (2H, t, J = 6.7 Hz), 4.09 (3H, s), 3.99 (2H, t, J = 6.0 Hz), 3.89 (2H, t, J = 6.9 Hz), 2.16 (2H, tt, J = 6.9 Hz, 6.0 Hz), 1.78 (2H, tt, J = 7.5 Hz, 6.7 Hz), 1.53 (2H, tq, J = 7.5 Hz, 7.4 Hz), 0.97 (3H, t, J = 7.4 Hz).

### Step (ii)

9-[4-(3-Aminopropoxy)benzyl]-2-butoxy-8-methoxyadenine

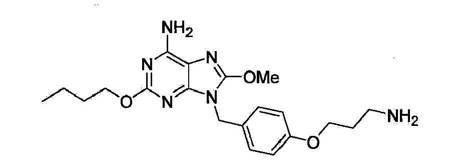

The compound 0.50g (0.94mmol) obtained in step (i) was suspended in ethanol 30ml and the suspension was completely dissolved by refluxing under heating for 20 minutes. Thereto was added hydrazine monohydrate 1ml and the mixture was stirred for 1 hour. After being cooled to room temperature, the resulting solid was filtered off and the filtrate was concentrated. To the residue was added aqueous sodium hydrogencarbonate and the mixture was extracted with chloroform/ ethanol (3/ 1). The organic layer was dried over anhydrous magnesium sulfate and concentrated in vacuo to give the object compound 0.34g as a white solid. Yield 91%
¹H NMR (DMSO-d₆) δ7.18 (2H, d, J = 8.6 Hz), 6.86 (2H, d, J = 8.6 Hz), 6.82 (2H, brs), 4.95 (2H, brs), 4.17 (2H, t, J = 6.6 Hz), 4.03 (3H, s), 3.97 (2H, t, J = 6.4 Hz), 2.65 (2H, t, J = 6.7 Hz), 1.74 (2H, tt, J = 6.7 Hz, 6.4 Hz), 1.65 (2H, tt, J = 7.5 Hz, 6.6 Hz), 1.40 (2H, tq, J = 7.5 Hz, 7.4 Hz), 0.92 (3H, t, J = 7.4 Hz).

### Step (iii)

3-(Methoxycarbonylmethyl)benzaldehyde

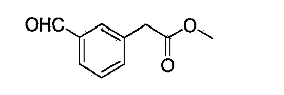

To a solution of methyl 3-(bromomethyl)phenylacetate 5.0g (20.7mmol) in DMSO 15ml was added portion wise N-methylmorpholin-N-oxide 3.63g (31mmol). After stirring at room temperature for 1.5 hours, to the mixture was added water and the mixture was extracted with ethyl acetate. The organic layer was washed twice with saturated brine, dried over anhydrous magnesium sulfate and concentrated in vacuo. The residue was purified by silica gel column chromatography to give the object compound 1.74g as a colorless oil. Yield 47%
¹H NMR (CDCl₃) δ10.00 (1H, s), 7.82-7.79 (2H, m), 7.58-7.49 (2H, m), 3.72 (2H, s), 3.72 (3H, s).

### Step (iv)

2-Butoxy-9-([3-{N-[3-(methoxycarbonylmethyl)benzyl]amino}propoxy]benzyl)-8-methoxyadenine

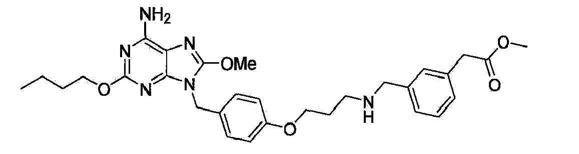

The compound 0.20g (0.51mmol) obtained in step (ii) and the compound 0.14g (0.76mmol) obtained in step (iii) were dissolved in methanol 10ml and the solution was stirred at room temperature for 1 hour. Thereto was added sodium cyanoborohydride 0.13g (2.5mmol), followed by stirring for 15 hours. Thereto was added acetic acid 0.85ml (15mmol) and the mixture was stirred for 10 minutes. After removal of the solvent, to the residue was added aqueous sodium hydrogencarbonate and the mixture was extracted with chloroform/ethanol (3/ 1). The organic layer was dried over anhydrous magnesium sulfate, concentrated in vacuo and the residue was purified by silica gel column chromatography to give the object compound 0.17g as a colorless oil. Yield 58%
¹H NMR (CDCl₃) δ7.30-7.15 (6H, m), 6.80 (2H, d, J = 8.7 Hz), 5.23 (2H, brs), 5.02 (2H, s), 4.30 (2H, t, J = 6.7 Hz), 4.08 (3H, s), 4.00 (2H, t, J = 6.1 Hz), 3.79 (2H, s), 3.67 (3H, s), 3.60 (2H, s), 2.81 (2H, t, J = 6.9 Hz), 2.00 (1H, brs), 1.97 (2H, tt, J = 6.9 Hz, 6.1 Hz), 1.77 (2H, tt, J = 7.5 Hz, 6.7 Hz), 1.51 (2H, tq, J = 7.5 Hz, 7.4 Hz), 0.97 (3H, t, J = 7.4 Hz).

### Step (v)

2-Butoxy-7,8-dihydro-9-([3-{N-[3-(methoxycarbonylmethyl)benzyl] amino}propoxy]benzyl)-8-oxoadenine

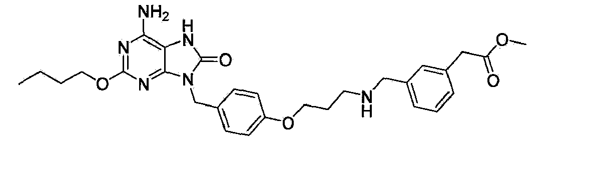

To the compound 0.17g (0.30mmol) obtained in step (iv) were added methanol 10ml and concentrated sulfuric acid 0.2ml, and the mixture was refluxed under stirring for 6 hours. After being cooled to 0°C, thereto was added aqueous sodium hydrogencarbonate. The resulting solid was filtered, and washed with water to give the titled compound 0.15g as a white solid. Yield 90%
¹H NMR (DMSO-d₆) δ9.97 (1H, brs), 7.25-7.19 (5H, m), 7.12-7.07 (1H, m), 6.84 (2H, d, J = 8.8 Hz), 6.44 (2H, brs), 4.76 (2H, s), 4.14 (2H, t, J = 6.6 Hz), 3.98 (2H, t, J = 6.4 Hz), 3.65 (2H, s), 3.62 (2H, s), 3.58 (3H, s), 2.60 (2H, t, J = 6.8 Hz), 1.83 (2H, tt, J = 6.8 Hz, 6.4 Hz), 1.63 (2H, tt, J = 7.4 Hz, 6.6 Hz), 1.38 (2H, tq, J = 7.4 Hz, 7.4 Hz), 0.91 (3H, t, J = 7.4 Hz).

### Example 4

2-Butoxy-7,8-dihydro-9-([3-{N-[3-(carboxymethyl)benzyl]amino}propoxy]benzyl)-8-oxoadenine

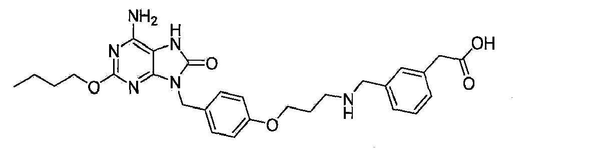

Using the compound 30mg (0.054mmol) obtained by example 3, in the same manner as comparative example 1, there was obtained the titled compound 22mg as a white solid. Yield 76%
¹H NMR (DMSO-d₆) δ10.07 (1H, brs), 7.24-7.17 (5H, m), 7.11-7.09 (1H, m), 6.85 (2H, d, J = 8.5 Hz), 6.47 (2H, brs), 4.76 (2H, s), 4.14 (2H, t, J = 6.6 Hz), 3.98 (2H, t, J = 6.4 Hz), 3.69 (2H, s), 3.49 (2H, s), 2.64 (2H, t, J = 6.6 Hz), 1.85 (2H, tt, J = 6.6 Hz, 6.4 Hz), 1.63 (2H, tt, J = 7.4 Hz, 6.6 Hz), 1.38 (2H, tq, J = 7.4 Hz, 7.4 Hz), 0.90 (3H, t, J = 7.4 Hz).

### Example 5

2-Butoxy-7,8-dihydro-9-(4-{N-(3-hydroxypropyl)-N-[3-(methoxylcarbonylmethyl)benzyl]aminomethyl}benzyl)-8-oxoadenine

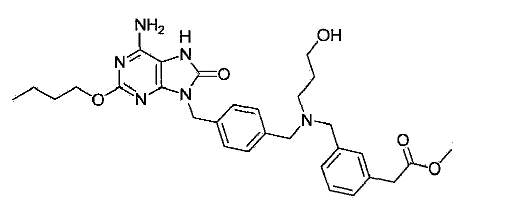

### Step (i)

2-Butoxy-9-[4-(hydroxymethyl)benzyl]-8-methoxyadenine

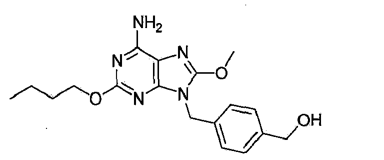

To a solution of 2-butoxy-8-methoxyadenine trifluoroacetic acid salt 10g (42.1mmol) in DMF (90ml) were added potassium carbonate 17.5g (126.4mmol) and 4-(hydroxymethyl)benzyl chloride 7.3g (46.4mmol), and the mixture was stirred at room temperature for 18 hours. After filtering off carbonate in the reaction system, the filtrate was concentrated. To the residue was added water and the mixture was extracted with 5% methanol-chloroform (800ml). The organic layer was washed with water and saturated brine, successively, and dried over sodium sulfate. To the residue were added chloroform 125ml, methanol 25ml and diethyl ether 125ml, and insoluble materials were removed by filtration. The filtrate was concentrated in vacuo and to the residue was added diethyl ether 150ml. The resulting white solid was taken by filtration and dried to give the object compound 7.2g (20.1mmol) as a white solid. Yield 71%
¹H NMR (DMSO-d₆) δ 7.26 (2H, d, J = 8.2 Hz), 7.19 (2H, d, J = 8.2 Hz), 6.47 (2H, brs), 5.15 (1H, t, J = 5.6 Hz), 5.01 (2H, s), 4.44 (2H, d, J = 5.6 Hz), 4.17 (2H, t, J = 6.6 Hz), 4.03 (3H, s), 1.68-1.59 (2H, m), 1.44-1.34 (2H, m), 0.91 (3H, t, J = 7.4 Hz).

### Step (ii)

2-Butoxy-7,8-dihydro-9-[4-(chloromethyl)benzyl]-8-oxoadenine

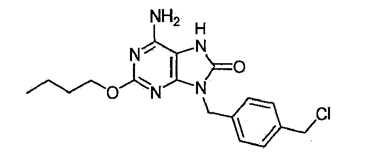

To a suspension of the compound 7.1g (19.6mmol) obtained in step (i) in dichloromethane 140ml was added thionyl chloride 4.3ml, and the mixture was stirred at 50°C for 2 hours. Thereto was added toluene 30ml and the solvent was removed by distillation. To the residue was added further toluene 100ml and the solvent was removed by distillation. The residue was dried in vacuo to give the object compound 7.2g (19.6mmol) as a yellowish white solid. Yield 99%
¹H NMR (DMSO-d₆) δ 7.39 (2H, d, J = 8.2 Hz), 7.30 (2H, d, J = 8.2 Hz), 4.88 (2H, s), 4.73 (2H, s), 4.21 (2H, t, J = 6.6 Hz), 1.68-1.59 (2H, m), 1.43-1.32 (2H, m), 0.90 (3H, t, J = 7.4 Hz).

### Step (iii)

2-Butoxy-7,8-dihydro-9-{4-[N-(3-hydroxypropyl)aminomethyl]benzyl}-8-oxoadenine

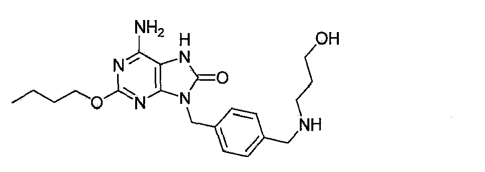

To a solution of the compound 7.2g (19.6mmol) obtained in step (ii) in DMF 140ml was added aminopropanol 15g (199mmol) and the mixture was stirred at room temperature for 15 hours. Thereto was added water 320ml and the resulting solid was taken by filtration and dried to give the object compound 7.8g (19.6mmol) as a yellowish white solid. Yield 99% ¹H NMR (DMSO-d₆) δ 7.25 (2H, bs) (2H, d, J = 8.2 Hz), 7.22 (2H, d, J = 8.2 Hz), 6.57 (2H, brs), 4.81 (2H, s), 4.13 (2H, t, J = 6.6 Hz), 3.61 (2H, s), 3.45 (2H, t, J = 6.3 Hz), 1.66-1.58 (2H, m), 1.58-1.51 (2H, m), 2.52-2.48 (2H, m), 1.42-1.32 (2H, m), 0.90 (3H, t, J = 7.4 Hz).

### Step (iv)

2-Butoxy-7, 8-dihydro-9-(4-{N-(3-hydroxypropyl)-N-[3-(methoxycarbonylmethyl)benzyl]aminomethyl}benzyl)-8-oxoadenine

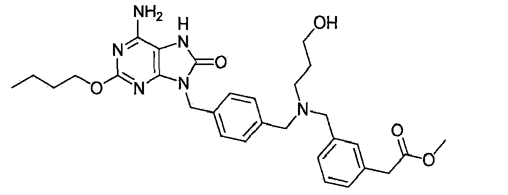

To a solution of the compound 300mg (0.70mmol) obtained in step (iii) in DMF 7ml were added potassium carbonate 116mg and 3-(methoxycarbonylmethyl)benzyl bromide 187mg (0.77mmol) and the mixture was stirred at room temperature for 3 hours. After removal of carbonate in the reaction system, the filtrate was concentrated. To the residue was added water and the mixture was extracted with 5%methanol-chloroform (200ml). The organic layer was washed with water, and brine, successively, and dried over sodium sulfate. After concentration, the residue was purified by silica gel column chromatography and dried in vacuo to give the titled compound 223mg (0.38mmol) as yellow liquid. Yield 54%
¹H NMR (DMSO-d₆) δ 9.96 (1H, brs), 7.30-7.19 (7H, m), 7.11 (1H, d, J = 7.2 Hz), 6.45 (2H, brs), 4.83 (2H, s), 4.31 (1H, ,brs), 4.13 (2H, t, J = 6.6 Hz), 3.65 (2H, s), 3.58 (2H, s), 3.48 (2H, s), 3.46 (2H, s), 3.39-3.33 (2H, m), 2.42-2.36 (2H, m), 1.65-1.57 (4H, m), 1.41-1.31 (2H, m), 0.89 (3H, t, J = 7.4 Hz).

### Example 6

2-Butoxy-9-(4-{N-(3-chloropropyl)-N-[3-(methoxycarbonylmethyl) benzyl]ammomethyl}benzyl)-7,8-dihydro-8-oxoadenine

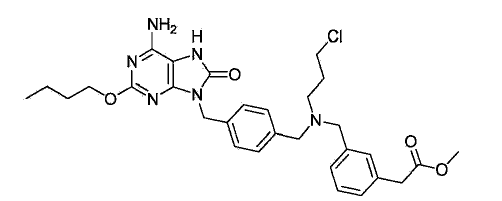

To a suspension of the compound 1.42g (2.52mmol) obtained by example 5 in dichloromethane 30ml was added thionyl chloride 552µl and the mixture was stirred at 50°C for 2 hours. Thereto was added toluene 15ml and the solvent was removed by distillation. To the residue was added further toluene 30ml and the solvent was removed by distillation. The residue was dried in vacuo to give the titled compound 1.46g (2.52mmol) as a yellowish white solid. Yield 99%

### Example 7

2-Butoxy-7,8-dihydro-9-(4-{N-[3-(methoxycarbonylmethyl)benzyl]-N-(3-morpholin-4-ylpropyl)aminomethyl}benzyl)-8-oxoadenine

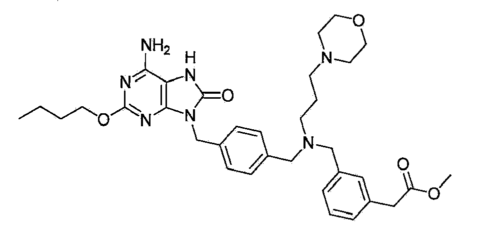

To a solution of the compound 1.46g (2.52mmol) obtained by example 6 in DMF 25ml were added morpholine 2.21ml (25.2mmol) and potassium iodide 419mg (2.52mmol) and the mixture was stirred at 75°C for 2.5 hours. After filtration, the filtrate was concentrated and thereto was added water, followed by extraction with 5% methanol-chloroform (200ml). The organic layer was washed with water and saturated brine, successively and dried over sodium sulfate. After concentration, the residue was purified by silica gel column chromatography and dried in vacuo to give the titled compound 1.2g (1.90mmol) as a white solid. Yield 75%
¹H NMR (DMSO-d₆) δ 9.93 (1H, brs), 7.29-7.20 (7H, m), 7.11 (1H, d, J = 7.2 Hz), 6.43 (2H, brs), 4.82 (2H, brs), 4.13 (2H, t, J = 6.6 Hz), 3.65 (2H, s), 3.58 (3H, s), 3.49 (2H, s), 3.46 (2H, s), 3.42 (4H, t, J = 4.5 Hz), 2.34 (2H, t, J = 6.9 Hz), 2.21-2.14 (6H, m), 1.65-1.52 (4H, m), 1.41-1.31 (2H, m), 0.89 (3H, t, J = 7.4 Hz).

### Example 8

2-Butoxy-7,8-dihydro-9-(4-{N-[3-(hydroxycarbonylmethyl)benzyl]-N-(3-morpholin-4-ylpropyl)aminomethyl}benzyl)-8-oxoadenine

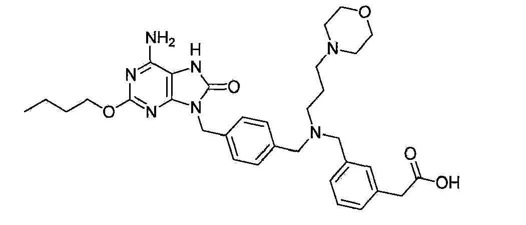

In the same manner as example 2, there was obtained the titled compound as a white solid. Yield 57%
¹H NMR (DMSO-d₆) δ 9.94 (1H, brs), 7.29-7.19 (7H, m), 7.11 (1H, d, J = 7.2 Hz), 6.43 (2H, brs), 4.82 (2H, brs), 4.13 (2H, t, J = 6.6 Hz), 3.54 (2H, s), 3.49 (2H, s), 3.45 (2H, s), 3.45-3.41 (4H, m), 2.34 (2H, t, J = 6.9 Hz), 2.26-2.15 (6H, m), 1.65-1.55 (4H, m), 1.41-1.32 (2H, m), 0.89 (3H, t, J = 7.4 Hz).

### Example 9

2-Butoxy-7,8-dihydro-9-[4-(N-{2-[3-(methoxycarbonylmethyl)phenoxy]ethyl}-N-methylaminomethyl)benzyl]-8-oxoadenine

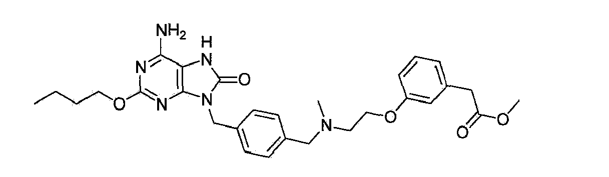

In the same manner as step (iii) of example 5, there was obtained the titled compound as a white solid. Yield 33%
¹H NMR (DMSO-d₆) δ 10.0 (1H, brs), 7.27-7.22 (4H, m), 7.19 (1H, t, J = 7.8 Hz), 6.82-6.79 (3H, m), 6.46 (2H, brs), 4.83 (2H, s), 4.13 (2H, t, J = 6.6 Hz), 4.05 (2H, t, J = 5.8 Hz), 3.62 (2H, s), 3.60 (3H, s), 3.52 (2H, s), 2.70 (2H, t, J = 5.8 Hz), 2.20 (3H, s), 1.65-1.58 (2H, m), 1.39-1.33 (2H, m), 0.89 (3H, t, J=7.4Hz).

### Example 10

2-Butoxy-7,8-dihydro-9-[4-(N-{2-[3-(hydroxycarbonylmethyl)phenoxy]ethyl}-N-methylaminomethyl)benzyl]-8-oxoadenine

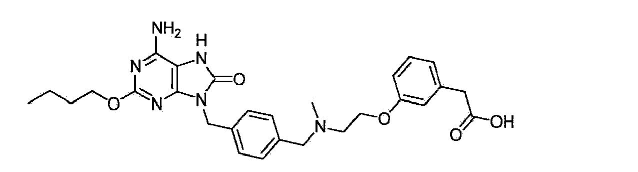

In the same manner as example 2, there was obtained the titled compound as a white solid. Yield 50%
¹H NMR (DMSO-d₆) δ 11.4 (1H, brs), 9.98 (1H, brs), 7.36-7.27 (3H, m), 7.21 (1H, t, J = 7.8 Hz), 6.85-6.81 (3H, m), 6.47 (2H, brs), 4.86 (2H, s), 4.19-4.15 (2H, m), 4.12 (2H, t, J = 6.6 Hz), 4.00-3.82 (2H, m), 3.52 (3H, brs), 3.45-3.19 (2H, m), 3.19-2.91 (2H, m), 1.65-1.58 (2H, m), 1.39-1.34 (2H, m), 0.89 (3H, t, J = 7.4 Hz).

### Example 11

2-Butoxy-7,8-dihydro-9-[4-(N-{2-[2-methoxy-5-(methoxycarbonylmethyl) phenoxy] ethyl}-N-methylaminomethyl)benzyl]-8-oxoadenine

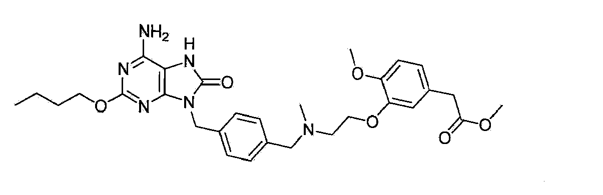

In the same manner as step (iii) of example 5, there was obtained the titled compound as a white solid. Yield 38%
¹H NMR (DMSO-d₆) δ 10.02 (1H, brs), 9.76 (1H, brs), 7.74-7.49 (2H, m), 7.40-7.36 (2H, m), 6.96-6.90 (1H, m), 6.90-6.85 (1H, m),6.48 (2H, brs), 4.89 (2H, s), 4.55-4.42 (1H, m), 4.38-4.21 (3H, m), 3.73 (3H, s), 3.60 (3H, s), 3.58 (2H, s), 3.52-3.38 (2H, m), 2.81 (3H, brs), 1.65-1.58 (2H, m), 1.39-1.34 (2H, m), 0.90 (3H, t, J = 7.4 Hz).

### Example 12

2-Butoxy-7,8-dihydro-9-{6-[4-(3-methoxycarbonylmethylbenzyl)aminobutoxy]pyridin-3-ylmethyl}-8-oxoadenine

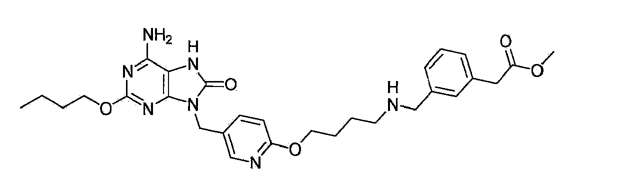

### Step (i)

2-Butoxy-9-(6-chloropyridin-3-ylmethyl) adenine

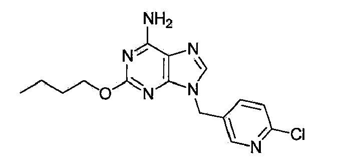

To a solution of 2-butoxyadenine 2.55g (12.3mmol) in DMF (50ml) were added potassium carbonate 5.11g (37.0mmol) and 2-chloro-5-chloromethylpyridine 2.0g (12.3mmol), and the mixture was stirred at room temperature for 14 hours. After removal of the solvent, to the residue was added water 80ml and the mixture was extracted with 5% methanol-chloroform (100ml). The organic layer was washed with water and saturated brine, successively, and dried over magnesium sulfate. After concentration in vacuo, the residue was purified by silica gel column chromatography to give the object compound 2.95g as a pale brown solid. Yield 72%
¹H NMR (CDCl₃) δ 8.46 (1H, d, J= 2.3 Hz), 7.69 (1H, s), 7.64 (1H, dd, J= 8.6 Hz, 2.3 Hz), 7.32 (1H, d, J= 8.6 Hz), 6.14 (2H, brs), 5.29 (2H, s), 4.35 (2H, t, J= 6.8 Hz), 1.81-1.74 (2H, m), 1.54-1.45 (2H, m), 0.97 (3H, t, J= 7.4 Hz).

### Step (ii)

2-Butoxy-9-[6-(4-hydroxybutoxy)pyridin-3-yl]metyladenine

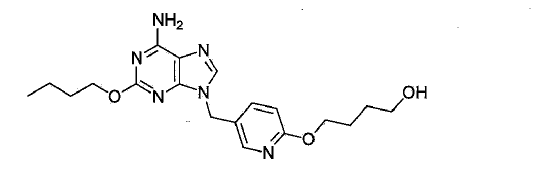

Sodium metal 350mg (15.0mmol) was dissolve in 1,4-butanediol (10ml) to prepare an alkoxide. Thereto was added the compound 1.0g (3.00mmol) prepared in step (i) and the mixture was refluxed at 130°C for 3 hours. After being cooled to 0°C, thereto was added water 50ml. The mixture was adjusted with 1N aqueous hydrochloric acid to pH9 and extracted with 30% methanol-chloroform (100ml). The organic layer was washed with water and saturated brine, successively and dried over magnesium sulfate. After concentration in vacuo, the residue was purified by silica gel column chromatography to give the object compound 892mg as a white solid. Yield 83%
¹H NMR (CDCl₃) δ 8.18 (1H, d, J= 2.2 Hz), 7.59 (1H, s), 7.56 (1H, dd, J= 8.6 Hz, 2.2 Hz), 6.69 (1H, d, J= 8.6 Hz), 5.60 (2H, brs), 5.19 (2H, s), 4.34-4.29 (4H, m), 3.72 (2H, q, 6.0 Hz), 1.91-1.70 (6H, m), 1.54-1.49 (2H, m), 0.98 (3H, t, J= 7.4 Hz).

### Step (iii)

2-Butoxy-8-bromo-9-[6-(4-hydroxybutoxy)pyridin-3-yl]adenine

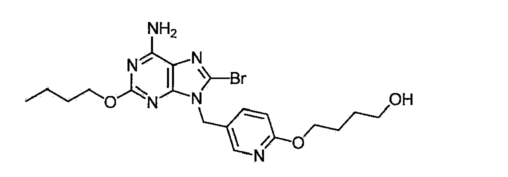

To a solution of the compound 892mg (2.49mmol) obtained in step (ii) and sodium acetate 613mg (7.47mmol) in chloroform (30ml) was added bromine 476mg and the mixture was stirred for 1 hour. Thereto was added aqueous sodium thiosulfate (10ml) and the mixture was neutralized with aqueous sodium hydrogencarbonate and extracted with chloroform (100ml). The organic layer was washed with water and brine, successively and dried over magnesium sulfate. After removal of the solvent, the residue was purified by silica gel column chromatography to give the object compound 919mg as a white solid. Yield 84%
¹H NMR (CDCl₃) δ 8.26 (1H, d, J= 2.3 Hz), 7.66 (1H, dd, J= 8.5 Hz, 2.3 Hz), 6.67 (1H, d, J= 8.5 Hz), 5.66 (2H, brs), 5.22 (2H, s), 4.36-4.28 (4H, m), 3.72 (2H, t, 5.5Hz), 1.87-1.70 (6H, m), 1.54-1.50 (2H, m), 0.98 (3H, t, J= 7.4 Hz).

### Step (iv)

2-Butoxy-9- [6-(4- hydroxybutoxy)pyridin-3-yl] -8-methoxyadenine

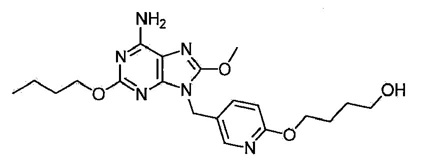

To a solution of the compound 919mg (2.10mmol) obtained in step (iii) in methanol (20 ml) was added 5 N aqueous sodium hydroxide (20 ml) and the mixture was refluxed at 100°C for 4 hours. After neutralization with hydrochloric acid and extraction with 20% methanol-chloroform (100ml), the organic layer was washed with brine, dried over magnesium sulfate and concentrated in vacuo to give the object compound 815mg as a white solid. Yield 99%
¹H NMR (CDCl₃) δ 8.19 (1H, d, J= 2.4 Hz), 7.64 (1H, dd, J= 8.6 Hz, 2.4 Hz), 6.66 (1H, d, J= 8.6 Hz), 5.21 (2H, brs), 5.01 (2H, s), 4.32-4.29 (4H, m), 4.10 (3H, s), 3.71 (2H, t, 6.3 Hz), 1.87-1.70 (6H, m), 1.54-1.50 (2H, m), 0.98 (3H, t, J= 7.4 Hz).

### Step (v)

Methyl 3-bromophenylacetate

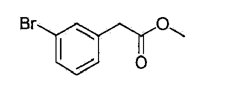

To 3-bromophenylacetic acid 10.0g (46.5mmol) were added methanol 150ml and sulfuric acid 5ml and the mixture was refluxed for 3 hours. After neutralization with aqueous ammonia and removal of the solvent by distillation, to the residue was added water and the mixture was extracted with ethyl acetate. The organic layer was dried over anhydrous magnesium sulfate, concentrated and the residue was purified by column chromatography (SiO₂ 200g, eluent: Hexane/EtOAc=6/ 1) to give the object compound 10.65g as a colorless oil. Quantitatively
¹H NMR (CDCl₃) δ 7.44 (1H, s), 7.41 (1H, m), 7.21 (2H, m), 3.71 (3H, s), 3.60 (2H, s).

### Step (vi)

Methyl 3-cyanophenylacetate

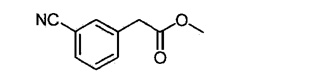

The compound 10.65g (46.5mmol) obtained in step (v) and zinc cyanide 3.44g (29.3mmol) were dissolved in DMF 120ml and stirred at room temperature for 30 minutes under an atmosphere of nitrogen. Thereto was added tetrakis(triphenylphosphine)palladium 2.15g (1.86mmol) and the mixture was refluxed at 90°C for 3 hours under an atmosphere of nitrogen. After lowering the temperature and filtration over Celite, the filtrate was concentrated. The residue was extracted with ethyl acetate, washed with 2N aqueous ammonia (50ml) and saturated brine. The organic layer was dried over anhydrous magnesium sulfate and concentrated. The residue was purified by column chromatography (SiO₂ 200g, eluent : Hexane/EtOAc=9/ 1) to give the object compound 4.28g as a pale yellow oil. Yield 53%
¹H NMR (CDCl₃) δ 7.52 (4H, m), 3.72 (3H, s), 3.67 (2H, s).

### Step (vii)

Methyl-3-(aminomethyl)phenylacetate hydrochloride

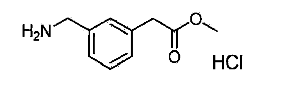

To the compound 5.82g (33.2mmol) obtained in step (vi) in methanol 60ml and 4M hydrochloric acid-dioxane 10ml was added 10% palladium-C 5g (4.72mmol) and the mixture was stirred at a pressure of 3.4 atmosphere of hydrogen at room temperature for 3 hours. After filtration over Celite and concentration of the filtrate, the resulting crystals were taken with ethyl acetate to give the object compound 6.89g (28.3mmol) as a white solid. Yield 85%
¹H NMR (CD₃OD) δ 7.42 (4H, m), 4.13 (2H, s), 3.73 (3H, s), 3.71 (2H, s).

### Step (viii)

2-Butoxy-8-methoxy-9-{6-[4-(3-methoxycarbonylmethylbenxyl)aminobutoxy]pyridin-3-ylmethyl}adenine

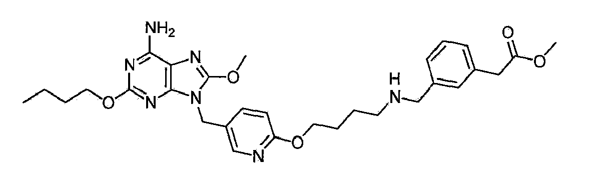

To a solution of the compound 200mg (0.48mmol) obtained in step (iv) in tetrahydrofuran (10ml) were added triethylamine (200µl) and 4-dimethylaminopyridine (12mg), and the mixture was stirred at room temperature for 10 minutes. After being cooled to 0°C, thereto was added methanesulfonyl chloride (56µl) and the mixture was stirred at room temperature for 30 minutes. Thereto was added water (50ml) and the mixture was extracted with chloroform (50 ml). The organic layer was washed with brine, dried over magnesium sulfate and concentrated in vacuo. To the residue in dimethylformamide (10ml) were added the compound 155mg (0.72mmol) obtained in step (vii), potassium carbonate 66mg (0.96mmol) and sodium iodide 216mg (1.44mmol), and the mixture was heated at 60°C for 48 hours. After removal of the solvent in vacuo, to the residue was added water (30ml) and the mixture was extracted with 30% methanol-chloroform (50ml). The organic layer was dried over magnesium sulfate, concentrated in vacuo and purified by silica gel column chromatography to give the object compound 79mg as a colorless oil. Yield 28%
¹H NMR (CDCl₃) δ 8.18 (1H, d, J= 2.4 Hz), 7.62 (1H, dd, J= 8.6 Hz, 2.4 Hz), 7.32-7.00 (4H, m), 6.64 (1H, d, J= 8.6 Hz), 5.12 (2H, brs), 5.00 (2H, s), 4.32-4.25 (4H, m), 4.10 (3H, s), 3.78 (2H, s), 3.70 (2H, s), 3.68 (3H, s), 2.69 (2H, t, 7.2Hz), 1.80-1.76 (4H, m), 1.67-1.50 (4H, m), 0.97 (3H, t, J= 7.4 Hz).

### Step (ix)

2-Butoxy-7,8-dihydro-9-{6-[4-(3-methoxycarbonylmethylbenzyl)aminobutoxy]pyridin-3-ylmethyl}-8-oxoadenine

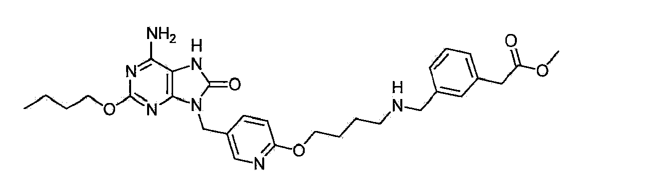

To the compound 79mg (0. 14mmol) obtained in step (viii) in methanol (5ml) was added concentrated sulfuric acid (0.5ml) and the mixture was refluxed at 60°C for 1 hour. After neutralization with 28% aqueous ammonia and removal of the solvent in vacuo, to the residue was added water 3ml, By adjusting to pH8 the resulting deposit was filtered and dried to give the titled compound 7mg as a white solid. Yield 11%
¹H NMR (DMSO-d₆) δ 9.95 (1H, brs), 8.13 (1H, s), 7.64 (1H, d, J= 8.5 Hz), 7.25-7.09 (4H, m), 6.74 (1H, d, J= 8.5 Hz), 6.47 (2H, brs), 4.80 (2H, s), 4.20 (2H, t, J= 6.5 Hz), 4.16 (2H, t, J= 6.5 Hz), 3.66 (2H, s), 3.63 (2H, s), 3.60 (3H, s), 3.37 (2H, t, J= 6.5 Hz), 1.73-1.60 (4H, m), 1.54-1.50 (2H, m), 1.41-1.35 (2H, m), 0.91 (3H, t, J= 7.3 Hz).

### Example 13

2-Butoxy-7,8-dihydro-9-(6-{3-[N-methyl-N-(3-methoxycarbonylmethyl)phenyloxyethyl]aminopropoxy}pyridin-3-ylmethyl)-8-oxoadenine

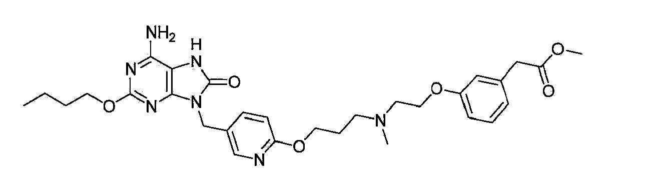

### Step (i)

2-Butoxy-9-[6-(3-hydroxypropoxy)pyridin-3-yl]methyladenine

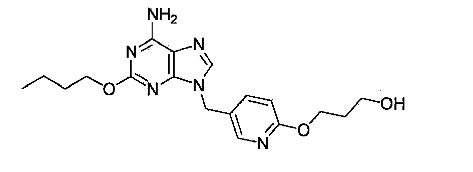

Using the compound 1.5g (4.51mmol) obtained in step (i) of example 12 and 1,3-propanediol (10ml), in the same manner as step (ii) of example 12, there was obtained the object compound 1.22g as a white solid. Yield 73%
¹H NMR (CDCl₃) δ 8.17 (1H, d, J= 2.4 Hz), 7.63 (1H, s), 7.58 (1H, dd, J= 8.6 Hz, 2.4 Hz), 6.72 (1H, d, J= 8.6 Hz), 5.95 (2H, brs), 5.20 (2H, s), 4.49 (2H, t, J= 5.9 Hz), 4.33 (2H, t, J= 6.7 Hz), 3.71 (2H, t, 5.9 Hz), 2.92 (1H, brs), 2.01-1.95 (2H, m), 1.82-1.75 (2H, m), 1.54-1.48 (2H, m), 0.98 (3H, t, J= 7.4 Hz).

### Step (ii)

2-Butoxy-8-bromo-9- [6-(3-hydroxypropoxy) pyridin-3-yl] adenine

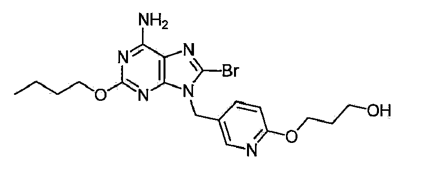

Using the compound 1.22g (2.95mmol) obtained in step (i), in the same manner as step (iii) of example 12, there was obtained the object compound 1.33g as a white solid. Yield 90%
¹H NMR (CDCl₃) δ 8.24 (1H, d, J= 2.3 Hz), 7.66 (1H, dd, J= 8.6 Hz, 2.3 Hz), 6.71 (1H, d, J= 8.6 Hz), 6.01 (2H, brs), 5.23 (2H, s), 4.49 (2H, t, J= 5.9 Hz), 4.41 (2H, t, J= 6.7 Hz), 3.70 (2H, t, 5.8Hz), 1.99-1.94 (2H, m), 1.83-1.76 (2H, m), 1.54-1.48 (2H, m), 0.98 (3H, t, J= 7.4 Hz).

### Step (iii)

2-Butoxy-9-[6-(3-hydroxypropoxy)pyridin-3-yl]-8-methoxyadenine

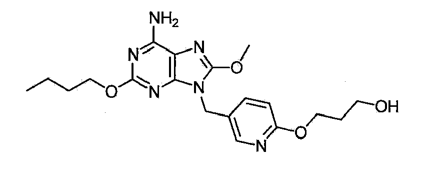

Using the compound 1.33g (2.95mmol) obtained in step (ii), in the same manner as step (iv) of example 12, there was obtained the object compound 1.18g as a white solid. Yield 99%
¹H NMR (CDCl₃) δ8.17 (1H, d, J= 2.2 Hz), 7.67 (1H, dd, J= 8.6 Hz, 2.4 Hz), 6.68 (1H, d, J= 8.6Hz), 5.14 (2H, brs), 5.01 (2H, s), 4.49 (2H, m, J= 5.8 Hz), 4.31 (2H, t, J= 6.7 Hz), 4.11 (3H, s), 3.68 (2H, q, 5.4 Hz), 1.99-1.93 (2H, m), 1.82-1.75 (2H, m), 1.54-1.48 (2H, m), 0.98 (3H, t, J= 7.4 Hz).

### Step (iv)

2-Butoxy-8-methoxy-9-(6-{3-[N-methyl-N-(3-methoxycarbonylmethyl)phenyloxyethyl]aminopropoxy}pyridin-3-ylmethyl)adenine

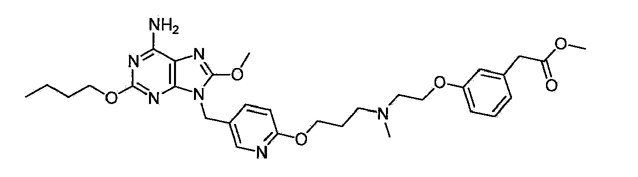

Using the compound 170mg (0.42mmol) obtained in step (iii), in the same manner as step (viii) of example 12, there was obtained the object compound 116mg as a colorless oil. Yield 45%
¹H NMR (CDCl₃) δ 7.56 (1H, d, J= 2.3 Hz), 7.47 (1H, dd, J= 9.4 Hz, 2.3 Hz), 7.24 (1H, t, J= 7.8 Hz), 6.93 (1H, d, J= 7.8 Hz), 6.83-6.78 (2H, m), 6.49 (1H, d, J= 9.4 Hz), 5.35 (2H, brs), 4.82 (2H, s), 4.48 (2H, brs), 4.31 (2H, t, J= 6.6 Hz), 4.14 (3H, s), 4.10 (2H, brs), 3.70 (3H, s), 3.60 (2H, s), 3.12 (2H, brs), 2.91 (3H, s), 2.44-2.41 (2H, m), 1.80-1.76 (2H, m), 1.59-1.47 (4H, m), 0.97 (3H, t, J= 7.4 Hz).

### Step (v)

2-Butoxy-7,8-dihydro-9-(6-{3-[N-methyl-N-(3-methoxycarbonylmethyl)phenyloxyethyl]aminopropoxy}pyridin-3-ylmethyl)-8-oxoadenine

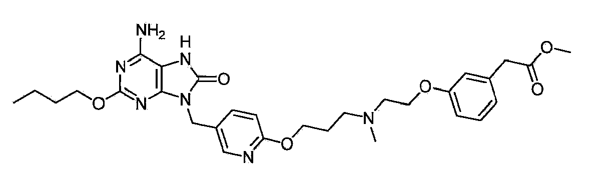

Using the compound 116mg (0.19mmol) obtained in step (iv), in the same manner as step (ix) of example 12, there was obtained the titled compound 86mg as a white solid. Yield 75%
¹H NMR (DMSO-d₆) δ 10.01 (1H, brs), 7.69 (1H, d, J= 2.3 Hz), 7.39 (1H, dd, J= 9.3 Hz, 2.3 Hz), 7.21 (1H, t, J= 7.8 Hz), 6.84-6.81 (3H, m), 6.45 (2H, brs), 6.34 (2H, d, J= 9.3 Hz), 4.59 (2H, s), 4.15 (2H, t, J= 6.5 Hz), 4.02 (2H, t, J= 5.8 Hz), 3.86 (2H, t, J= 6.5 Hz), 3.63 (2H, s), 3.60 (3H, s), 2.69 (2H, t, J= 5.7 Hz), 2.36 (2H, t, J= 6.2 Hz), 2.21 ,(3H, s), 1.77-1.72 (2H, m), 1.65-1.61 (2H, m), 1.39-1.36 (2H, m), 0.90 (3H, t, J= 7.4 Hz).

### Example 14

2-Butoxy-7,8-dihydro-9-(6-{3-[N-methyl-N-(3-hydroxycarbonylmethyl)phenyloxyethyl]aminopropoxy}pyridin-3-ylmethyl)-8-oxoadenine

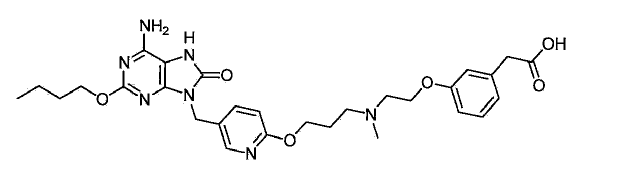

To the compound 40mg (0.067mmol) obtaining by example 13 in methanol (2ml) was added 5N aqueous sodium hydroxide and the mixture was stirred at room temperature for 3 hours. After neutralization with 1N hydrochloric acid and removal of the solvent in vacuo, to the residue was added water 3 ml to adjust pH7. The resulting deposit was filtered and dried to give the titled compound 1.3mg as a white solid. Yield 3%
¹H NMR (DMSO-d₆) δ 12.29 (1H, brs), 9.97 (1H, s), 7.70 (1H, d, J= 2.3 Hz), 7.40 (1H, dd, J= 9.4 Hz, 2.3 Hz), 7.21 (1H, t, J= 7.8 Hz), 6.83-6.80 (3H, m), 6.46 (2H, s), 6.34 (1H, d, J= 9.4 Hz), 4.60 (2H, s), 4.15 (2H, t, J= 6.6 Hz), 4.03 (2H, m), 3.87 (2H, t, J= 6.9 Hz), 3.52 (2H, s), 2.71 (2H, brs), 2.39 (2H, brs), 2.24 (3H, brs), 1.77 (2H, brs), 1.67-1.60 (2H, m), 1.43-1.34 (2H, m), 0.91 (3H, t, J= 7.4 Hz).

### Example 15

2-Butoxy-7,8-dihydro-9-[6-(4-[{N-methyl-N-[3-(methoxycarbonylmethyl)benzyl]}amino]butoxy)pyridin-3-ylmethyl]-8-oxoadenine

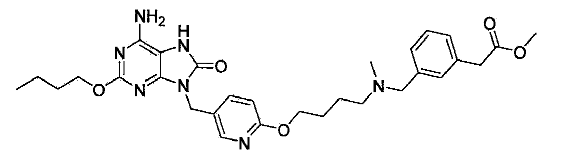

### Step (i)

2-Butoxy-9-[6-(4-[{N-methyl-N-[3-(methoxycarbonylmethyl)benzyl]}amino]butoxy)pyridin-3-ylmethyl]-8-methoxyadenine

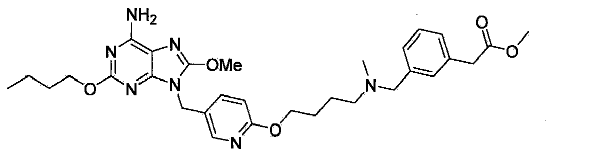

To a solution of the compound 0.21g (0.50mmol) obtained in step (iv) of example 12 in tetrahydrofuran (15 ml) were added at 0°C mesyl chloride 0.058ml (0.75mmol), triethylamine 0.11ml (0.75mmol) and 4-dimethylaminopyridine (10mg), and the mixture was elevated to room temperature, followed by stirring for 30 minutes. Then water was added thereto and the mixture was extracted with chloroform. The organic layer was washed with brine, dried over magnesium sulfate and concentrated in vacuo. To the residue were added THF 3ml and 30% methylamine /methanol 3ml and the mixture was stirred at room temperature for 30 hours. After removal of the solvent by distillation, the residue was purified by silica gel column chromatography and treated it in the same manner as example 3 step (iv) to give the object compound 0.13g as a colorless oil. Yield 43%
¹H NMR (CDCl₃) δ8.19 (1H, d, J = 2.2 Hz), 7.62 (1H, dd, J = 8.5 Hz, 2.2 Hz), 7.27-7.21 (3H, m), 7.18-7.15 (1H, m), 6.64 (1H, d, J = 8.5 Hz), 5.15 (2H, brs), 5.01 (2H, s), 4.31 (2H, t, J = 6.6 Hz), 4.26 (2H, t, J = 6.4 Hz), 4.10 (3H, s), 3.73 (3H, s), 3.63 (2H, s), 3.60-3.46 (2H, m), 2.51-2.41 (2H, m), 2.21 (3H, brs), 1.83-1.65 (6H, m), 1.50 (2H, tq, J = 7.5 Hz, 7.4 Hz), 0.97 (3H, t, J = 7.4 Hz).

### Step (ii)

2-Butoxy-7, 8-dihydro-9-[6-(4-[{N-methyl-N-[3-(methoxycarbonylmethyl)benzyl]}amino]butoxy)pyridin-3-ylmethyl]-8-oxoadenine)

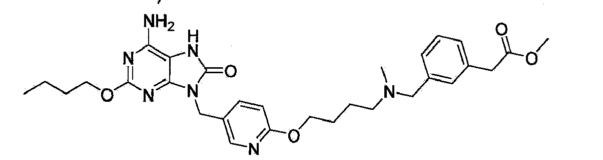

Using the compound 126mg (0.21mmol) obtained in step (i), in the same manner as example 3 step (v), there was obtained the titled compound 86mg as a white solid. Yield 70%
¹H NMR (DMSO-d₆) δ 10.03 (1H, brs), 8.13 (1H, d, J = 2.2 Hz), 7.63 (1H, dd, J = 8.5 Hz, 2.2 Hz), 7.22 (1H, dd, J = 7.5 Hz, 7.4 Hz), 7.16 (1H, s), 7.14 (1H, d, J = 7.4 Hz), 7.09 (1H, d, J = 7.5 Hz), 6.73 (1H, d, J = 8.5 Hz), 6.47 (2H, brs), 4.79 (2H, s), 4.19 (2H, t, J = 6.5 Hz), 4.15 (2H, t, J = 6.6 Hz), 3.63 (2H, s), 3.58 (3H, s), 3.38 (2H, s), 2.32 (2H, t, J = 7.1 Hz), 2.07 (3H, s), 1.69-1.54 (6H, m), 1.35 (2H, tq, J = 7.5 Hz, 7.4 Hz), 0.91 (3H, t, J = 7.4 Hz).

### Example 16

2-Butoxy-7,8-dihydro-9-[6-(4-[{N-[3-(carboxymethyl)benzyl]-N-methyl}amino]butoxy)pyridin-3-ylmethyl]-8-oxoadenine

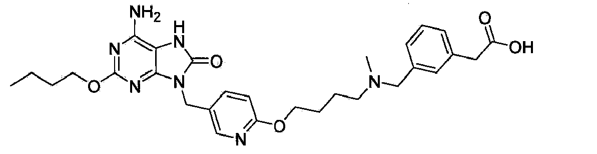

Using the compound 36mg (0.063mmol) obtained by example 15, in the same manner as example 2, there was obtained the titled compound 23mg as a white solid. Yield 64%
¹H NMR (DMSO-d₆) δ10.07 (1H, brs), 8.13 (1H, d, J = 2.2 Hz), 7.64 (1H, dd, J = 8.6 Hz, 2.2 Hz), 7.66-7.18 (4H, m), 6.74 (1H, d, J = 8.6 Hz), 6.51 (2H, brs), 4.80 (2H, s), 4.20 (2H, t, J = 5.8 Hz), 4.15 (2H, t, J = 6.6 Hz), 3.56 (2H, s), 3.40-3.25 (2H, m), 2.80-2.60 (2H, m), 2.50-2.20 (3H, m), 1.70-1.58 (6H, m), 1.35 (2H, tq, J = 7.5 Hz, 7.4 Hz), 0.91 (3H, t, J = 7.4 Hz).

### Example 17

2-Butoxy-7,8-dihydro-9-(6-[4-{[N-(3-methoxycarbonylmethylbenzyl)-N-(3-morpholinopropyl)]amino}butoxy]pyridin-3-ylmethyl)-8-oxoadenine

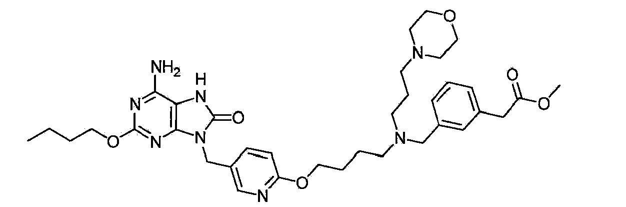

### Step (i)

2-Butoxy-8-methoxy-9-(6-chloropyridin-3-ylinethyl)adenine

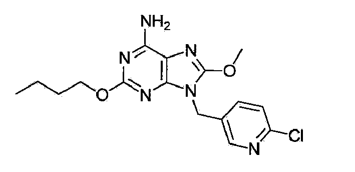

To 2-butoxy-8-methoxyadenine 1.5 g (6.33-mmol) in DMF (50ml) were added potassium carbonate 2.62g (19.0mmol) and 2-chloro-5-chloromethylpyridine 1.13g (6.96mmol), and the mixture was stirred at room temperature for 14 hours. After removal of the solvent by distillation, to the residue was added water 80ml and the mixture was extracted with 5% methanol-chloroform (100ml). The organic layer was washed with water and saturated brine, successively, dried over magnesium sulfate and concentrated in vacuo. The residue was purified by silica gel column chromatography and the resulting solid was repulp-washed with ethyl acetate (30ml) to give the object compound 1.20g as a pale brown solid. Yield 52%.
¹H NMR (CDCl₃) δ 8.45 (1H, d, J= 2.3 Hz), 7.67 (1H, dd, J= 8.2 Hz, 2.3 Hz), 7.28 (1H, d, J= 8.2 Hz), 5.15 (2H, brs), 5.08 (2H, s), 4.31 (2H, t, J= 7.0 Hz), 4.10 (3H, s), 1.81-1.74 (2H, m), 1.53-1.46 (2H, m), 0.97 (3H, t, J= 7.4 Hz).

### Step (ii)

2-Butoxy-7,8-dihydro-9-[6-(4-hydroxybutoxy)pyridin-3-ylinethyl]-8-oxoadenine

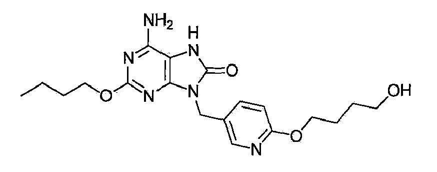

Using the compound 1.0g (2.87mmol) obtained in step (i), in the same manner as example 12 step (ii), there was obtained the object compound 458mg as a white solid. Yield 40%
¹H NMR (DMSO-d₆) δ9.95 (1H, brs), 8.14 (1H, d, J= 2.3 Hz), 7.64 (1H, dd, J= 8.6 Hz, 2.3 Hz), 6.75 (1H, d, J= 8.6 Hz), 6.45 (2H, brs), 4.99 (2H, s), 4.43 (1H, t, J= 5.2 Hz), 4.21 (2H, t, J= 6.6 Hz), 4.16 (2H, t, J= 6.6 Hz), 3.43 (2H, t, J= 5.9 Hz), 1.72-1.62 (4H, m), 1.53-1.49 (2H, m), 1.41-1.38 (2H, m), 0.92 (3H, t, J= 7.4 Hz).

### Step (iii)

2-Butoxy-7,8-dihydro-9-{6-[4-(3-hydroxypropylamino)butoxy]pyridin-3-ylmethyl}-8-oxoadenine

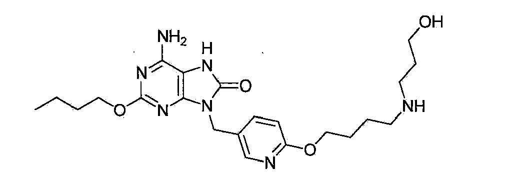

To the compound 229mg (0.55mmol) obtained in step (ii) in methylene chloride 7ml was added thionylchloride 136mg (1.10mmol) and the mixture was refluxed at 60°C for 2 hours. After concentration of the solvent the dried residue was dissolve in dimethylformamide 5ml and thereto were added aminopropanol 411mg (5.46mmol) and sodium iodide 98mg (0.66mmol), followed by heating at 65°C for 24 hours. After concentration of the solvent, to the residue was added water 10ml to adjust pH7. The resulting solid was filtered to give the object compound 251mg as a white solid. Quantitatively
¹H NMR (DMSO-d₆) δ 10.04 (1H, brs), 8.15 (1H, d, J= 2.3 Hz), 7.66 (1H, dd, J= 8.6 Hz, 2.3 Hz), 6.77 (1H, d, J= 8.6 Hz), 6.50 (2H, brs), 4.81 (2H, s), 4.24 (2H, t, J= 6.6 Hz), 4.16 (2H, t, J= 6.6 Hz), 3.46 (2H, t, 6.0 Hz), 3.41 (2H, brs), 2.94 (2H, t, J= 7.6 Hz), 2.56 (1H, brs), 1.73-1.38 (10H, m), 0.92 (3H, t, J= 7.4 Hz).

### Step (iv)

2-Butoxy-7,8-dihydro-9-{6-[4-(3-t-butyldimethylsiloxypropyl)aminobutoxy]pyridin-3-ylmethyl}-8-oxoadenine

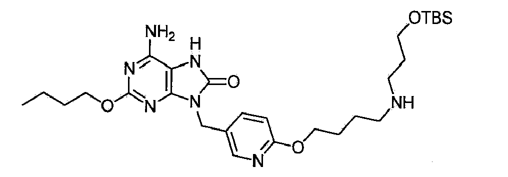

To sodium hydride 95mg (2.18mmol) in tetrahydrofuran (20ml) was added at 0°C the compound 244mg (0.53mmol) obtained in step (iii), and the mixture was stirred at room temperature for 30 minutes. Thereto was added at 0°C t-butyldimethylchlorosilane 384mg (2.55mmol) and the mixture was stirred at room temperature for 2 hours. The reaction was quenched by adding water at 0°C, and the solvent was concentrated in vacuo. The residue was extracted with 20% methanol-chloroform (100ml). The organic layer was washed with water and brine, successively and dried over magnesium sulfate. After removal of the solvent, the residue was purified by silica gel column chromatography to give the object compound 116mg a white solid. Yield 38%
¹H NMR (DMSO-d₆) δ 9.82 (1H, brs), 8.13 (1H, d, J= 2.3 Hz), 7.64 (1H, dd, J= 8.6 Hz, 2.3 Hz), 6.74 (1H, d, J= 8.6 Hz), 6.44 (2H, brs), 4.79 (2H, s), 4.20 (2H, t, J= 6.6 Hz), 4.15 (2H, t, J= 6.6 Hz), 3.61 (2H, t, 6.2 Hz), 2.54 (4H, brs), 2.45 (1H, brs), 1.65-1.37 (10H, m), 0.91 (3H, t, J= 7.4 Hz), 0.83 (9H, s), 0.01 (6H, s).

### Step (v)

2-Butoxy-7,8-dihydro-9-(6-[4-{[N-(3-t-butyldimethylsiloxypropyl)-N-(3-methoxycarbonylmethylbenzyl)]amino}butoxy]pyridin-3-ylmethyl)-8-oxoadenine

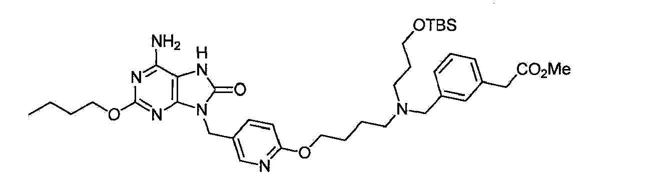

A solution of the compound 115mg (0.20mmol) obtained in step (iv) and the compound 46mg (0.26mmol) obtained by example 3 step (iii) in methanol (20ml) was added sodium cyanoborohydride 80mg (1.3mmol), and the mixture was stirred at room temperature for 7 days. After concentration of the solvent in vacuo, the residue was extracted with 20% chloroform-methanol (80ml) and dried over magnesium sulfate. After concentration of the solvent the residue was purified by silica gel column chromatography to give the object compound 69mg as a white solid. Yield 46%
¹H NMR (DMSO-d₆) δ10.18 (1H, brs), 8.12 (1H, d, J= 2.4 Hz), 7.63 (1H, dd, J= 8.6 Hz, 2.4 Hz), 7.22-7.14 (3H, m), 7.08 (1H, d, J= 7.4 Hz), 6.71 (1H, d, J= 8.6 Hz), 6.49 (2H, brs), 4.79 (2H, s), 4.16-4.13 (4H, m), 3.60 (2H, s), 3.58 (3H, s), 3.56 (2H, t, J= 6.2 Hz), 3.45 (2H, s), 2.41 (2H, t, J= 7.0 Hz), 2.36 (2H, t, J= 7.0 Hz), 1.66-1.48 (8H, m), 1.40-1.35 (2H, m), 0.91 (3H, t, J= 7.4 Hz), 0.78 (9H, s), -0.05 (6H, s).

### Step (vi)

2-Butoxy-7,8-dihydro-9-(6-[4-{[N-(3-hydroxypropyl)-N-(3-methoxycarbonylmethylbenzyl)]amino}butoxy]pyridin-3-ylmethyl)-8-oxoadenine

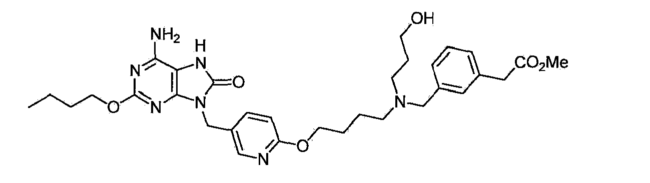

To the compound 68mg (0.092mmol) obtained step (ii) in ethanol (5ml) was added 1N hydrochloric acid 2ml and the mixture was stirred at room temperature for 2 hours. After neutralization with aqueous sodium hydrogencarbonate, the solvent was concentrated. Thereto was added water 2ml and the resulting white solid was taken to give the object compound 53mg as a white solid. Yield 84%
¹H NMR (DMSO-d₆) δ 10.19 (1H, brs), 8.12 (1H, d, J= 2.3 Hz), 7.64 (1H, dd, J= 8.6 Hz, 2.3 Hz), 7.23-7.15 (3H, m), 7.08 (1H, d, J= 7.4 Hz), 6.73 (1H, d, J= 8.6 Hz), 6.52 (2H, brs), 4.79 (2H, s), 4.36 (1H, brs), 4.17-4.14 (4H, m), 3.62 (2H, s), 3.59 (3H, s), 3.48 (2H, s), 3.39 (2H, t, J= 6.2 Hz), 2.43-2.36 (4H, m), 1.67-1.51 (8H, m), 1.41-1.35 (2H, m), 0.93 (3H, t, J= 7.4 Hz).

### Step (vii)

2-Butoxy-7,8-dihydro-9-(6-[4-{[N-(3-methoxycarbonylmethylbenzyl)-N-(3-morpholinopropyl)]amino}butoxy]pyridin-3-ylmethyl)-8-oxoadenine

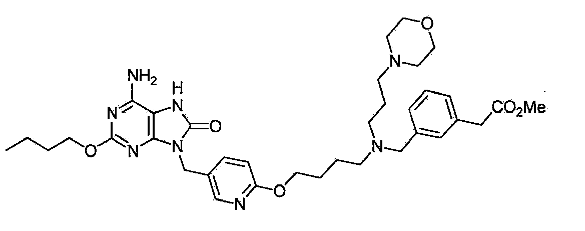

To the compound 53mg (0.085mmol) obtained in step (vi) in tetrahydrofuran (3ml) were added triethylamine 20µl and methanesulfonyl chloride (10µl), and the mixture was stirred for 30 minutes at room temperature, followed by extraction with chloroform (30ml). The organic layer was washed with brine and dried over magnesium sulfate. After removal of the solvent, to the residue in dimethylformamide 5 ml was added morpholine 74mg (0.85mmol) and the mixture was heated at 70°C for 4 hours. After removal of the solvent, the residue was extracted with 20% methanol-chloroform and the organic layer was washed with brine, and dried over magnesium sulfate. After concentration of the solvent in vacuo, the residue was purified by silica gel column chromatography to give the titled compound 44mg as a white solid. Yield 74%
¹H NMR (DMSO-d₆) δ10.06 (1H, brs), 8.13 (1H, d, J= 2.3 Hz), 7.64 (1H, dd, J= 8.6 Hz, 2.3 Hz), 7.23-7.15 (3H, m), 7.08 (1H, d, J= 7.3 Hz), 6.73 (1H, d, J= 8.6 Hz), 6.48 (2H, brs), 4.80 (2H, s), 4.19-4.14 (4H, m), 3.61 (2H, s), 3.59 (3H, s), 3.48 (6H, brs), 2.40-2.35 (4H, m), 2.23-2.18 (6H, m), 1.67-1.60 (4H, m), 1.56-1.50 (4H, m), 1.43-1.33 (2H, m), 0.91 (3H, t, J= 7.4 Hz).

### Example 18

2-Butoxy-7,8-dihydro-9-(6-{4-[N-(3-hydroxycarbonylmethylbenzyl)-N-(3-morphlinopropyl)]aminobutoxy}pyridine-3-ylmethyl)-8-oxoadenine

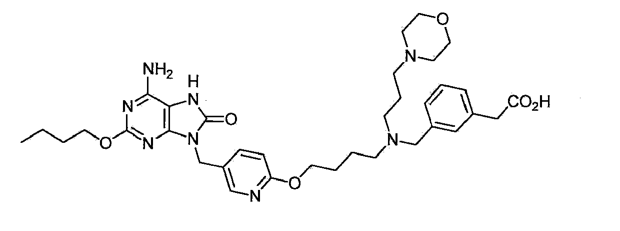

Using the compound 12mg (0.017mmol) obtained by example 17, in the same manner as example 2, there was obtained the titled compound 8mg as a white solid. Yield 71%
¹H NMR (DMSO-d₆) δ12.03 (1H, brs), 8.09 (1H, d, J= 2.3 Hz), 7.64 (1H, dd, J= 8.5 Hz, 2.3 Hz), 7.05-6.96 (6H, m), 6.71 (1H, d, J= 8.5 Hz), 4.76 (2H, s), 4.28 (2H, t, J= 6.3 Hz), 4.15 (2H, t, J= 6.6 Hz), 3.47 (4H, t, J= 4.4 Hz), 3.39 (2H, s), 3.16 (2H, s), 2.34 (2H, t, J= 8.1 Hz), 2.25 (2H, t, J= 7.1 Hz), 2.20 (4H, brs), 2.11 (2H, t, J= 7.0 Hz), 1.67-1.60 (4H, m), 1.45-1.34 (6H, m), 0.92 (3H, t, J= 7.4 Hz).

### Example 19

7,8-Dihydro-9-[4-{N-[2-(3-methoxycarbonylmethylphenyl-1-yl) oxyethyl]-N-methylamino}methylbenzyl]-2-(2-methoxyethoxy)- 8-oxoadenine

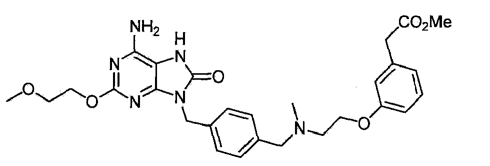

### Step (i)

9-[4-(Hydroxymethyl)benzyl]-2-(2-methoxyethoxy)-8-methoxyadenine

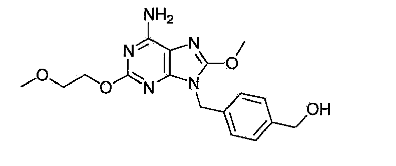

In the same manner as example 5 step (i), there was obtained the object compound as a white solid. Yield 84%
¹H NMR (DMSO-d₆) δ 7.26 (2H, d, J = 8.1 Hz), 7.19 (2H, d, J = 8.1 Hz), 6.87 (2H, brs), 5.15 (1H, t, J = 5.7 Hz), 5.01 (2H, s), 4.44 (2H, d, J = 5.7 Hz), 4.29 (2H, dd, J = 4.7, 4.8 Hz), 4.03 (3H, s), 3.60 (2H, d, J = 4.7, 4.8 Hz), 3.16 (3H, s).

### Step (ii)

7,8-Dihydro-9-[4-(chloromethyl)benzyl]-2-(2-methoxyethoxy)-8-oxoadenine

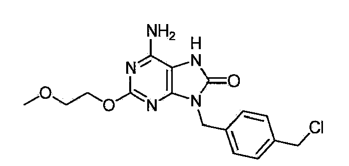

In the same manner as example 5 step (ii), there was obtained the object compound as a white solid. Yield 83%
¹H NMR (DMSO-d₆) δ 7.39 (2H, d, J = 8.1 Hz), 7.31 (2H, d, J = 8.1 Hz), 4.88 (2H, s), 4.73 (2H, s), 4.34 (2H, dd, J = 4.7, 4.8 Hz), 3.60 (2H, dd, J = 4.7, 4.8 Hz), 3.27 (3H, s).

### Step (iii)

7,8-Dihydro-9-[4-{N-[2-(3-Methoxycarbonylmethylphenyl-1-yl)oxyethyl]-N-methylamino}methylbenzyl]-2-(2-methoxyethoxy)-8-oxoadenine

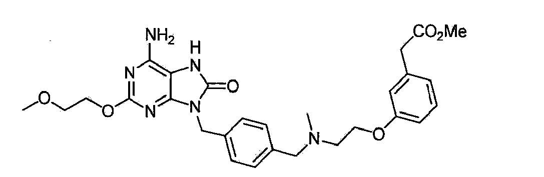

The compound 223mg (0.614mmol) obtained in step (ii), methyl {3-[2-(N-methylamino)ethoxy]phenyl}acetate hydrochloride 158mg (0.614mmol) and diisopropylethylamine 320µl (1.86mmol) were mixed at room temperature and the mixture was stirred at 60°C for 14.5 hours. After removal of the solvent by distillation, to the residue was added saturated brine 5 ml and the mixture was extracted with 33% ethanol-chloroform (30ml). The organic layer was dried over anhydrous magnesium sulfate and concentrated in vacuo. The residue was purified by silica gel column chromatography and thereto was added acetone. The resulting solid was filtered to give the titled compound 186mg as a white solid. Yield 55%
¹H NMR (DMSO-d₆) δ 9.95 (1H, brs), 7.28-7.24 (4H, m), 7.21-7.16 (1H, m), 6.83-6.78 (3H, m), 6.47 (2H, brs), 4.83 (2H, s), 4.26 (2H, t, J= 4.6 Hz), 4.05 (2H, t, J= 5.9 Hz), 3.62 (2H, s), 3.61 (3H, s), 3.60-3.57 (2H, m), 3.56 (2H, s), 3.26 (3H, s), 2.70 (2H, t, J= 5.9 Hz), 2.20 (3H, s).

### Example 20

7,8-Dihydro-9-[4-{N-[2-(3-hydroxycarbonylmethylphenyl-1-yl)oxyethyl]-N-methylamino}methylbenzyl]-2-(2-methoxyethoxy)-8-oxoadenine

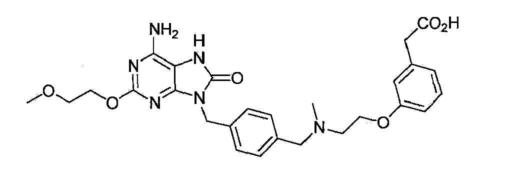

Using the compound 65.4mg (0.119mmol) obtained by example 19, in the same manner as example 2, there was obtained the titled compound 50.5mg as a white solid. Yield 79%
¹H NMR (DMSO-d₆) δ7.31-7.11 (4H, m), 6.83-6.70 (4H, m), 4.83 (2H, s), 4.25 (2H, t, J= 4.6 Hz), 4.01 (2H, t, J= 5.8 Hz), 3.58 (2H, t, J= 4.6 Hz), 3.52 (2H, s), 2.68 (2H, t, J= 5.8 Hz), 2.21 (3H, s).

### Example 21

2-Butoxy-7,8-dihydro-9-(4-{4-[3-(methoxycarbonylmethyl)phenoxymethyl]piperidin-1-ylmethyl}benzyl)-8-oxoadenine

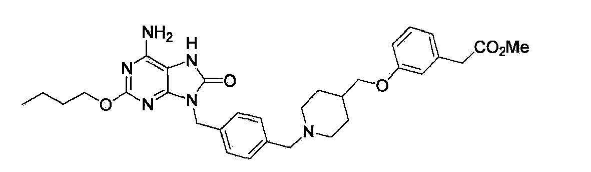

In the same manner as example 5 step (iii), there was obtained the titled compound as a white solid. Yield 49%
¹H NMR (DMSO-d₆) δ 9.99 (1H, s), 7.21 (5H, m), 6.80 (3H, m), 6.46 (2H, s), 4.83 (2H, s), 4.14 (2H, t, J=6.6 Hz), 3.78 (2H, d, J=5.8 Hz), 3.62 (2H, s), 3.60 (3H, s), 3.40 (2H, s), 2.80 (2H, d, J=11.2 Hz), 1.91 (2H, t, J=10.8 Hz), 1.65 (5H, m), 1.33 (4H, m), 0.90 (3H, t, J=7.2 Hz).

### Example 22

2-Butoxy-7,8-dihydro-9-(4-{4-[3-(carboxylmethyl)phenoxymethyl]piperidin-1-ylmethyl}benzyl)-8-oxoadenine

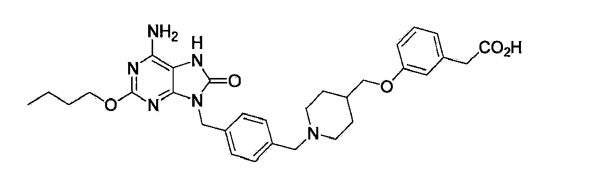

In the same manner as example 2, there was obtained the titled compound as a white solid. Quantitatively
¹H NMR (DMSO-d₆) δ 12.29, (1H, s), 10.19 (1H, s), 7.47 (4H, m), 7.21 (1H, m), 6.81 (3H, s), 6.58 (2H, s), 4.88 (2H, s), 4.24 (2H, m), 4.14 (2H, t, J=6.6 Hz), 3.81 (2H, s), 3.52 (2H, s), 2.95 (2H, m), 1.91 (4H, m), 1.61 (3H, m), 1.37 (3H, m), 0.91 (3H, t, J=7.2 Hz)

### Example 23

### Human TLR7 reporter assay

HEK293 cells in which human TLR7 or rat TLR7 plasmid and reporter plasmid (NF-kB-SEAP) are stably introduced are dispersed in DMEM broth (10%FBS, 1%NEAA, 10µg/mL blastocidin S HCl, 100 ug/mL Zeocin), and were seeded to 96 well plate per 90µl/well (hTLR7/seap-293: 20000cells/well, rTLR7/seap-293:25000cells/well).

Test compound (DMSO stock solution (2µl) was diluted with the broth (200µl) by 100times) was added to the seeded cells to a 96well plate (10µl/well) (final concentration; 1nM - 10µM, common ratio). After stirring by tapping side of the plate, the cells were cultured in a CO₂ incubator for 20 hours. A substrate (50µl/well) for reporter assay (substrate for SEAP, pNPP) was added to cells stimulated by test sample. Ten minutes after adding the substrate, the reaction quenching solution (4N NaOH) was added by 50µl/well to cease enzymatic reaction. Sealing a top seal A on the plate, the absorbance was measured by a micro plate reader (405nm).

Human TLR7 binding activity (EC₅₀) of each compound is shown in Table 1.

**Table 1**

| Compound | EC₅₀ (nM) |
|---|---|
| Example 1 | 26.0 |
| Example 3 | 20.4 |
| Example 5 | 40.8 |
| Example 7 | 24.1 |
| Example 8 | 253.1 |
| Example 9 | 28.3 |
| Example 11 | 21.0 |
| Example 12 | 9.1 |
| Example 15 | 9.4 |
| Example 16 | 29.6 |
| Example 17 | 20.7 |
| Example 19 | 51.5 |

### Example 24

2-Butoxy-7,8-dihydro-9-[4-(N-{2-[1-methoxy-5-(methoxycarbonylmethyl)phenoxy]ethyl}aminomethyl)benzyl]-8-oxoadenine

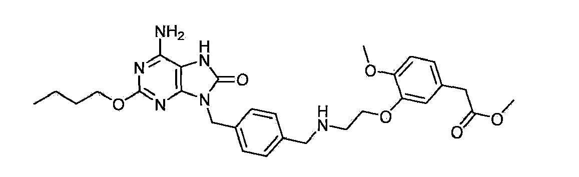

In the same manner as example 5 step (iii), there was obtained the titled compound as a white solid. Yield 19%
¹H NMR (DMSO-d₆) δ 9.96 (1H, brs), 7.31-7.23 (4H, m), 6.88-6.85 (2H, m), 6.76 (1H, dd, J= 1.8, 8.2 Hz), 6.44 (2H, brs), 4.83 (2H, s), 4.14 (2H, t, J= 6.6 Hz), 3.97 (2H, t, J= 5.7 Hz), 3.73 (2H, s), 3.71 (3H, s Hz), 3.59 (3H, s), 3.56 (2H, s), 2.80 (2H, t, J= 5.7 Hz), 1.65-1.58 (2H, m), 1.41-1.32 (2H, m), 0.89 (3H, t, J= 7.4 Hz).

### Example 25

2-Butoxy-7,8-dihydro-9-[4-(N-{2-[1-methoxy-5-(carboxymethyl)phenoxy]ethyl}aminomethyl)benzyl]-8-oxoadenine

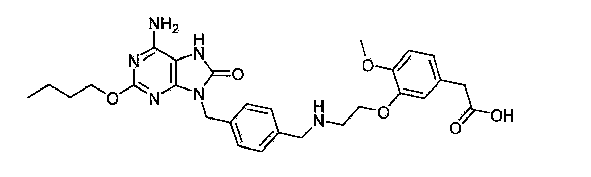

Using the compound 60mg (0.11mmol) obtained by example 24, in the same manner as example 2, there was obtained the titled compound 42mg as a white solid. Yield 72%
¹H NMR (DMSO-d₆) δ 10.10 (1H, brs), 7.31-7.23 (4H, m), 6.87-6.85 (2H, m), 6.76 (1H, dd, J= 1.8, 8.2 Hz), 6.49 (2H, brs), 4.82 (2H, s), 4.14- (2H, t, J= 6.6 Hz), 3.96 (2H, t, J= 5.7 Hz), 3.72 (2H, s), 3.70 (3H, s), 3.47 (2H, s), 2.80 (2H, t, J= 5.7 Hz), 1.64-1.58 (2H, m), 1.40-1.34 (2H, m), 0.90 (3H, t, J= 7.4 Hz).

### Example 26

7,8-Dihydro-9-(4-{4-[3-(methoxycarbonylmethyl)phenoxy]piperidin-1-ylmethyl}benzyl)-2-(2-methoxyethoxy)-8-oxoadenine

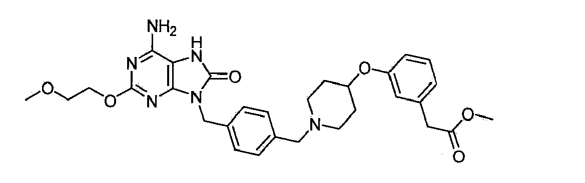

In the same manner as example 19 step (iii), there was obtained the titled compound as a white solid. Yield 46%
¹H NMR (DMSO-d₆) δ 10.04 (1H, brs), 7.25 (4H, s), 7.18 (1H, t, J= 7.9 Hz), 6.83 (2H, s), 6.81-6.77 (2H, m), 6.49 (2H, brs), 4.83 (2H, s), 4.5-4.32 (1H, m), 4.26 (2H, dd, J= 4.6, 5.1 Hz), 3.61 (2H, s), 3.60 (3H, s), 3.60-3.57 (2H, m), 3.44 (2H, s), 3.26 (3H, s), 2.65-2.62 (2H, m), 2.22-2.17 (2H, m), 1.71-1.88 (2H, m), 1.62-1.54 (2H, m).

### Example 27

7,8-Dihydro-9-(4-{4-[3-(carboxymethyl)phenoxy]piperidinylmethyl}benzyl)-2-(2-methoxyethoxy)-8-oxoadenine

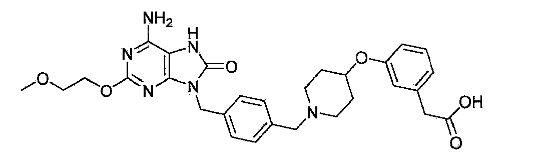

Using the compound 80mg (0.14mmol) obtained by example 26, in the same manner as example 2, there was obtained the titled compound 40mg as a white solid. Yield 52%
¹H NMR (DMSO-d₆) δ 12.28 (1H, brs), 10.08 (1H, brs), 7.41-7.29 (4H, m), 7.22 (1H, t, J= 7.9 Hz), 6.88-6.80 (3H, m), 6.59 (2H, brs), 4.86 (2H, s), 4.48-4.44 (1H, m), 4.27 (2H, dd, J= 4.6, 5.1 Hz), 3.58 (2H, dd, J= 4.6, 5.1 Hz), 3.51 (2H, s), 3.31 (2H, s), 3.26 (3H, s), 2.65-2.62 (2H, m), 2.22-2.17 (2H, m), 1.71-1.88 (2H, m), 1.62-1.54 (2H, m).

### Example 28

2-Butoxy-7,8-dihydro-9-(3-methoxycarbonyl-4-{N-[3-(methoxycarbonylmethy)benzyl]-N-methylaminomethylpropargyl}benzyl)-8-oxoadenine

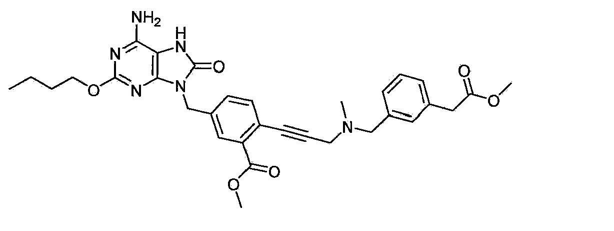

### Step (i)

4-Iodo-3-methoxycarbonyltoluene

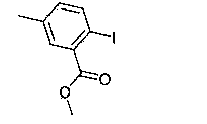

To 4-amino-3-methoxycarbonyltoluene 1.36g (8.2mmol) in THF (20ml) were added cupper iodide 1.56g (8.2mmol), diiodomethane 3.32ml (41.2mmol) and isoamyl nitrite 3.32ml (41.2mmol), and the mixture was stirred at 70°C for 1.5 hours. After filtration and concentration, the residue was purified by silica gel column chromatography to give the titled compound 1.7g (6.2mmol) as an yellow oil. Yield 75%
¹H NMR (CDCl₃) δ 7.86 (1H, d, J = 8.2 Hz), 7.63 (1H, s), 6.99 (1H, d, J = 8.2 Hz), 3.94 (3H, s), 2.34 (3H, s).

### Step (ii)

2-Butoxy-9-(4-iodo-3-methoxycarbonylbenzyl)-8-methoxyadenine

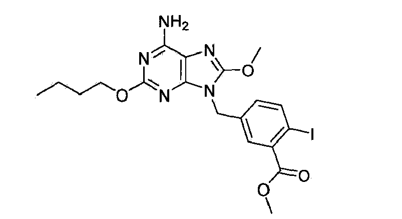

To 4-iodo-3-methoxycarbonyltoluene 1.7 g (6.2mmol) obtained in step (i) in carbon tetrachloride (30ml) were added NBS 1.2g (6.8mmol) and perbenzoic acid 100mg, and the mixture was stirred at 90°C for 6.5 hours. After filtration and concentration, to the residue was added water and the mixture was extracted with chloroform. The organic layer was washed with water and saturated brine, dried over Na₂SO₄ and concentrated. The residue dried in vacuo was dissolved in DMF (30ml). Thereto were added 2-butoxy-8-methoxyadenine, trifluoroacetic acid 1.73g (4.9mmol) and potassium carbonate 1.7g (12.3mmol) and the mixture was stirred at room temperature for 13 hours. After filtration and concentration, to the residue was added water, and the mixture was extracted with chloroform-methanol. The organic layer was washed with water and saturated brine, dried over Na₂SO₄, and concentrated. The residue was purified by silica gel column chromatography to give the titled compound 620mg (1.2mmol) as a reddish brown oil. Yield 25%
¹H NMR (DMSO-d₆) δ 7.97 (1H, d, J = 8.2 Hz), 7.64 (1H, brs), 7.10 (1H, d, J = 8.2 Hz), 6.87 (2H, brs), 5.04 (2H, s), 4.15 (2H, t, J = 6.6 Hz), 4.05 (3H, s), 3.83 (3H, s), 1.65-1.61 (2H, m), 1.40-4.35 (2H, m), 0.92 (3H, t, J = 7.4 Hz).

### Step (iii)

2-Butoxy-8-methoxy-9-{3-methoxycarbonyl-4-[3-(N-methylamino)-1-propyn-1-yl] benzyl}adenine

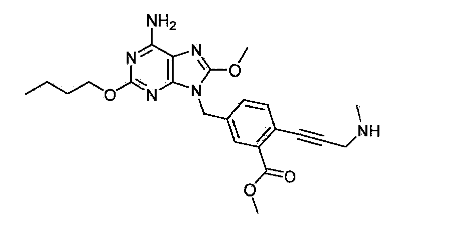

To 2-butoxy-9-(4-iodo-3-methoxycarbonylbenzyl)-8-methoxyadenine) 620mg (1.2mmol) obtained in step (ii) in THF (5ml) were added triethylamine 204µl (2.4mmol), tetrakis(triphenylphosphine) paradium 150mg (0.1mmol) and further cupper iodide 23mg (0.1mmol) and N-methyl-N-propargylamine in THF. The mixture was stirred at 70°C for 3.5 hours and concentrated. To the residue was added water, and the mixture was extracted with chloroform-methanol. The organic layer was washed with water and saturated brine, and dried over Na₂SO₄. The residue was purified by silica gel column chromatography to give the titled compound 180mg (0.4mmol) as an orange solid. Yield 33%
¹H NMR (DMSO-d₆) δ 7.75 (1H, s), 7.51 (1H, d, J = 8.2 Hz), 7.37 (1H, d, J = 8.2 Hz), 6.89 (2H, brs), 5.09 (2H, s), 4.16 (2H, t, J = 6.6 Hz), 4.03 (3H, s), 3.82 (3H, s), 3.47 (2H, s), 2.35 (3H, s), 1.64-1.61 (2H, m), 1.40-1.35 (2H, m), 0.90 (3H, t, J = 7.4 Hz).

### Step (iv)

2-Butoxy-7,8-dihydro-9-(3-methoxycarbonyl-4-{N-[3-(methoxycarbonylmethy)benzyl]-N-methylaminomethylpropargyl}benzyl)-8-oxoadenine)

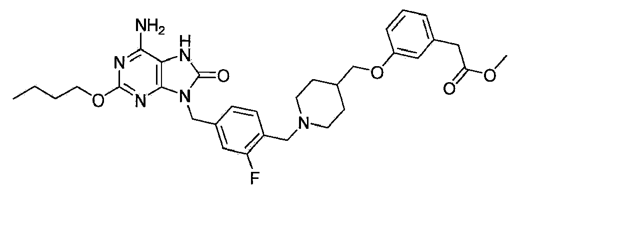

To 2-butoxy-8-methoxy-9-[3-methoxycarbonyl-4-(N-methylaminomethylpropargyl)benzyl]adenine) 180mg (0.40mmol) obtained in step (iii) in DMF (5ml) were added potassium carbonate 66mg (0.48mmol) and 3-(methoxycarbonyl)benzyl bromide 106mg (0.44mmol), and the mixture was stirred at room temperature for 4.5 hours. After concentration, to the residue dried in vacuo were added methanol (18ml) and concentrated sulfuric acid 200µl, and the mixture was stirred at 85°C for 3 hours. After neutralization with aqueous ammonia, to the residue concentrated was added water, and the mixture was extracted with chloroform-methanol. The organic layer was washed with saturated brine, dried over Na₂SO₄ and concentrated. The residue was purified by column chromatography to give the titled compound 80mg (0.10mmol) as a white solid. Yield 33%
¹H NMR (DMSO-d₆) δ 10.00 (1H, brs), 7.81 (1H, s), 7.57 (1H, d, J = 8.2 Hz), 7.48 (1H, d, J = 8.2 Hz), 7.29 (1H, t, J = 7.5 Hz), 7.25-7.19 (2H, m), 7.15 (1H, t, J = 7.4 Hz), 6.48 (2H, brs), 4.91 (2H, s), 4.13 (2H, t, J = 6.6 Hz), 3.83 (3H, s), 3.67 (2H, s), 3.59 (3H, s), 3.58 (2H, s), 3.52 (2H, s), 2.27 (3H, s), 1.63-1.59 (2H, m), 1.39-1.33 (2H, m), 0.89 (3H, t, J = 7.4 Hz).

### Example 29

2-Butoxy-7,8-dihydro-9-[3-fluoro-4-{4-[3-(methoxycarbonylmethyl)phenoxymethyl]piperidin-1 -yl}methylbenzyl]-8-oxoadenine

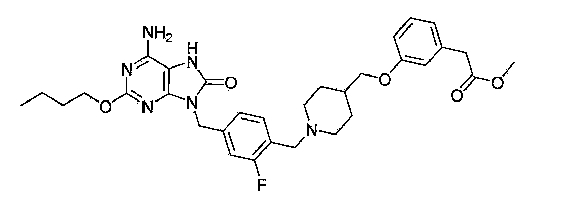

### Step (i)

4-Bromo-1-(tert-butyldimethylsiloxymethyl)-2-fluorobenzene

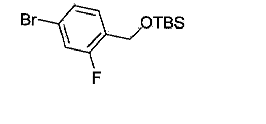

4-Bromo-2-fluoro-1-(methoxycarbonyl)benzene 3.0g (12.9mmol) was dissolved in THF (50ml) and thereto was added portion wise under ice cooling lithium aluminum hydride 732mg (19.3mmol). After 1.5 hours, the reaction was quenched with 1N aqueous sodium hydroxide. After filtration over Celite, the filtrate was concentrated and thereto was added water. The mixture was extracted with ethyl acetate and the organic layer was washed with water and saturated brine, dried over Na₂SO₄, concentrated and dried in vacuo. The residue was dissolved in DMF (30ml) and thereto were added imidazole 871mg (12.8mmol) and TBSC1 1.93g (12.8mmol), followed by stirring at room temperature for 10 hours. After concentration, thereto was added water and the mixture was extracted with ethyl acetate. The organic layer was washed with water and saturated brine, dried over Na₂SO₄, and concentrated. The residue was purified by column chromatography to give the titled compound 2.7 g (8.5mmol) as a colorless transparent oil. Yield 69%
¹H NMR (DMSO-d₆) δ 7.35 (1H, dd, J = 7.7, 7.7 Hz), 7.27 (1H, dd, J = 1.8, 8.3 Hz), 7.16 (1H, dd, J = 1.8, 9.6 Hz), 4.72 (2H, s), 0.92 (9H, s), 0.10 (6H, s).

### Step (ii)

4-bromo-1-(tert-butyldimethylsiloxymethyl)-2-hydroxymethylbenzene

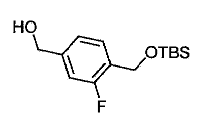

To 4-bromo-1-(tert-butyldimethylsiloxymethyl)-2-fluorobenzene 1.5g (4.7mmol) obtained in step (i) in THF (30ml) was added at -78°C n-BuLi hexane (5.2mmol). After 5 minutes, thereto was added DMF 687mg (9.4mmol) and the temperature was raised to freezing, followed by stirring for 1.5 hours. After the reaction was quenched with water, the mixture was concentrated and to the residue was added water. The mixture was extracted with ethyl acetate. The organic layer was washed with water and a saturated brine and dried over Na₂SO₄. To the residue was added MeOH (30ml), and to the solution was added under ice cooling sodium borohydride, followed by stirring for 30 minutes. The reaction was quenched with aqueous saturated ammonium chloride. After concentration, thereto was added water and the mixture was extracted with ethyl acetate. The organic layer was washed with water and saturated brine, dried over Na₂SO₄, and concentrated. The residue was purified by silica gel column chromatography to give the titled compound 330mg (1.2mmol) as a colorless transparent oil. Yield 26%
¹H NMR (CDCl₃) δ 7.35 (1H, dd, J = 7.7, 7.7 Hz), 7.01 (1H, dd, J = 1.8, 8.3 Hz), 6.93 (1H, dd, J = 1.8, 9.6 Hz), 4.68 (2H, s), 4.57 (2H, s), 0.83 (9H, s), 0.00 (6H, s).

### Step (iii)

2-butoxy-9-[4-(tert-butyldimethylsiloxymehtyl)-3-fluorobenzyl]-8-methoxyadenine

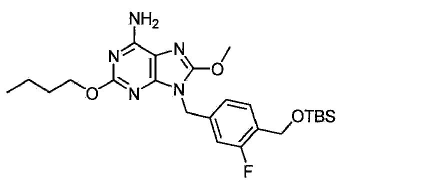

To 4-bromo-1-(tert-butyldimethylsiloxymethyl)-2-hydroxymethylbenzene 254 mg (0.94mmol) obtained in step (ii) in THF (5ml) were added triethylamine 154µl (1.13mmol) and DMAP11mg (0.09mmol) and then was dropped methanesulfonyl chloride 87µl. The mixture was stirred at room temperature for 5 minutes, and concentrated. To the residue was added water and the mixture was extracted with ethyl acetate. The organic layer was washed with water and saturated brine, dried over Na₂SO₄ and concentrated. The residue was dissolved in DMF 5ml and thereto were added 2-butoxy-8-methoxyadenine trifluoroacetate 300mg (0.84mmol) and potassium carbonate 350mg (2.54mmol), followed by stirring at 45°C for 3 hours. After concentration, thereto was added water and the mixture was extracted with chloroform-methanol. The organic layer was washed with water and saturated brine, dried over Na₂SO₄, and concentrated. The residue was purified by silica gel column chromatography to give the titled compound 250mg (0.5mmol) as a white solid. Yield 60%
¹H NMR (DMSO-d₆) δ 7.39 (1H, dd, J = 7.7, 7.7 Hz), 7.05-7.03 (2H, m), 6.87 (2H, brs), 5.04 (2H, s), 4.67 (2H, s), 4.16 (2H, t, J = 6.6 Hz), 4.03 (3H, s), 1.66-1.59 (2H, m), 1.41-1.35 (2H, m), 0.92 (3H, t, J = 7.4 Hz), 0.88 (9H, s), 0.07 (6H, dd, s).

### Step (iv)

2-Butoxy-7,8-dihydro-9-[3-fluoro-4-{4-[3-(methoxycarbonylmethyl)phenoxymethyl]piperidin-1-yl}methylbenzyl]-8-oxoadenine

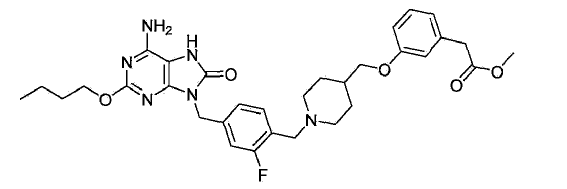

To 2-butoxy-9-[4-(tert-butyldimethylsiloxymethyl)-3-fluorobenzyl]-8-methoxyadenine) 250mg (0.51mmol) obtained in step (iii) in THF (5ml) was added TBAF (1.53mmol) and the mixture was stirred at room temperature for 20 minutes, concentrated and purified by column chromatography. The residue was dissolved in dichloromethane (5 ml) and thereto was added thionyl chloride 93 µl (1.28mmol). After stirring at 40°C for 2 hours, the solution was concentrated and the residue was dissolved in DMF (5ml). Thereto were added 4-[3-(methoxycarbonylmethyl)benzyloxymethyl]piperidine hydrochloride 207 mg (0.69mmol) and diisopropylethylamine 275µl (1.60mmol) and the mixture was stirred at 55°C for 4 hours. To the reaction mixture were added methanol and concentrated sulfuric acid 500µl and the mixture was stirred at 80°C for 1 hour. After neutralization with aqueous ammonia and concentration, to the residue was added water. After extracting with chloroform-methanol, the organic layer was washed with water and saturated brine, dried over Na₂SO₄ and concentrated. The residue was purified by silica gel column chromatography. To the purified solid was added methanol 3ml and chloroform 0.5ml and the resulting solid was filtered to give the titled compound 177mg (0.29mmol) as a white solid.
¹H NMR (DMSO-d₆) δ 10.04 (1H, brs), 7.34 (1H, dd, J = 7.7, 7.7 Hz), 7.21-7.17(1H, m), 7.08-7.06 (2H, m), 6.81-6.78 (3H, m), 6.49 (2H, brs), 4.85 (2H, s), 4.13 (2H, t, J = 6.6 Hz), 3.76 (2H, d, J = 5.8 Hz), 3.62 (2H, s), 3.60 (3H, s), 3.45 (1H, s), 2.82-2.79 (2H, m), 1.69-1.61 (2H, m), 1.61-1.58 (2H, m), 1.39-1.33 (2H, m), 1.33-1.25 (2H, m), 0.89 (3H, t, J = 7.4 Hz).

### Example 30

2-Butoxy-7,8-dihydro-9-[3-fluoro-4-{4-[3-(carboxymethyl)phenoxymethyl]piperidin-1-yl}methylbenzyl]-8-oxoadenine

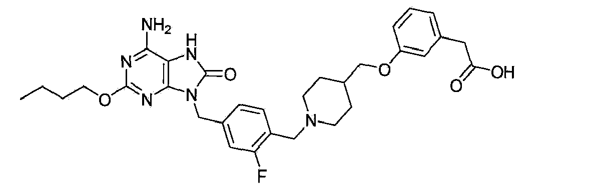

Using 2-butoxy-7,8-dihydro-9-[3-fluoro-4-{4-[3-(methoxycarbonylmethyl)benzyloxymethyl]piperidin-1 -yl}benzyl]-8-oxoadenine 64mg (0.11mmol) obtained by example 29, in the same manner as example 2, there was obtained the titled compound 45mg (0.08mmol) as a white solid. Yield 72%
¹H NMR (DMSO-d₆) δ 12.28 (1H, brs), 10.14 (1H, brs), 7.44-7.40 (1H, m), 7.19 (1H, dd, J = 7.7, 7.7 Hz), 7.16-7.10 (2H, m), 6.82-6.77 (2H, m), 6.55 (2H, brs), 4.87 (2H, s), 4.13 (2H, t, J = 6.6 Hz), 3.79-3.77 (2H, m), 3.51 (2H, s), 3.51 (1H, s), 3.35 (2H, s), 2.92-2.80 (2H, m), 2.10-1.89 (2H, m), 1.81-1.71 (2H, m), 1.63-1.58 (2H, m), 1.39-1.32 (2H, m), 0.89 (3H, t, J = 7.4 Hz).

### Example 31

[3-(4-{[4-(6-Amino-2-butoxy-8-oxo-7,8-dihydropurin-9-ylmethyl)benzyl]ethylamino}butoxy)phenyl]acetic acid methyl ester

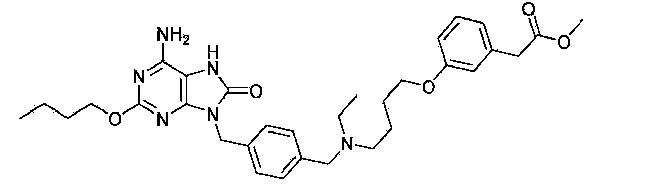

In the same manner as example 5 step (iii), there was obtained the titled compound as a white solid. Yield 20%
¹H NMR (DMSO-d₆) δ 10.03 (1H, brs), 7.18-7.26 (5H, m), 6.76-6.81 (3H, m), 6.47 (2H, brs), 4.82 (2H, s), 4.14 (2H, t, J = 6.6 Hz), 3.90 (2H, t, J = 6.4 Hz), 3.63 (2H, s), 3.60 (3H, s), 3.47 (2H, s), 2.38-2.42 (4H, m), 1.54-1.69 (6H, m), 1.37 (2H, m), 0.88-0.96 (6H, m).

### Example 32

[3-(3-{4-[4-(6-Amino-2-butoxy-8-oxo-7, 8-dihydropurin-9-ylmethyl)benzyl]piperazin-1-yl}propoxy)phenyl]acetic acid methyl ester

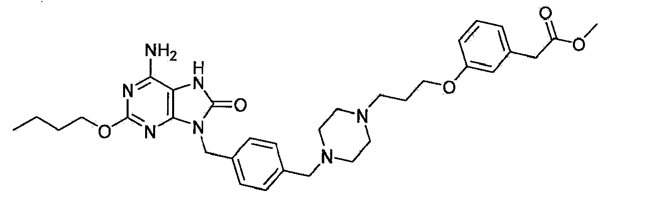

In the same manner as example 5 step (iii), there was obtained the titled compound as a white solid. Yield 33%
¹H NMR (DMSO-d₆) δ 9.96 (1H, brs), 7.18-7.29 (5H, m), 6.80-6.86 (3H, m), 6.46 (2H, brs), 4.83 (2H, s), 4.14 (2H, t, J = 6.6 Hz), 3.95 (2H, t, J = 6.4 Hz), 3.63 (2H, s), 3.60 (3H, s), 3.40 (2H, s), 2.34-2.42 (8H, m), 1.81-1.84 (2H, m), 1.58-1.65 (2H, m), 1.34-1.40 (2H, m), 0.90 (3H, t, J = 7.4 Hz).

### Example 33

[3-(2-{4-[5-(6-Amino-2-butoxy-8-oxo-7,8-dihydropurin-9-ylmethyl)pyridin-2-yl]-[1,4]diazepan-1-yl}ethoxy)phenyl]acetic acid methyl ester

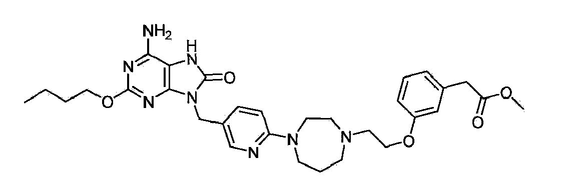

In the same manner as example 12, there was obtained the titled compound as a white solid. Yield 47%
¹H NMR (DMSO-d₆) δ 9.95 (1H, brs), 8.07 (1H, d, J = 3.2 Hz), 7.47 (1H, dd, J = 8.8, 2.4 Hz), 7.20 (1H, t, J = 7.6 Hz), 6.79-6.82 (3H, m), 6.56 (1H, d, J = 8.8 Hz), 6.44 (2H, brs), 4.69 (2H, s), 4.17 (2H, t, J = 6.6 Hz), 4.00 (2H, t, J = 6.0 Hz), 3.56-3.68 (9H, m), 2.77-2.84 (4H, m), 2.63 (2H, m), 1.83 (2H, m), 1.63-1.67 (2H, m), 1.39-1.40 (2H, m), 0.92 (3H, t, J = 7.4 Hz).

### Example 34

[3-(2-{4 [4-(6-Amino-2-butoxy-8-oxo-7,8-dihydropurin-9-ylmethyl) -2-nitrophenyl]-[1,4]diazepan-1-yl}ethoxy)phenyl]acetic acid methyl ester

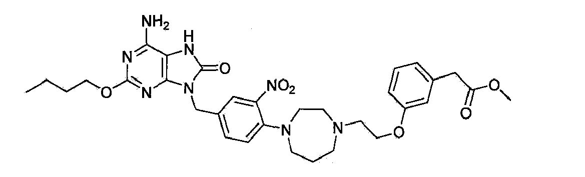

In the same manner as example 12, there was obtained the titled compound as a white solid. Yield 61%
¹H NMR (DMSO-d₆) δ 10.01 (1H, brs), 7.73 (1H, s), 7.43 (1H, dd, J = 8.8, 2.2 Hz), 7.16-7.21 (2H, m), 6.79-6.87 (3H, m), 6.48 (2H, brs), 4.79 (2H, s), 4.16 (2H, t, J = 6.6 Hz), 4.00 (2H, t, J = 6.0 Hz), 3.63 (2H, s), 3.60 (3H, s), 3.25 (9H, m), 2.85 (2H, m), 2.79 (2H, t, J = 5.6 Hz), 2.68 (2H, m), 1.83 (2H, m), 1.61-1.66 (2H, m), 1.37-1.41 (2H, m), 0.91 (3H, t, J = 7.4 Hz).

### Example 35

[3-(2-{4-[2-Amino-4-(6-amino-2-butoxy-8-oxo-7,8-dihydropurin-9-ylmethyl)phenyl]-[1,4]diazepan-1-yl}ethoxy)phenyl]acetic acid methyl ester

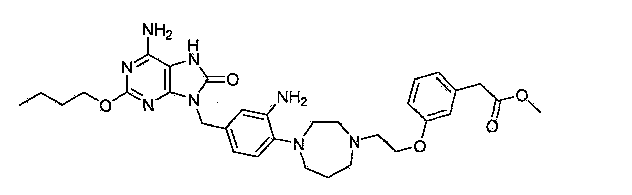

To a solution of the compound 0.35g obtained in example 34 in THF 20ml was added 10% Pd(OH)₂/C 0.1g and the mixture was stirred under an atmosphere of hydrogen for 5 hours. After removal of catalyst by filtration, the solvent was removed by distillation. To the residue was added methanol to give the titled compound as a pale yellow solid. Yield 32%
¹H NMR (CDCl₃) δ 9.97 (1H, s), 7.25-7.29 (1H, m), 6.86-6.93 (4H, m), 6.61(1H, s), 6.48-6.54 (3H, m), 4.82 (2H, brs), 4.72 (2H, s), 4.20 (2H, t, J = 6.6 Hz), 4.10 (2H, t, J = 6.0 Hz), 3.70 (2H, s), 3.65 (3H, s), 2.90-3.30 (8H, m), 1.87 (2H, m), 1.66-1.71 (2H, m), 1.41-1.47 (2H, m), 0.97 (3H, t, J = 7.4 Hz).

### Example 36

Methyl (3-{2-[(1-{4-[(6-amino-2-butoxy-8-oxo-7,8-dihydra-9H-purin-9-yl)methyl]benzyl}piperidin-4-yl)(methyl)amino]ethoxy}phenyl)acetate

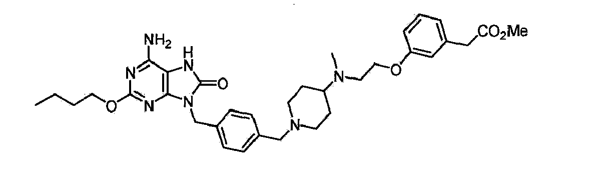

### Step (i)

tert-Butyl 4-[{2-[3-(2-methoxy-2-oxoethyl)phenoxy]ethyl}(methyl)amino]piperidine-1-carboxylate

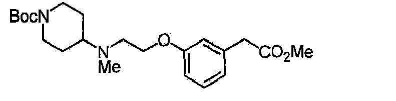

To a mixture of tert-butyl 4-oxopiperidine-1-carboxylate (399mg, 2mmol), methyl {3-[2-(methylamino)ethoxy]phenyl}acetate (447mg, 2mmol) and chloroform (8ml) was added moderate amount of acetic acid, followed by stirring at room temperature for 1 hour. Thereto was added sodium triacetoxyborohydride (424mg, 2mmol) and the mixture was reacted at room temperature over night. After being cooled, thereto was added saturated sodium hydrogencarbonate and the mixture was extracted with chloroform. The organic layer was washed with saturated brine and dried over sodium sulfate. After removal of the solvent, the residue was purified by silica gel column chromatography to give the object compound 545mg as a colorless oil. Yield 67%
¹H NMR (CDCl₃) δ7.22 (1H, t, J = 7.8 Hz), 6.89-6.75 (3H, m), 4.20-4.10 (1H, br), 4.03 (2H, t, J = 6.0 Hz), 3.69 (3H, s), 3.59 (2H, s), 2.86 (2H, t, J = 6.0 Hz), 2.66-2.51 (3H, m), 2.37 (3H, s), 1.84-1.64 (4H, m), 1.46 (9H, s).

### Step (ii)

Methyl (3-{2-[methyl(piperidin-4-yl)amino]ethoxy}phenyl)acetate

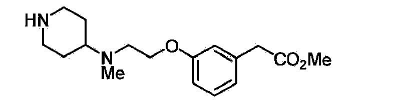

To methyl (3-{2-[methyl(piperidin-4-yl)]aminoethoxy}phenyl)acetate (545mg, 1.3mmol) was added 4N hydrochloric acid-dioxane (6ml) and the mixture was stirred at room temperature for 2 hours. After concentration, the residue was subjected to azeotropic distillation three times with methanol. The residue was neutralized with saturated sodium hydrogencarbonate and extracted with chloroform. The organic layer was washed with saturated brine, dried over sodium sulfate and the solvent was removed by distillation. The residue was dried in vacuo to give the object compound 325mg as an orange oil. Yield 79%
¹H NMR (CDCl₃) δ7.22 (1H, t, J = 7.8 Hz), 6.90-6.75 (3H, m), 4.03 (2H, t, J = 6.1 Hz), 3.64 (3H, s), 3.58 (2H, s), 3.20-3.09 (2H, m), 2.87 (2H, t, J = 6.1 Hz), 2.65-2.45 (3H, m), 2.38 (3H, s), 1.85-1.60 (3H, m), 1.51-1.33 (2H, m).

### Step (iii)

Methyl (3-{2-[(1-{4-[(6-amino-2-butoxy-8-methoxy-9H-purin-9-yl)methyl]benzyl}piperidin-4-yl)(methyl)amino]ethoxy}phenyl)acetate

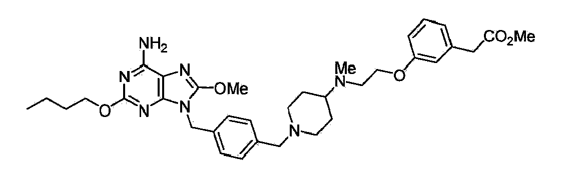

To {4-[(6-amino-2-butoxy-8-methoxy-9H-purin-9-yl)methyl]phenyl}methanol (715mg, 2mmol) in tetrahydrofuran (20ml) was added triethylamine (0.34ml, 2.4mmol) under an atmosphere of nitrogen and thereto was added potion wise under ice cooling methanesulfonyl chloride (0.159ml, 2.05mmol), followed by reacting under ice cooling for 1 hour. The reaction was quenched with cooled 10% aqueous citric acid and the mixture was extracted with ethyl acetate. The organic layer was washed with 10% aqueous citric acid, water, saturated sodium hydrogencarbonate and saturated brine, and dried over sodium sulfate.
The solvent was removed to give 4-[(6-amino-2-butoxy-8-methoxy-9H-purin-9-yl)methyl]benzyl methanesulfonate (131mg, 0.3mmol) as a yellow oil. The product in dimethylformamide (3ml) was added to a suspension of methyl (3-{2-[methyl(piperidin-4-yl)amino]ethoxy}phenyl)acetate dihydrochloride (118mg, 0.32mmol) previously prepared and potassium carbonate (69 mg, 0.5mmol) and dimethylformamide (2ml) and the mixture was reacted at 80°C for 2 hours. After concentration, to the residue was added water-chloroform and the mixture was separated by a separating funnel. The organic layer was washed with saturated brine and dried over sodium sulfate. After removal of the solvent by distillation, the residue was purified by silica gel column chromatography to give the object compound 98mg as a pale yellow oil. Yield 50%
¹H NMR (CDCl₃) δ7.35-7.14 (5H, m), 6.89-6.73 (3H, m), 5.15 (2H, s), 5.07 (2H, s), 4.30 (2H, t, J = 6.6 Hz), 4.09 (3H, s), 4.02 (2H, t, J = 6.1 Hz), 3.68 (3H, s), 3.58 (2H, s), 3.44 (2H, s), 2.95-2.87 (2H, m), 2.85 (2H, t, J = 6.1 Hz), 2.46-2.38 (1H, m), 1.93 (3H, s), 1.98-1.87 (2H, m), 1.80-1.65 (4H, m), 1.60-1.45 (4H, m), 0.96 (3H, t, J = 7.3 Hz).

### Step (iv)

Methyl (3-{2-[(1-{4-[(6-amino-2-butoxy-8-oxo-7,8-dihydro-9H-purin-9-yl)methyl]benzyl}piperidin-4-yl)(methyl)amino]ethoxy}phenyl)acetate

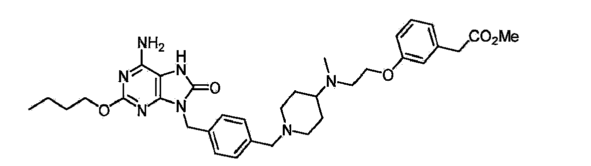

To methyl (3-{2-[(1-{4-[(6-amino-2-butoxy-8-methoxy-9H-purin-9-yl)methyl]benzyl}piperidin-4-yl)(methyl)amino]ethoxy}phenyl)acetate (53mg, 0.082mmol) was added 4M hydrochloric acid/methanol (2 ml) and the mixture was stirred at room temperature for 5 hours. After concentration, the residue was subjected to azeotropic distillation three times with methanol. To the residue was added saturated sodium hydrogencarbonate to neutralize and the resulting solid was filtered and washed with water to give the titled compound 43mg as a white solid. Yield 85%
¹H NMR (DMSO-d₆) δ9.96 (1H, s), 7.28-7.15 (5H, m), 6.85-6.76 (3H, m), 6.44 (2H, s), 4.83 (2H, s), 4.14 (2H, t, J = 6.6 Hz), 3.97 (2H, t, J = 6.0 Hz), 3.62 (2H, s), 3.60 (3H, s), 3.38 (2H, s), 2.84-2.73 (4H, m), 2.43-2.38 (1H; m), 2.25 (3H, s), 1.95-1.83 (2H, m), 1.65-1.60 (4H, m), 1.43-1.33 (4H, m), 0.90 (3H, t, J = 7.3 Hz).

### Example 37

Methyl (3-{2-[{2-[{4-[(6-amino-2-butoxy-8-oxo-7,8-dihydro-9H-purin-9-yl)methyl]benzyl}(methyl)amino]ethyl}(methyl)amino]ethoxy}phenyl)acetate

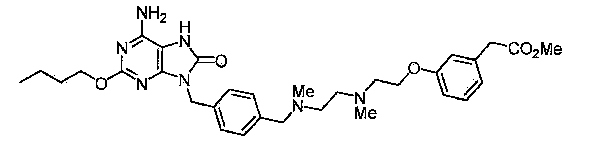

### Step (i)

Methyl (3-{2-[{2-[(tert-butoxycarbonyl)(methyl)amino]ethyl}(methyl)amino]ethoxy}phenyl)acetate

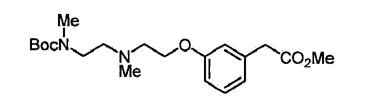

N,N'-Dimethyethane-1,2-diamine (882mg, 10mmol) and potassium carbonate (415mg, 3mmol) were suspended in dimethylformamide (6ml) and thereto was added portion wise at room temperature methy [3-(2-bromoethoxy)phenyl]acetate (546mg, 2mmol), followed by stirring at room temperature for 2 hours. Thereto was added portion wise di-tert-butyldicarbonate (6.55g, 30mmol) taking care for emission of gas, and the mixture was stirred overnight. After concentration, thereto was added water-ethyl acetate and the solution was separated by a separating funnel. The organic layer was washed with aqueous 10% citric acid, water, saturated sodium hydrogencarbonate and saturated brine, and dried over sodium sulfate. After removal of the solvent by distillation, the residue was purified by silica gel column chromatography to give the object compound 630mg as an orange oil. Yield 82%
¹H NMR (CDCl₃) δ7.22 (1H, t, J = 7.8 Hz), 6.88-6.77 (3H, m), 4.04 (2H, t, J = 5.8 Hz), 3.69 (3H, s), 3.59 (2H, s), 3.40-3.30 (2H, br), 2.87 (3H, s), 2.84 (2H, t, J = 5.8 Hz), 2.65-2.55 (2H, br), 2.38 (3H, s), 1.45 (9H, s).

### Step (ii)

Methyl [3-(2-{methyl[2-(methylamino)ethyl]amino}ethoxy)phenyl]acetate

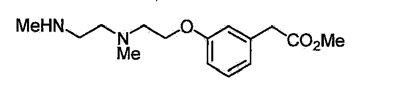

To methyl (3-{2-[{2-[(tert-butoxycarbonyl) (methyl) amino] ethyl} (methyl) amino] ethoxy}phenyl) acetate (630mg, 1.65mmol) were added 4N hydrochloric acid-dioxane (6ml) and methanol (1.5ml), and the mixture was reacted at room temperature for 2 hours. After concentration and neutralization with saturated sodium hydrogencarbonate, the mixture was extracted with chloroform. The organic layer was washed with saturated brine and dried over sodium sulfate. The solvent was removed by distillation to give the object compound 413mg as an orange oil. Yield 89%
¹H NMR (CDCl₃) δ7.22 (1H, t, J = 7.8 Hz), 6.88-6.77 (3H, m), 4.04 (2H, t, J = 5.8 Hz), 3.74 (3H, s), 3.64 (2H, s), 2.80 (2H, s), 2.70-2.64 (2H, m), 2.63-2.55 (2H, m), 2.34 (3H, s), 1.75 (1H, br).

### Step (iii)

Methyl (3-{2-[{2-[{4-[(6-amino-2-butoxy-8-oxo-7,8-dihydro-9H-purin-9-yl)methyl]benzyl}(methyl)amino]ethyl}(methyl)amino]ethoxy}phenyl)acetate

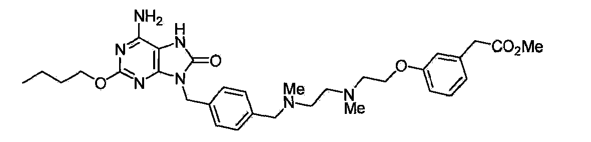

In the same manner as example 36, there was obtained the titled compound 73mg as a white solid. Yield 57%
¹H NMR (DMSO-d₆) δ9.94 (1H, s), 7.28-7.13 (5H, m), 6.83-6.74 (3H, m), 6.41 (2H, s), 4.82 (2H, s), 4.14 (2H, t, J = 6.6 Hz), 3.98 (2H, t, J = 5.8 Hz), 3.59 (5H, s), 3.37 (2H, s), 2.66 (2H, t, J = 5.8 Hz), 2.39 (2H, t, J = 7.0 Hz), 2.32 (2H, t, J = 7.0 Hz), 2.21 (3H, s), 2.05 (3H, s), 1.67-1.55 (2H, m), 1.42-1.32 (2H, m), 0.90 (3H, t, J = 7.3 Hz).

### Example 38

Methyl (3-{2-[4-{4-[(6-amino-2-butoxy-8-oxo-7,8-dihydro-9H-purin-9-yl)methyl]benzyl}(methyl)amino piperidin-1-yl]ethoxy}phenyl)acetate

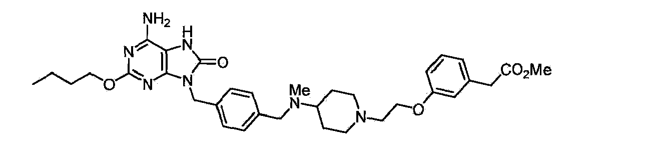

### Step (i)

Methyl [3-(2-{4-[(tert-butoxycarbonyl)(methyl)ainino]pipendin-1-yl}ethoxy)phenyl] acetate

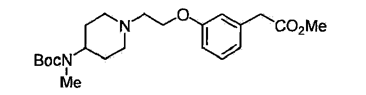

tert-Butyl methyl(piperidin-4-yl)carbamate (331mg, 1.54mmol) and potassium carbonate (276mg, 2mmol) were suspended in dimethylformamide (6ml) and thereto was added portion wise at room temperature methyl [3-(2-bromoethoxy)phenyl]acetate (382mg, 1.4mmol), followed by stirring at room temperature overnight. After concentration, to the residue was added water-ethyl acetate and the mixture was separated by a separating funnel. The organic layer was washed with water and saturated brine and dried over sodium sulfate. After removal of the solvent by distillation, the residue was purified by silica gel column chromatography to give the object compound 562mg as a colorless oil. Yield 89%.
¹H NMR (CDCl₃) δ7.22 (1H, t, J = 7.8 Hz), 6.89-6.75 (3H, m), 4.08 (2H, t, J = 5.8 Hz), 3.69 (3H, s), 3.59 (2H, s), 3.09-23.02 (2H, m), 2.79 (2H, t, J = 5.8 Hz), 2.73 (3H, s), 2.23-2.13 (2H, m), 1.79-1.59 (5H, m), 1.46 (9H, s).

### Step (ii)

Methyl (3-{2-[4-(methylamino)piperidin-1-yl]ethoxy}phenyl)acetate

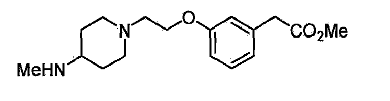

To methyl [3-(2-{4-[(tert-butoxycarbonyl)(methyl)amino]piperidin-1-yl}ethoxy)phenyl]acetate (562mg, 1.38mmol) were added 4N hydrochloric acid-dioxane (6ml) and methanol (1.5ml) and the mixture was reacted at room temperature for 2 hours. After concentration and neutralization with saturated sodium hydrogencarbonate, the mixture was extracted with chloroform. The organic layer was washed with saturated sodium hydrogencarbonate and dried over sodium sulfate. The solvent was removed by distillation to give the object compound 370mg as an orange oil. Yield 87%
¹H NMR (CDCl₃) δ7.22 (1H, t, J = 7.8 Hz), 6.88-6.78 (3H, m), 4.09 (2H, t, J = 6.0 Hz), 3.69 (3H, s), 3.59 (2H, s), 3.00-2.92 (2H, m), 2.79 (2H, t, J = 6.0 Hz), 2.43 (3H, s), 2.40-2.34 (1H, m), 2.22-2.12 (2H, m), 1.93-1.85 (2H, m), 1.60-1.50 (1H, br), 1.43-1.35 (2H, m).

### Step (iii)

Methyl (3-{2-[4-{4-[(5-amino-2-butoxy-8-oxo-7,8-dihydro-9H-purin-9-yl)methyl]benzyl}(methyl)aminopiperidin-1-yl]ethoxy}phenyl)acetate

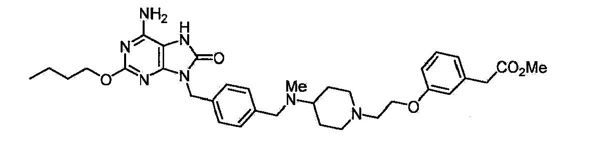

In the same manner as example 36, there was obtained the titled compound 76mg as a white solid. Yield 55%
¹H NMR (DMSO-d₆) δ9.97 (1H, s), 7.31-7.17 (5H, m), 6.85-6.78 (3H, m), 6.43 (2H, s), 4.84 (2H, s), 4.14 (2H, t, J = 6.6 Hz), 4.04 (2H, brs), 3.62 (2H, s), 3.61 (3H, s), 3.59-3.48 (2H, m), 3.08-2.98 (2H, br), 2.80-2.65 (2H, br), 2.20-2.03 (5H, br), 1.85-1.55 (7H, m), 1.43-1.33 (2H, m), 0.91 (3H, t, J = 7.3 Hz).

### Example 39

Methyl (3-{2-[{[1-(4-{[6-amino-2-(2-methoxyethoxy)-8-oxo-7,8-dihydro-9H-purin-9-yl] methyl}benzyl)-4-hydroxypiperidin-4-yl]methyl}(methyl)amino]ethoxy}phenyl)acetate

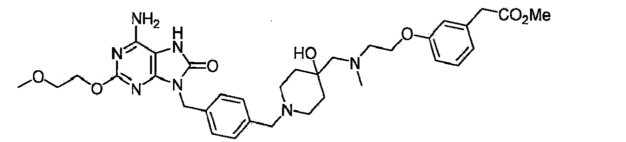

### Step (i)

9-[4-(Chloromethyl)benzyl]-8-methoxy-2-(2-methoxyethoxy)-9H-purin-6-amine 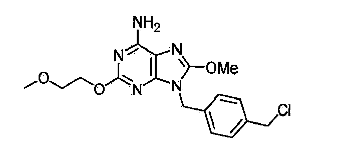 8-Methoxy-2-(2-methoxyethoxy)-9H-purine-6-amine (7.18g, 30mmol) and potassium carbonate (6.22g, 45mmol) were suspended in dimethylformamide (150ml) and thereto was added at room temperature 1,4-bis(chloromethyl)benzene (25g, 142mmol), followed by stirring at room temperature overnight. To the reaction mixture was added cold water and the mixture was stirred. The resulting crystals were filtered, washed with water and dried by aeration. The crystals on filter paper were dissolved in chloroform, dried over sodium sulfate and the filtrate was purified by silica gel column chromatography to give white crystals. The crystals were washed with ethyl acetate, filtered, washed with cold ethyl acetate and concentrated in vacuo to give the object compound 4.19g as white crystals. Yield 37%
¹H NMR (CDCl₃) δ7.38-7.29 (4H, m), 5.61 (2H, brs), 5.08 (2H, s), 4.55 (2H, s), 4.81 (2H, t, J = 5.0 Hz), 4.11 (3H, s), 3.75 (2H, t, J = 5.0 Hz), 3.40 (3H, s).

### Step (ii)

tert-Butyl 4-hydroxy-4-{[{2-[3-(2-methoxy-2-oxoethyl)phenoxy]ethyl}(methyl)amino]methyl}piperidine-1-carboxylate

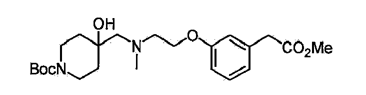

To methyl {3-[2-(methylamino)ethoxy]phenyl}acetate (1.21g, 5.4mmol) in methanol (10ml) was added tert-butyl 1-oxa-6-azaspiro[2.5]octane-6-carboxylate (1.15g, 5.4mmol) and the mixture was refluxed under heating for 3 hours. After being cooled, the reaction mixture was concentrated and the residue was purified by silica gel column chromatography to give the object compound 2.09g as a colorless oil. Yield 88%
¹H NMR (CDCl₃) δ7.24 (1H, t, J = 7.8 Hz), 6.88 (1H, t, J = 7.4 Hz), 6.85-6.78 (2H, m), 4.23-3.78 (4H, br), 3.69 (3H, s), 3.60 (2H, s), 3.22-2.90 (5H, br), 2.75-2.35 (5H, br), 1.90-1.60 (2H, br), 1.45 (9H, s), 1.44-1.35 (2H, m).

### Step (iii)

Methyl (3-{2-[{[1-(4-{[6-amino-2-(2-methoxyethoxy)-8-oxo-7,8-dihydro-9H-purin-9-yl]methyl}benzyl)-4-hydroxypiperidin-4-yl]methyl}(methyl)amino]ethoxy}phenyl)acetate

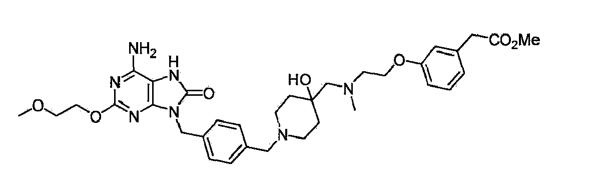

tert-Butyl 4-hydroxy-4-{[{2-[3-(2-methoxy-2-oxoethyl)phenoxy]ethyl}(methyl)amino]methyl}piperidine-1-carboxylate (2.09g, 4.78mmol) was dissolved in chloroform (24ml). The solution was ice-cooled under an atmosphere of nitrogen solution and thereto was added portion wise trimethylsilyltrifluoromethane sulfonate (1.93ml, 10mmol), followed by a reaction under ice cooling. To the reaction mixture was added under ice cooling saturated sodium hydrogencarbonate. After stirring for a while, the mixture was salted out and extracted with chloroform. The organic layer was dried over sodium sulfate. The solvent was removed by distillation to give an orange colored oily product (2.10g) containing methyl (3-{2-[methyl({4-[(trimethylsilyl)oxy]piperidin-4-yl}methyl)amino]ethoxy}phenyl)acetate mainly and methyl(3-{2-[[(4-hydroxypiperidin-4-yl)methyl](methyl)amino]ethoxy}phenyl)acetate partly. Part of thus obtained oily product (336mg, about 1mmol) was reacted with 6-amino-9-[4-(chloromethyl)benzyl]-2-(2-methoxyethoxy)-7,9-dihydro-8H-purin-8-one (200mg, 0.55mmol) previously prepared in dimethylformamide (3ml) under potassium carbonate as a base at room temperature overnight. To the reaction mixture was added water and the mixture was stirred for a while. The aqueous layer was removed and the residue was subjected to azeotoropic distillation with methanol. After treating under ice cooling with 10% hydrochloric acid-methanol for 2 hours, the mixture was concentrated and the residue was subjected to azeotropic distillation with methanol three times. To the residue was added saturated sodium hydrogencarbonate to neutralize and the resulting solid was filtered, washed with water and dried in vacuo to give the object compound 228mg as a pale brown solid. Yield 62%
¹H NMR (DMSO-d₆) δ9.96 (1H, s), 7.30-7.15 (5H, m), 6.83-6.77 (3H, m), 6.45 (2H, s), 4.83 (2H, s), 4.26 (2H, t, J = 4.7 Hz), 4.00 (2H, t, J = 5.8 Hz), 3.85 (1H, brs), 3.63-3.55 (7H, m), 3.49-3.35 (2H, br), 3.27 (3H, s), 2.80 (2H, t, J = 5.8 Hz), 2.40-2.32 (7H, m), 2.31-2.23 (2H, m), 1.56-1.47 (2H, m), 1.44-1.37 (2H, m).

### Example 40

Methyl (3-{2-[9-(4-{[6-amino-2-(2-methoxyethoxy)-8-oxo-7,8-dihydro-9H-purin-9-yl]methyl}benzyl)-1-oxa-4,9-diazaspiro[5.5]undec-4-yl]ethoxy}phenyl)acetate

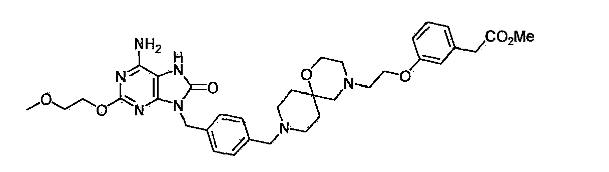

### Step (i)

tert-Butyl 4-{2-[3-(2-methoxy-2-oxoethyl)phenoxy]ethyl}-1-oxa-4,9-diazaspiro[5.5]undecane-9-carboxylate

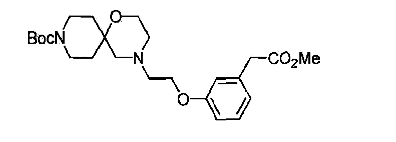

tert-Butyl 1-oxa-4,9-diazaspiro[5.5]undecane-9-carboxylate (769mg, 3mmol) and potassium carbonate (622mg, 4.5mmol) were suspended in dimethylformamide (15ml) and thereto was added at room temperature methyl [3-(2-bromoethoxy)phenyl]acetate (1.09g, 4mmol), followed by stirring at 85°C for 15 hours. After being cooled, thereto was added water and the mixture was extracted with ethyl acetate. The organic layer was washed with water and saturated brine and dried over sodium sulfate.
After removal of the solvent by distillation, the residue was purified by silica gel column chromatography to give the object compound 565mg as a colorless oil. Yield 42%
¹H NMR (CDCl₃) δ7.27-7.23 (1H, m), 6.93-6.78 (3H, m), 4.13-4.06 (2H, m), 3.90-3.65 (4H, br), 3.71 (3H, s), 3.61 (2H, s), 3.22-3.15 (2H, br), 2.73 (2H, br), 2.54 (2H, brs), 2.37 (2H, brs), 2.00-1.91 (2H, br), 1.51-1.41 (2H, br), 1.46 (9H, s).

### Step (ii)

Methyl {3-[2-(1-oxa-4,9-diazaspiro[5.5]undec-4-yl)ethoxy]phenyl}acetate

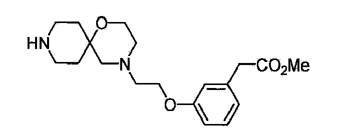

To tert-butyl 4-{2-[3-(2-methoxy-2-oxoethyl)phenoxy]ethyl}-1-oxa-4,9-diazaspiro[5.5]undecane-9-carboxylate (565mg, 1.26mmol) was added 4N hydrochloric acid-dioxane (5ml) and methanol (1ml) and the mixture was reacted at room temperature for 2 hours. The mixture was concentrated and the residue was subjected to azeotropic distillation with methanol three times. To the residue was added saturated sodium hydrogencarbonate to neutralize and the mixture was extracted with chloroform. The organic layer was washed with saturated brine and dried over sodium sulfate. The solvent was removed by distillation to give the object compound 474mg as a colorless oil.
¹H NMR (CDCl₃) δ7.26-7.19 (1H, m), 6.92-6.72 (3H, m), 4.06 (2H, t, J = 5.7 Hz), 3.74-3.70 (2H, m), 3.69 (3H, s), 3.59 (2H, s), 3.52-3.40 (1H, br), 3.02-2.93 (2H, m), 2.91-2.85 (2H, br), 2.71 (2H, t, J = 5.7 Hz), 2.55-2.50 (2H, m), 2.37 (2H, s), 2.00-1.92 (2H, m), 1.65-1.52 (2H, m).

### Step (iii)

Methyl (3-{2-[9-(4-{[6-amino-2-(2-methoxyethoxy)-8-oxo-7,8-dihydro-9H-purin-9-yl]methyl}benzyl)-1-oxa-4,9-diazaspiro[5.5]undec-4-yl]ethoxy}phenyl)acetate

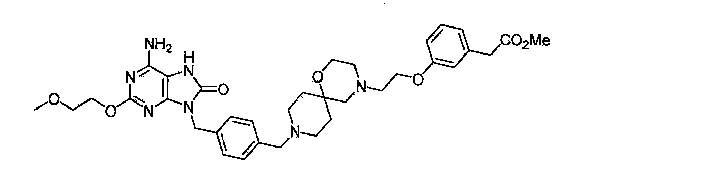

Methyl {3-[2-(1-oxa-4, 9-diazaspiro[5.5]undec-4-yl)ethoxy]phenyl}acetate (348mg, 1mmol) and potassium carbonate (138mg, 1mmol) were suspended in dimethylformamide (3ml), and thereto was added 6-amino-9-[4-(chloromethyl)benzyl]-2-(2-methoxyethoxy)-7,9-dihydro-8H-purin-8-one (200mg, 0.55mmol), followed by reaction at room temperature overnight. To the reaction mixture was added water. After stirring for a while, the resulting solid was filtered, washed with water and dried in vacuo. The residue was purified by silica gel column chromatography to give the titled compound 154mg as a colorless solid. Yield 41 %
¹H NMR (DMSO-d₆) δ9.92 (1H, s), 7.32-7.16 (5H, m), 6.84-6.78 (3H, m), 6.41 (2H, s), 4.82 (2H, s), 4.26 (2H, t, J = 4.8 Hz), 4.04 (2H, t, J = 5.7 Hz), 3.63-3.52 (9H, m), 3.42-3.36 (2H, m), 3.27 (3H, s), 2.61 (2H, t, J = 5.7 Hz), 2.48-2.15 (8H, m), 1.84-1.72 (2H, m), 1.54-1.40 (2H, m).

### Example 41

Methyl {3-[({3-[(4-{[6-amino-2-(2-methoxyethoxy)-8-oxo-7,8-dihydro-9H-purin-9-yl]methyl}benzyl) (methyl)amino]propyl}amino)methyl]phenyl}acetate

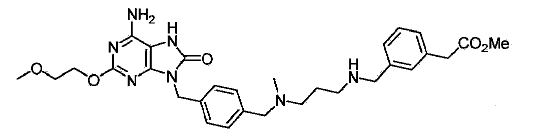

### Step (i)

(3-{[(tert-Butoxycarbonyl)(3-hydroxypropyl)amino]methyl}phenyl)acetie acid

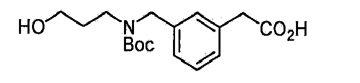

3-Aminopropan-1-ol (3.76 g, 10mmol) in dimethylformamide (100 ml) was stirred at room temperature, and thereto was added portion wise under stirring methyl [3-(bromomethyl)phenyl]acetate (2.43g, 10mmol) in dimethylformamide (20ml), followed by stirring at room temperature for 4 hours. To the reaction mixture were added under ice cooling potassium carbonate (1.66g, 12mmol) and di-tert-butyldicarbonate (13.10g, 60mmol). After reaction at room temperature overnight, to the reaction mixture was added water and the mixture was extracted with ethyl acetate-hexane (3 : 1). The organic layer was washed with water and saturated brine, and dried over sodium sulfate. After removal of the solvent by distillation, the residue was dried in vacuo, dissolved in tetrahydrofuran (80ml) - methanol (20ml). Thereto was added portion wise under ice cooling 1N aqueous sodium hydroxide. After reaction at room temperature for 2 hours and removal of the solvent, to the residue was added water (200ml) and the mixture was washed eight times with hexane-ether (1 : 1) (150ml). The aqueous layer was acidified (around pH 4) with 10% aqueous potassium hydrogensulfate and extracted with ethyl acetate. The organic layer was washed with saturated brine and dried over sodium sulfate. The solvent was removed by distillation to give the object compound 2.41g as a colorless oil. Yield 74%

### Step (ii)

Methyl (3-{[(tert-butoxycarbonyl)(3-hydroxypropyl)amino]methyl}phenyl)acetate

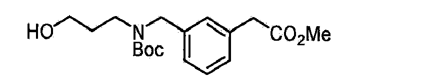

To (3-{[(tert-butoxycarbonyl)(3-hydroxypropyl)amino]methyl}phenyl)acetic acid (2.10g, 6.5mmol) in toluene (45.5ml)-methanol(13ml) was added portion wise at room temperature trimethylsilyldiazomethane (2M hexane, 4.3ml, 8.45mmol). After stirring for 3 hours and removal of the solvent, the residue was purified by silica gel column chromatography to give the object compound 1.12g as a colorless oil. Yield 51%
¹H NMR (CDCl₃) δ7.33-7.27 (1H, m), 7.21-7.12 (3H, m), 4.38 (2H, s), 3.69 (3H, s), 3.62 (2H, s), 3.55 (2H, t, J = 5.6 Hz), 3.41-3.27 (2H, br), 2.98 (2H, brs), 1.66-1.59 (2H, m), 1.47 (9H, s).

### Step (iii)

8-Methoxy-2-(2-methoxyethoxy)-9-{4-[(methylamino)methyl]benzyl}-9H-purin-6-amine

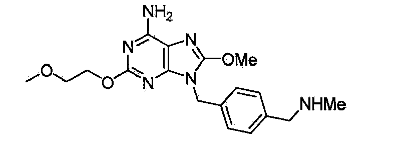

9-[4-(Chloromethyl)benzyl]-8-methoxy-2-(2-methoxyethoxy)-9H-purin-6-amine (1.13g, 3mmol) was added a mixture of 30% methylaminemethanol (20ml) and methanol (10ml), and the mixture was reacted at room temperature for 3 hours. After removal of the solvent by distillation, the residue was subjected to azeotropic distillation with methanol three times, and the resulting crystals were washed with methanol and filtered. The crystals were dried in vacuo to give the object compound 724mg as white crystals. Yield 59%
¹H NMR (CDCl₃) δ7.30-7.26 (4H, m), 5.39 (2H, s), 5.06 (2H, s), 4.45 (2H, t, J = 4.9 Hz), 4.08 (3H, s), 3.77-3.71 (4H, m), 3.41 (3H, s), 2.98-2.72 (1H, br), 2.44 (3H, s).

### Step (iv)

Methyl (3-{[(3-[(4-{[6-amino-8-methoxy-2-(2-methoxyethoxy)-9H-purin-9-yl]methyl}benzyl)(methyl)amino]propyl}(tert-butoxycarbonyl)amino]methyl}phenyl)acetate

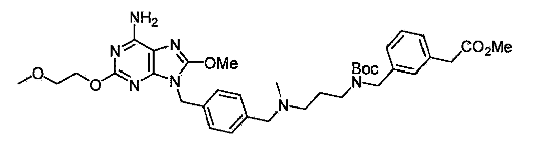

To methyl (3-{[(tert-butoxycarbonyl)(3-hydroxypropyl)amino]methyl}phenyl)acetate (1.01g, 3mmol) in tetrahydrofuran (15 ml) was added triethylamine (0.63ml, 4.5mmol) and thereto was added portion wise under ice cooling methanesulfonyl chloride (0.26ml, 3.3mmol). After reaction under ice cooling for 1 hour, the reaction mixture was diluted with cold water and extracted with ethyl acetate. The organic layer was washed with 10% aqueous citric acid, water, saturated sodium hydrogencarbonate and saturated brine, and dried over sodium sulfate. After removal of the solvent, the residue was diluted with dimethylformamide (10ml) and thereto were added potassium carbonate (691mg, 5mmol) and 8-methoxy-2-(2-methoxyethoxy)-9-{4-[(methylamino)methyl]benzyl}-9H-purin-6-amine (724mg, 1.94mmol), followed by stirring at 60°C for 2 hours. After being cooled, thereto was added water and the mixture was extracted with ethyl acetate. The organic layer was washed with water and saturated brine, and dried over sodium sulfate. After removal of the solvent by distillation, the residue was purified by silica gel column chromatography and further purified by PTLC to give the object compound 145mg as a colorless oil. Yield 10%
¹H NMR (CDCl₃) δ7.64-7.22 (5H, br), 7.21-7.05 (3H, br), 5.14 (2H, s), 5.07 (2H, s), 4.45 (2H, t, J = 4.9 Hz), 4.39 (2H, br), 4.09 (3H, s), 3.75 (2H, t, J = 4.9 Hz), 3.68 (3H, s), 3.61 (2H, s), 3.42 (3H, s), 3.39-3.13 (3H, br), 3.11-2.88 (1H, br), 2.59-1.92 (4H, br), 1.88-1.62 (3H, br), 1.43 (9H, s).

### Step (v)

Methyl {3-[({3-[(4-{[6-amino-2-(2-methoxyethoxy)-8-oxo-7,8-dihydro-9H-purin-9-yl]methyl}benzyl)(methyl)amino]propyl}amino)methyl]phenyl}acetate)

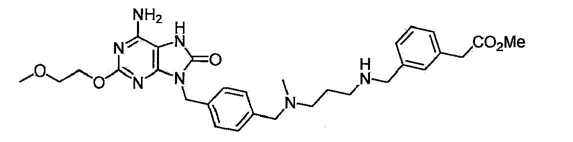

To methyl (3-{[{3-[(4-{[6-amino-8-methoxy-2-(2-methoxyethoxy)-9H-purin-9-yl] methyl}benzyl) (methyl)amino]propyl}(tert-butoxycarbonyl)amino]methyl}phenyl)acetate (145mg, 0.2mmol) were added 4N hydrochloric acid-dioxane (8ml) and methanol (2ml), and the mixture was reacted at room temperature for 4 hours. After concentration, the residue was subjected to azeotropic distillation with methanol three times, and the solution was neutralized with saturated sodium hydrogencarbonate. The resulting solid was filtered and washed with water. The solvent was dried in vacuo to give the titled compound 33mg as a white solid. Yield 27%
¹H NMR (DMSO-d₆) δ7.28-7.14 (7H, m), 7.08 (1H, d, J = 7.2 Hz), 6.46 (2H, s), 4.82 (2H, s), 4.26 (2H, t, J = 4.7 Hz), 3.65-3.51 (9H, m), 3.27 (3H, s), 2.33 (2H, t, J = 6.8 Hz), 2.05 (3H, s), 1.65-1.54 (2H, m).

### Example 42

(3-{2-[(3-{[5-(6-Amino-2-butoxy-8-oxo-7,8-dihydropurin-9-ylmethyl)pyridin-2-yl]methylamino}propyl)methylamino]ethoxy}phenyl)acetic acid methyl ester

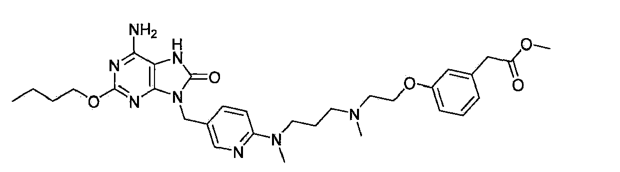

The compound 500mg (1.38mmol) obtained by example 17 step (i) was suspended in N,N'-dimethylpropanediamime (2ml) and stirred at 140°C for 48 hours. After cooling to room temperature, the mixture was neutralized at 0°C with 1N aqueous hydrochloric acid. The crystals resulted at pH7.5 were filtered, and dried to give the product containing the salt (697mg). One hundred and fifty mg of them without purification were dissolved in DMF (15ml) and thereto were added diisopropylethylamine 140mg (1.1mmol) and methyl 3-(2-bromoethoxy)phenylacetate 119mg (0.43mmol), followed by stirring at 80°C for 24 hours. After concentration of the solvent, thereto was added water 3 ml and the deposit at pH7 was filtered, and dried. The solid was purified by column chromatography (silica gel 10 g, chloroform:methanol=25:1→chloroform:methanol:28% aqueous ammonia= 100:4: 1) to give the titled compound 41mg as a white solid. Yield 19%
¹H NMR (CDCl₃) δ 10.1 (1H, brs), 8.07 (1H, d, J= 2.4 Hz), 7.46 (1H, dd, J= 8.8 Hz, 2.4 Hz), 7.20 (1H, t, J= 7.8 Hz), 6.83-6.80 (3H, m), 6.55 (1H, d, J= 8.8 Hz), 6.47 (2H, brs), 4.69 (2H, s), 4.16 (2H, t, J= 6.6 Hz), 4.16 (2H, t, J= 5.8 Hz), 3.62 (2H, s), 3.60 (3H, s), 3.47 (2H, t, J= 7.3 Hz), 2.94 (3H, s), 2.68 (2H, t, J= 5.8 Hz), 3.38 (2H, t, J= 6.9 Hz), 2.22 (3H, s), 1.66-1.62 (4H, m), 1.41-1.36 (2H, m), 0.92 (3H, t, J= 7.4 Hz).

### Example 43

(3-{2-[(3-{[5-(6-Amino-2-butoxy-8-oxo-7,8-dihydropurin-9-ylmethyl)pyridin-2-yl]methylamino}propyl)xnethylamino]ethoxy}phenyl)acetic acid

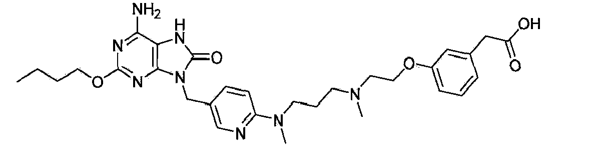

Using the compound 10mg (0.016mmol) obtained by example 42, in the same manner as example 2, there was obtained the titled compound 7mg as a white solid. Yield 71%
¹H NMR (DMSO-d₆) δ 11.7 (1H, brs), 8.05 (1H, d, J= 2.2 Hz), 7.44 (1H, dd, J= 8.7 Hz, 2.2 Hz), 7.12 (1H, t, J= 7.8 Hz), 6.87 (2H, brs), 6.80-6.76 (2H, m), 6.71 (1H, d, J= 8.1 Hz), 6.55 (1H, d, J= 8.7 Hz), 4.69 (2H, s), 4.15 (2H, t, J= 6.6 Hz), 3.93 (2H, t, J= 5.8 Hz), 3.48 (2H, t, J= 7.1 Hz),3.40 (2H, s), 2.93 (3H, s), 2.61 (2H, t, J= 5.8 Hz), 2.31 (2H, t, J= 6.9 Hz), 2.20 (3H, s), 1.66-1.60 (4H, m), 1.42-1.37 (2H, m), 0.92 (3H, t, J= 7.4 Hz).

### Example 44

7,8-Dihydro-2-methoxyethylamino-9-(4-[4-{2-[3-(methoxycarbonylmethyl)phenoxy]ethyl}piperazinylmethyl]benzyl)-8-oxoadenine

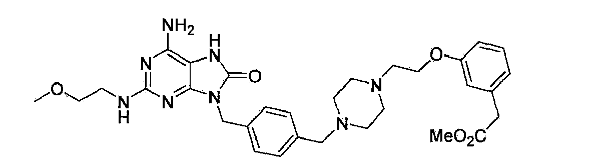

### Step (i)

2-Methoxyethylamino-9-(tetrahydropyran-2-yl)adenine

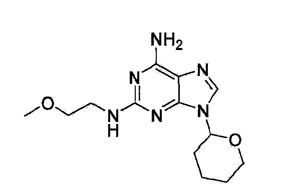

To 2-Chloro-9-(tetrahydropyran-2-yl)adenine (2.5g, 10mmol) in 1-butanol (100mL, 0.1M) were added iPr2EtN (646mg, 5.0eq) and methoxyethylamine (8.73mL, 10.0eq.), and the mixture was stirred at 150°C for 13 hours in an autoclave. After checking disappearance of the starting materials by TLC analysis (10% methanol-chloroform), the mixture was cooled to room temperature. Thereto were added water (500mL) and 5% methanol-chloroform (500mL x 2) and the mixture was separated by a separating funnel. The organic layers were combined and washed with brine (500mL). After removal of the solvent by distillation, the yellow oily residue was dissolved in ethyl acetate (10mL). To the solution was added at 0°C hexane (30mL) and the solution was stirred for 1 hour to give crystals. The crystals were filtered, and washed with hexane (200mL).
The crystals were dried in vacuo to give the titled compound 2.60g as white crystals. Yield 89%
¹H NMR (CDCl₃) δ 7.70 (1H, s), 5.53 (1H, t), 5.39 (2H, bs), 4.13 (1H, m), 3.74 (1H, m), 3.60-3.64(2H, m), 3.55-3.58 (2H, m), 3.38 (3H, s), 2.01-2.05 (3H, m), 1.63-1.76 (3H, m).

### Step (ii)

8-Bromo-2 -methoxyethylamino-9- (tetrahydropyran-2 -yl)adenine

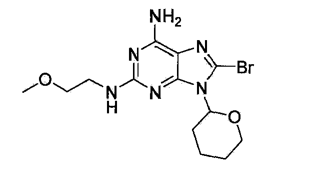

To the compound (2.92 g, 10.0mmol) obtained in step (i) in chloroform (100mL, 0.1M) was added at 0 °C in a period of 30 minutes bromine (0.54mL, 1.68g, 10.5mmol, 1.05eq.) in chloroform (105mL, 0.1M), and the mixture was stirred at 0°C for 30 minutes. After checking disappearance of the starting materials by LCMS, thereto were added saturated sodium hydrogencarbonate (100mL) and aqueous saturated thiosodium sulfate (100mL) to quench the reaction. To the mixture were added water (100mL) and 5% methanol-chloroform (250ml x 2) and the mixture was separated by a separating funnel. The organic layer was washed with brine (200mL) and the solvent was removed by distillation.
The resulting green oily product was dissolved in ethyl acetate (5mL) and sonicated to give crystals. The crystals were repulp-washed with ethyl acetate (10mL), and thereto was added at room temperature hexane (30mL). After stirring for 1 hour and completely crystallization, the crystals were filtered and washed with hexane (100mL). The crystals were dried in vacuo to give the titled compound 2.95g as pale green crystals. Yield 79%
¹H NMR (CDCl₃) δ 5.51 (1H, dd), 5.39(1H, bs), 5.30 (2H, bs), 4.15-4.19 (1H, m), 3.66-3.72 (1H, m), 3.55-3.61 (4H, m), 3.38 (3H, s), 3.04-3.20 (1H, m), 2.04-2.11 (1H, m), 1.58-1.82 (4H, m).

### Step (iii)

8-Methoxy-2-methoxyethylamino-9-(tetrahydropyran-2-yl)adenine

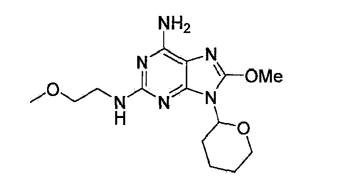

To the compound(2.95g, 7.94mmol) obtained in step (ii) in methanol (79mL, 0.1M) was added 1N aqueous sodium hydroxide (79mL, 100%v/v) and the mixture was refluxed at 90°C for 16 hours. After checking disappearance of the starting materials with LCMS, the mixture was cooled to room temperature and the reaction was quenched (neutralized) with acetic acid (4.52mL, 79mmol). After making the mixture week basic with sat.NaHCO₃ aq. (10 mL), the solvent was removed. To the residue were added water (500mL) and 5% methanol-chloroform (500mL x 2) and the mixture was separated by a separating funnel. The organic layer was washed with brine (500mL) and the solvent was removed to give the object compound as a white amorphous.
¹H NMR (CDCl₃) δ 5.51(2H, bs), 5.41 (1H, dd), 4.09-4.14 (1H, m), 4.09 (3H, s), 3.63 (1H, t), 3.55-3.57 (4H, m), 3.37 (3H, s), 2.76-2.82 (1H, m), 2.02-2.07 (1H, m), 1.54-1.76 (4H, m).

### Step (iv)

8-Methoxy-2-methoxyethylaminoadenine trifluoroacetate

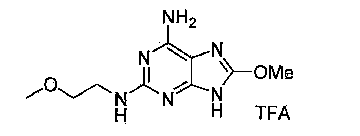

To the compound (7.94mmol) obtained in step (iii) in methanol (79mL, 0.1M) was gradually added at 0°C in a period of 30 minutes trifluoroacetic acid (39mL, 50%v/v), and the mixture was stirred at room temperature for 14 hours. After checking disappearance of the starting materials with TLC (10% methanol-chloroform), MeOH and TFA were removed by distillation. The residue was crystallized from hexane (10 mL)-EtOAc (2mL). After stirring at 0°C for 1 hour, the resulting white deposit was completely crystallized. The crystals were filtered, washed with hexane (100 mL), and dried in vacuo at 40°C to give the object compound 1.96g as white crystals. Yield 71%
¹H NMR (DMSO-d₆) δ 4.05 (3H, s), 3.48 (4H, bs), 3.28 (3H, s).

### Step (v)

8-Methoxy-2-methoxyethylamino-9-(4-hydroxymethylbenzyl)adenine

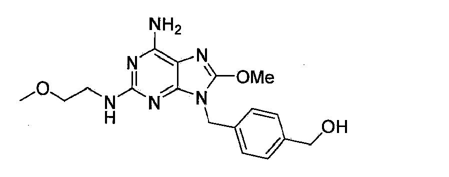

To the compound (1.96g, 5.56mmol) obtained in step (iv) in DMF (56mL, 0.1M) were added K₂CO₃ (3.8g, 5.0eq.) and 4-chloromethylbenzyl alcohol (915mg, 1.05eq), and the mixture was stirred at room temperature for 24 hours. After checking disappearance of the starting materials with LCMS, the reaction mixture was extracted with 10% MeOH-CHCl₃ (500mL x 2)-water, and the organic layer was washed with brine. After removal of the solvent by distillation, the residue was repulp-washed with acetone (ca. 10mL). After crystallization of white deposit by stirring at 0°C for 1 hour, the crystals were filtered and dried in vacuo at 40°C to give the object compound 1.12g as white crystals. Yield 56%
¹H NMR (DMSO-d₆) δ 7.25 (2H, d), 7.18 (2H, d,), 6.37 (2H, bs), 6.03 (1H, t, J = 5.6 Hz), 5.16 (1H, t, J = 5.8 Hz), 4.96 (2H, s), 4.44 (2H, d, J = 5.6 Hz), 3.99 (3H, s), 3.30-3.44 (4H, m), 3.24 (3H, s).

### Step (vi)

7,8-Dihydro-2-methoxyethylamino-9-(4-chloromethylbenzyl)-8-oxoadenine

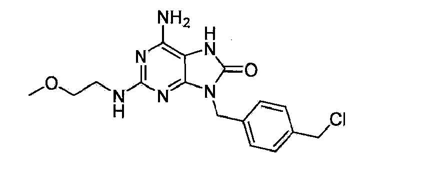

To a suspension of the compound (402mg, 1.12mmol) obtained in step (v) in CHCl₃ (12mL, 0.1M) was added SOCl₂ (1.18g, 10.0eq.) and the mixture was stirred at room temperature for 3 hours. After checking disappearance of the starting material by LCMS, the solvent was removed by azeotropic distillation with toluene (twice) to give the object compound 460mg as pale brown crystals. Yield 100%
¹H NMR (DMSO-d₆) δ 7.40 (2H, d), 7.32 (2H, d), 4.86 (2H, s), 4.74 (2H, s), 3.45 (4H, s), 3.25 (3H, s).

### Step (vii)

7,8-Dihydro-2-methoxyethylamino-9-(4-[4-{2-[3-(methoxycarbonylmethyl)phenoxy]ethyl}piperazinylmethyl]benzyl)-8-oxoadenine

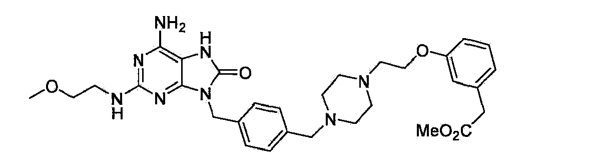

To the compound (447mg, 1.12mmol) obtained in step (vi) in DMF (0.1M) was added methyl 3-(2-N-piperazinylethoxy)benzylcarboxylate (1.23mmol) and ⁱPr₂EtN(10.0mmol), and the mixture was stirred in a bath at 60°C. After checking disappearance of the starting material by LCMS, the mixture was cooled to room temperature. After removal of the solvent by distillation, the residue was purified by column chromatography to give the titled compound 220mg as a white solid. Yield 33%
¹H NMR (DMSO-d₆) δ 7.26-7.20 (5H, m), 6.84 (3H, m), 6.13 (2H, brs), 6.09 (1H, t, J = 5.6 Hz), 4.80 (2H, s), 4.07 (2H, t, J = 5.45 Hz), 3.64 (2H, m), 3.61 (4H, m), 3.41-3.38 (2H, m), 3.35-3.32 (2H, m), 3.23 (3H, s), 2.72-2.70 (2H, m), 1.30-1.20 (6H, m).

### Example 45

7,8-Dihydro-2-(4-pyridylmethylamino)-9-(4-[N-methyl-N-{4-[3-(methoxycarbonylmethyl)phenoxy]butyl}aminomethyl]benzyl)-8-oxoadenine

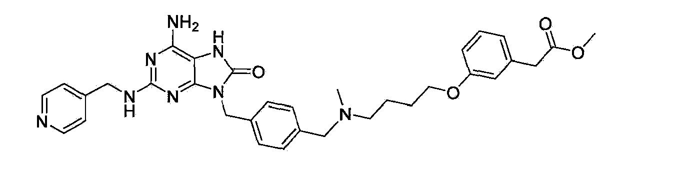

### Step (i)

2-Chloro-9-(4-hydroxymethylbenzyl)adenine

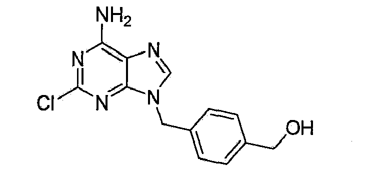

To 2-chloro-6-aminopurine (1.69g, 10mmol) in DMF (50mL, 0.2M) were added K₂CO₃ (4.15g, 3.0eq) and 4-chloromethylbenzyl alcohol (2.34g, 1.5eq.), and the mixture was stirred at room temperature for 24 hours. Thereto were added water (1.0L) and 10% MeOH-GHCl₃ (1.0L x 2) and the mixture was separated by a separating funnel. The organic layers were combined, and washed with water and brine (500mL). After removal of the solvent by distillation, to the resulting residue were added ethyl acetate (20mL) and hexane (200mL) and the mixture was stirred at room temperature for 1 hour to crystallize. The resulting crystals were filtered, washed with hexane (200mL), and dried at 40°C in vacuo to give the object compound 2.32g as pale brawn crystals. Yield 80%
¹H NMR (DMSO-d₆) δ 8.25 (1H, s), 7.80 (2H, bs), 7.29 (2H, d), 7.23 (2H, d), 5.31 (2H, s), 5.18 (1H, t), 4.46 (2H, d).

### Step (ii)

2-(4-Pyridylinethylamino)-9-(4-hydroxymethylbenzyl)adenine

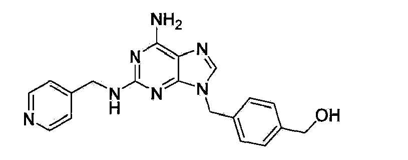

To the compound (2.90g, 10mmol) obtained in step (i) in NMP (20mL, 0.5M) were added ⁱPr₂EtN (3.88g, 3.0eq) and 4-pyridylmethylamine (5.0mL, 25%v/v.) and the mixture was stirred at 180°C in an autoclave for 20 hours. After checking disappearance of the starting material with LCMS, the temperature was cooled to room temperature. Thereto were added water (500mL) and 5% methanol-chloroform (1L x 2) and the mixture was separated by a separating funnel. The organic layers were combined, washed with brine (500mL) and the solvent was removed by distillation. The residue was purified by column chromatography (SiO₂: eluate: CHCl₃→2% MeOH-CHCl₃→5% MeOH-CHCl₃), and the resulting pale brawn amorphous was repulp-washed with acetone (20mL). The resulting crystals were filtered and dried in vacuo to give the object compound 900mg as white crystals. Yield 25%
¹H NMR (DMSO-d₆) δ 8.44 (2H, dd), 7.79 (1H, s), 7.30 (2H, dd), 7.14-7.19 (4H, m), 6.97 (1H, t), 6.72 (2H, bs), 5.13 (1H, t), 5.09 (2H, s), 4.48 (2H, d), 4.43 (2H, d).

### Step (iii)

8-Bromo-2-(4-pyridylmethylamino)-9-(4-hydroxymethylhenzyl)adenine

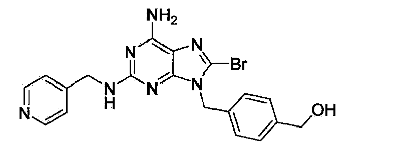

To the compound (460mg, 1.27mmol) obtained in step (ii) in 10% MeOH-chloroform (46mL) was dropped at 0°C over a period of 30 minutes bromine (203mg, 1.27mmol, 1.0 eq.) in chloroform (12.7mL, 0.1M). After checking disappearance of the starting material with LCMS, thereto were added sat. NaHCO₃ aq. (100mL) and sat. Na₂S₂O₃ aq. (100mL) to quench the reaction. The reaction solution was extracted with water (100mL) and 25% EtOH-chloroform (500ml x 2) by a separating funnel and the solvents are removed by distillation. To the resulting pale pink crystal was added ethyl acetate (5mL) and the mixture was repulp-washed while stirring at room temperature for 1 hour. After filtration and drying in vacuo (40°C), the object compound 548mg as pale pink crystals were obtained. Yield 98%
¹H NMR (DMSO-d₆) δ 8.44 (2H, dd), 7.29 (2H, d), 7.16-7.19 (3H, m), 7.09 (2H, d), 6.97 (2H, bs), 5.18 (1H, t), 5.09 (2H, s), 4.47 (2H, d), 4.43 (2H, d).

### Step (iv)

8-Methoxy-2-(4-pyridylmethylamino)-9-(4-hydroxymethylbenzyl)adenine

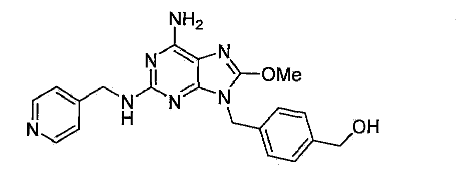

To a suspension of the compound (352mg, 0.8mrnol) obtained in step (iii) in methanol (200mL) was added potassium methoxide (1.12g, 20eq.) and the mixture was stirred in an autoclave at 120°C for 12 hours. After checking disappearance of the starting material with LCMS, the reaction mixture was cooled to room temperature, and thereto were added water (300mL) and 25% EtOH-chloroform (500mL x 2) to extract by a separating funnel. After removal of the solvent by distillation, to the residue was added ethyl acetate (5mL). After crystallization by ultrasonicating, the crystals were repulp-washed while stirring for 1 hour at room temperature, filtered and dried at 40°C in vacuo to give the object compound 250mg as white crystals. Yield 80%
¹H NMR (DMSO-d₆) δ 8.44 (2H, dd), 7.29 (2H, d), 7.18 (2H, d), 7.08 (2H, d), 6.87 (1H, t), 6.41 (2H, bs), 5.15 (1H, t), 4.89 (2H, bs), 4.42-4.45 (4H, m), 3.98 (3H, s).

### Step (v)

7,8-Dihydro-2-(4-pyridylmethylamino)-9-(4-chloromethylbenzyl)-8-oxoadenine

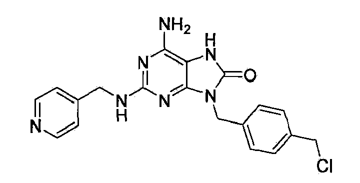

To a suspension of the compound (200mg, 0.512mmol) obtained in step (iv) in CHCl₃ (51mL, 0.01M) was added SOCl₂ (1.8mL, 50eq.) and the mixture was stirred at room temperature for 3 hours. After checking disappearance of the starting material with LCMS, the reaction mixture was cooled to room temperature and the solvent was removed by distillation to give the titled compound 205mg as white crystals. Yield 100%
¹H NMR (DMSO-d₆) δ 11.1 (1H, bs), 8.80 (2H, d), 8.17-8.55 (2H, bs), 7.97 (2H, s), 7.25 (2H, d), 7.04 (2H, d), 4.80 (2H, s), 4.74 (2H, s), 4.71 (2H, s).

### Step (vi)

7,8-Dihydro-2-(4-pyridylmethyl)amino-9-(4-[N-methyl-N-{4-[3-(methoxycarbonylmethyl)phenoxy]butyl}aminomethyl]benzyl)-8-oxoadenine

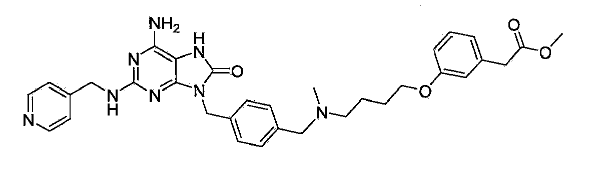

To the compound (100mg, 0.23mmol) obtained in step (v) in DMF (0.1M) were added methyl 3-[4-(N-methylamino)butoxy]benzylcarboxylate (0.28mmol) and ⁱPr₂EtN (1.15mmol) and the mixture was stirred at bath temperature 50°C. After checking disappearance of the starting material with LCMS, the reaction mixture was cooled to room temperature and the solvent was removed by distillation. The residue was isolated by column chromatography to give the titled compound 28 mg as white crystals.
Yield 20%
¹H NMR (MeOH-d₄) δ 8.25-8.27 (2H, m), 7.24 (2H, d, J = 6.0 Hz), 7.03-7.07 (5H, m), 6.63-6.69 (3H, m), 4.73 (2H, s), 4.45 (2H, s), 3.81 (2H, t, J = 5.7 Hz), 3.53 (3H, s), 3.47 (2H, s), 3.37 (2H, s), 2.29 (2H, t, J = 7.6 Hz), 2.06 (3H, s), 1.56-1.64 (6H, m).

## Claims

1. An adenine compound represented by the following formula (1): 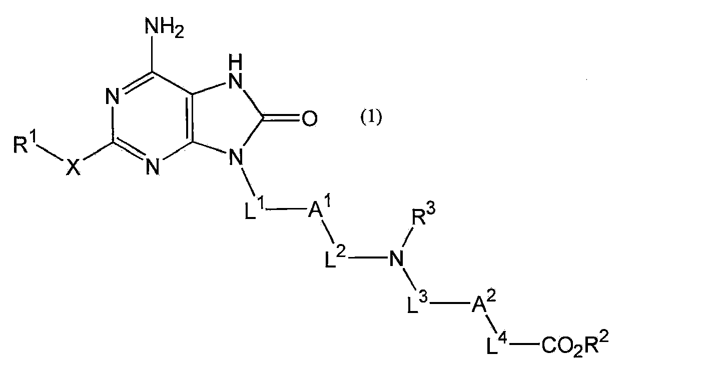 [wherein
A¹ and A² are independently, optionally substituted aromatic carbocyclic group or optionally substituted aromatic heterocyclic group;
L⁴ is optionally substituted alkylene or a single bond;
R¹ is halogen atom, optionally substituted alkyl group, optionally substituted alkenyl group, optionally substituted alkynyl group, optionally substituted cycloalkyl group, optionally substituted saturated heterocyclic group, optionally substituted aryl group or optionally substituted heteroaryl group;
R² is hydrogen atom or optionally substituted alkyl group;
R³ is optionally substituted alkyl group, optionally substituted alkenyl group, optionally substituted alkynyl group, optionally substituted cycloalkyl group, optionally substituted saturated heterocyclic group, optionally substituted aryl group or optionally substituted heteroaryl group;
X is oxygen atom, sulfur atom, NR⁹ (wherein R⁹ is hydrogen atom or alkyl group), SO, SO₂ or a single bond, provided that X is a single bond when R¹ is halogen atom;
and
L¹, L² and L³ are independently, alkylene, alkenylene, alkynylene or a single bond and any methylene group in said group may be substituted by oxygen atom, sulfur atom, SO, SO₂, carbonyl group, NR⁴, NR⁴CO, CONR⁴, NR⁴SO₂, SO₂NR⁴, NR⁴CO₂. OCONR⁴, NR⁵CONR⁴, NR⁶C(=NR⁴)NR⁵ or C(=NR⁴) NR⁵ (wherein R⁴, R⁵ and R⁶ are independently, hydrogen atom or optionally substituted alkyl group), and any methylene, methyne or imino in L² or L³ may be bound with N atom adjacent to L² and L³ to form 4 to 7 membered saturated nitrogen containing heterocycle, and when methylene in L³ is substituted by NR⁴, optionally substituted alkyl group in R⁴ may be bound with carbon atom in L³ to form 4 to 7 membered saturated nitrogen containing heterocycle.]
or its pharmaceutically acceptable salt.

2. The adenine compound or its pharmaceutically acceptable salt according to claim 1, wherein substituted aromatic carbocyclic group or substituted aromatic heterocyclic group in A¹ and A² of the formula (1) is substituted by one or more substituents selected from the group consisting of halogen atom, hydroxy group, C₁₋₆ alkyl group, C₁₋₆ alkoxy group, C₁₋₆ haloalkyl group, C₁₋₆ haloalkoxy group, carboxy group, C₂₋₆ alkoxycarbonyl group, nitro group and amino group optionally substituted by one or more C₁₋₆ alkyl groups;
substituted alkyl group in R⁴, R⁵ and R⁶ is substituted by one or more substituents independently selected from the group consisting of halogen atom, hydroxy group, C₁₋₆ alkoxy group and optionally substituted amino group;
substituent of the above substituted amino group is a group selected from the group (a') or a group (b');
(a') C₁₋₆ alkyl group, C₂₋₆ alkenyl group, C₂₋₆ alkynyl group, C₂₋₆ alkylcarbonyl group, C₂₋₆ alkoxycarbonyl group, C₁₋₆ alkylsulfonyl group, C₁₋₆alkylsulfinyl group, 3 to 8 membered cycloalkyl group, 3 to 8 membered cycloalkylcarbonyl group, 3 to 8 membered cycloalkoxycarbonyl group, 3 to 8 membered cycloalkylsulfonyl group and 3 to 8 membered cycloalkylsulfinyl group (wherein the group in this group may be substituted by halogen atom, hydroxy group, C₁₋₆ alkyl group, C₁₋₆ alkoxy group, carboxy group and C₂₋₆ alkoxycarbonyl group.);
(b') 4 to 7 membered saturated heterocyclic group having one to three hetero atoms selected from 1 to 3 nitrogen atoms, 0 to 1 oxygen atom and 0 to 1 sulfur atom which is formed by combining two substituents with the nitrogen atom (said saturated heterocyclic group may be substituted on its carbon atom or nitrogen atom, if chemically stable, by halogen atom, hydroxy group, carboxy group, C₁₋₆ alkyl group, C₁₋₆ alkoxy group, C₂₋₆ alkoxycarbonyl group or C₂₋₆ alkylcarbonyl group.),
substituted alkyl group in R¹, R² and R³, and substituted alkenyl group and substituted alkynyl group in R¹ and R³ is substituted by one or more substituents independently selected from the group consisting of groups (a) to (c) below;
(a) halogen atom, hydroxy group, carboxy group, C₁₋₆ haloalkoxy group and mercapt group;
(b) C₁₋₆ alkoxy group, C₁₋₆ alkylthio group, C₂₋₆ alkylcarbonyl group, C₂₋₆ alkylcarbonyloxy group, C₁₋₆ alkylsulfonyl group, C₁₋₆ alkylsulfinyl group, and C₂₋₆ alkoxycarbonyl group (wherein the group of this group may be substituted by one or more substituents independently selected from the group consisting of halogen atom, hydroxy group, carboxy group, C₁₋₆ alkoxy group, C₂₋₆ alkoxycarbonyl group, amino group optionally substituted by the same or different and one or two C₁₋₆ alkyl groups, carbamoyl group optionally substituted by the same or different and one or two C₁₋₆ alkyl groups, sulfamoyl group optionally substituted by the same or different and one or two C₁₋₆ alkyl groups, and C₁₋₆ alkylsulfonyl group);
(c) optionally substituted amino group, optionally substituted carbamoyl group and optionally substituted sulfamoyl group (wherein the group of this group may be substituted by one or more substituents selected from the group consisting of groups (j), (k) and (1) below), optionally substituted 3 to 8 membered cycloalkyl group and optionally substituted 4 to 8 membered saturated heterocyclic group (wherein the group of this group may be substituted by one or more substituents selected from the group consisting of groups (d), (e) and (f) below), and optionally substituted 6 to 10 membered aryl group, optionally substituted 5 to 10 membered heteroaryl group, optionally substituted 6 to 10 membered aryloxy group and optionally substituted 5 to 10 membered heteroaryloxy group (wherein the group of this group may be substituted by one or more substituents selected from the group consisting of groups (g), (h) and (i) below);
substituted 3 to 8 membered cycloalkyl group and substituted 4 to 8 membered saturated heterocyclic group in R¹ and R³ is substituted by one or more substituents independently selected from the group consisting of groups (d) to (f) below;
(d) halogen atom, hydroxy group, carboxy group, mercapto group, C₁₋₆ haloalkyl group and C₁₋₆ haloalkoxy group;
(e) C₁₋₆ alkyl group, C₂₋₆ alkenyl group, C₂₋₆ alkynyl group, C₁₋₆ alkoxy group, C₂₋₆ alkylcarbonyl group, C₂₋₆ alkylcarbonyloxy group, C₂₋₆ alkoxycarbonyl group, C₁₋₆ alkylthio group, C₁₋₆ alkylsulfonyl group, and C₁₋₆ alkylsulfinyl group (wherein the group of this group may be substituted by one or more substituents independently selected from the group consisting of halogen atom, hydroxy group, carboxy group, C₁₋₆ alkoxy group, C₂₋₆ alkoxycarbonyl group, amino group optionally substituted by the same or different and one or two C₁₋₆ alkyl groups, carbamoyl group optionally substituted by the same or different and one or two C₁₋₆ alkyl groups, sulfamoyl group optionally substituted by the same or different and one or two C₁₋₆ alkyl groups, and C₁₋₆ alkylsulfonyl group.);
(f) optionally substituted amino group, optionally substituted carbamoyl group and optionally substituted sulfamoyl group (wherein the group of this group may be substituted by one or two substituents selected from the group consisting of groups (j), (k) and (l) below), and optionally substituted 6 to 10 membered aryl group and optionally substituted 5 to 10 membered heteroaryl group (the group of this group may be substituted by one or more substituents selected from the group consisting of groups (g), (h) and (i) below);
substituted aryl group and substituted heteroaryl group in R¹ and R³ are substituted by one or more substituents independently selected from the group consisting of groups (g) to (i) below;
(g) halogen atom, hydroxy group, mercapto group, cyano group, nitro group, C₁₋₆ haloalkyl group, and C₁₋₆ haloalkoxy group;
(h) C₁₋₆ alkyl group, C₂₋₆ alkenyl group, C₂₋₆ alkynyl group, C₁₋₆ alkoxy group, C₂₋₆ alkylcarbonyl group, C₂₋₆ alkylcarbonyloxy group, C₁₋₆ alkylthio group, C₁₋₆ alkylsulfonyl group, C₁₋₆ alkylsulfinyl group, 3 to 8 membered cycloalkyl group and 4 to 8 membered saturated heterocyclic group (wherein the group of this group may be substituted by one or more substituents independently selected from a group consisting of halogen atom, hydroxy group, C₁₋₆ alkyl group, C₁₋₆ alkoxy group, amino group optionally substituted by the same or different and one or two C₁₋₆ alkyl groups, carbamoyl group optionally substituted by the same or different and one or two C₁₋₆ alkyl groups, sulfamoyl group optionally substituted by the same or different and one or two C₁₋₆ alkyl groups and C₁₋₆ alkylsulfonyl group.);
(i) optionally substituted amino group, optionally substituted carbamoyl group, and optionally substituted sulfamoyl group (the group of this group may be substituted by one or two substituents selected from group consisting of groups (j), (k) and (l) below);
substituted amino group, substituted carbamoyl group and substituted sulfamoyl group in the above groups (a) to (i) are substituted by one or two substituents independently selected from the group consisting of groups (j) to (l) below;
(j) C₁₋₆ alkyl group, C₂₋₆ alkenyl group, C₂₋₆ alkynyl group, C₂₋₆ alkylcarbonyl group, C₂₋₆ alkoxycarbonyl group, C₁₋₆ alkylsulfonyl group, C₁₋₆ alkylsulfinyl group, 3 to 8 membered cycloalkyl group, 3 to 8 membered cycloalkylcarbonyl group, 3 to 8 membered cycloalkoxycarbonyl group, 3 to 8 membered cycloalkylsulfonyl group, 3 to 8 membered cycloalkylsulfinyl group (wherein the group of this group may be substituted by one or more substituents independently selected from the group consisting of halogen atom, hydroxy group, carboxy group, C₁₋₆ alkyl group, C₁₋₆ alkoxy group, C₂₋₆ alkoxycarbonyl group, amino group optionally substituted by the same or different and one or two alkyl groups, carbamoyl group optionally substituted by the same or different and one or two alkyl groups, sulfamoyl group optionally substituted by the same or different and one or two alkyl groups and alkylsulfonyl group.);
(k) 6 to 10 membered aryl group, 6 to 10 membered arylcarbonyl group, 6 to 10 membered aryloxycarbonyl group, 6 to 10 membered arylsulfonyl group, 6 to 10 membered arylsulfinyl group, 5 to 10 membered heteroaryl group, 5 to 10 membered heteroarylcarbonyl group, 5 to 10 membered heteroaryloxycarbonyl group, 5 to 10 membered heteroarylsulfonyl group, and 5 to 10 membered heteroarylsulfinyl group (wherein the group of this group may be substituted by halogen atom, hydroxy group, C₁₋₆ alkyl group, C₁₋₆ alkoxy group, amino group optionally substituted by the same or different and one or two C₁₋₆ alkyl groups, carbamoyl group optionally substituted by the same or different and one or two C₁₋₆ alkyl groups, sulfamoyl group optionally substituted by the same or different and one or two C₁₋₆ alkyl groups, or C₁₋₆ alkylsulfonyl group);
(l) 4 to 7 membered saturated heterocyclic group containing 1 to 4 hetero atoms selected from 1 to 2 nitrogen atoms, 0 to 1 oxygen atom, and 0 to 1 sulfur atom which is formed by combining two substituents with the nitrogen atom (said saturated heterocyclic group may be substituted on appropriate carbon atom or nitrogen atom, if chemically stable, by halogen atom, hydroxy group, carboxy group, C₁₋₆ alkyl group, C₁₋₆ alkoxy group, C₂₋₆ alkoxycarbonyl group, C₂₋₆ alkylcarbonyl group, amino group which may be substituted by the same or different and one or two C₁₋₆ alkyl groups, carbamoyl group which may be substituted by the same or different and one or two C₁₋₆ alkyl groups, sulfamoyl group which may be substituted by the same or different and one or two C₁₋₆ alkyl groups, or C₁₋₆ alkylsulfonyl group.

3. The adenine compound or its pharmaceutically acceptable salt according to claim 1 or 2, wherein in the formula (1), A¹ and A² are independently, optionally substituted benzene ring, or optionally substituted 5 to 6 membered aromatic heterocyclic group containing 1 to 3 hetero atoms selected from 0 to 3 nitrogen atoms, 0 to 1 oxygen atom and 0 to 1 sulfur atom.

4. The adenine compound or its pharmaceutically acceptable salt according to any one of claims 1 to 3, wherein in the formula (1), R² is C₁₋₄ alkyl group.

5. The adenine compound or its pharmaceutically acceptable salt according to claim 4, wherein in the formula (1), R² is methyl group.

6. The adenine compound or its pharmaceutically acceptable salt according to any one of claims 1 to 5, wherein in the formula (1), L¹ is C₁₋₄ alkylene.

7. The adenine compound or its pharmaceutically acceptable salt according to any one of claims 1 to 6, wherein in the formula (1), L⁴ is a single bond or C₁₋₄ alkylene.

8. The adenine compound or its pharmaceutically acceptable salt according to claim 7, wherein in the formula (1), L⁴ is methylene.

9. The adenine compound or its pharmaceutically acceptable salt according to any one of claims 1 to 8, wherein in the formula (1), -L2-NR3-L3- is a formula selected from the formulas (2)~(7):
formula (2): -(O)ₚ-(CH₂)ₘ-NR³'-(CH₂)ₙ-(O)_{q}-
wherein R³' is hydrogen atom; C₁₋₆ alkyl group; or C₂₋₆ alkyl group substituted by halogen atom, optionally substituted amino group, or hydroxy group, p and q are independently 0 or 1, and m and n are independently 1 to 4 integers, provided that when p is 1, m is 2 or more, and when q is 1, n is 2 or more.; 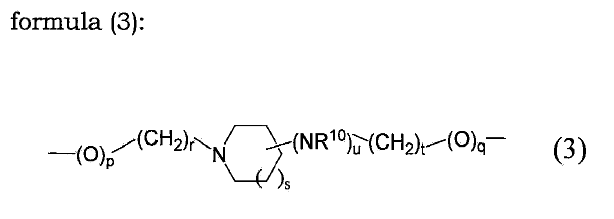 wherein R¹⁰ is hydrogen atom or C₁₋₆ alkyl group, p and q are the same as defined above, r and t are independently an integer of 0 to 4, s is an integer of 0 to 2, u is 0 or 1, provided that when p is 1, r is 2 or more, and when both u and q are 1, t is 2 or more; 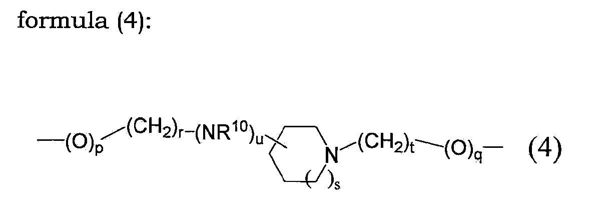 wherein R¹⁰, p and q are the same as defined above, r and t are independently an integer of 0 to 4, s is an integer of 0 to 2 u is 0 or 1, provided that when both p and u are 1, r is 2 or more, and when q is 1, t is 2 or more; 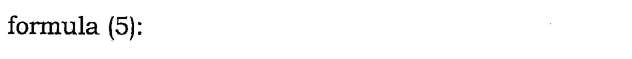 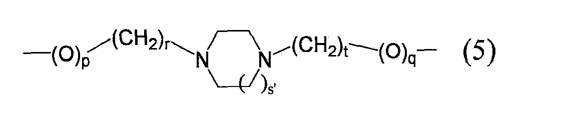 wherein p and q are the same as defined above, r and t are independently an integer of 0 to 4, s' is 1 or 2, provided that when p is 1, r is 2 or more, and when q is 1, t is 2 or more;
formula (6): -≡-(CH₂)ᵥ-NR^{3'}-(CH₂)ₙ-(O)_{q}- (6)
wherein R^{3'}, q and n are the same as defined above, and v is an integer of 0 to 3, provided that when q is 1, n is 2 or more;
formula (7): -CO-NR³'-(CH₂)ₙ-(O)_{q}-
wherein R³', q and n are the same as defined above, provided that when q is 1, n is 2 or more.

10. The adenine compound or its pharmaceutically acceptable salt according to claim 1, which is represented by formula (8): 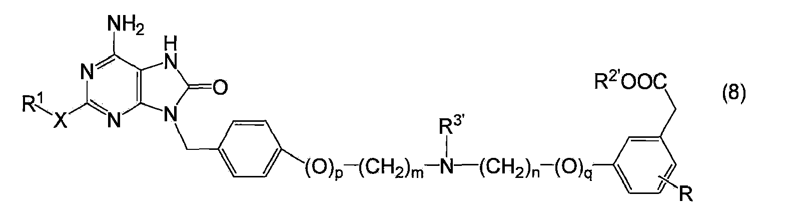 wherein R¹ and X is the same as defined in the formula (1), R is hydrogen atom, halogen atom, C₁₋₆ alkyl group or C₁₋₆ alkoxy group, R²' is hydrogen atom or methyl group, and R^{3'}, m, n, p and q are the same as the definition in the formula (2).

11. The adenine compound or its pharmaceutically acceptable salt according to claim 1 selected from the group of the following compounds:
2-Butoxy-7,8-dihydro-[(3-[{N-methyl-N-[3-(methoxycarbonylmethyl)benzyl]}amino]propoxy)benzyl]-8-oxoadenine;
2-Butoxy-7,8-dihydro-[(3-[{N-[3-(carboxymethyl)benzyl]-N-methyl}amino]propoxy)benzyl]-8-oxoadenine;
2-Butoxy-7,8-dihydro-9-([3-{N-[3-(methoxycarbonylmethyl)benzyl]amino}propoxy]benzyl)-8-oxoadenine;
2-Butoxy-7,8-dihydro-9-([3-{N-[3-(carboxymethyl)benzyl]amino}propoxy]benzyl)-8-oxoadenine;
2-Butoxy-7,8-dihydro-9-(4-{N-(3-hydroxypropyl)-N-[3-(methoxycarbonylmethyl)benzyl]aminomethyl}benzyl)-8-oxoadenine);
2-Butoxy-9-(4-{N-(3-chloroprapyl)-N-[3-(methoxycarbonylmethyl)benzyl]aminomethyl}benzyl)-7,8-dihydro-8-oxoadenine;
2-Butoxy-7,8-dihydro-9-(4-{N-[3-(methoxycarbonylmethyl)benzyl]-N-(3-morpholin-4-ylpropyl)aminomethyl}benzyl)-8-oxoadenine;
2-Butoxy-7,8-dihydro-9-(4-{N-[3-(hydroxycarbonylmethyl)benzyl]-N-(3-morpholin-4-ylpropyl)aminomethyl}benzyl)-8-oxoadenine;
2-Butoxy-7,8-dihydro-9-[4-(N-{2-[3-(methoxycarbonylmethyl)phenoxy]ethyl}-N-methylaminomethyl)benzyl]-8-oxoadenine;
2-Butoxy-7,8-dihydro-9-[4-(N-{2-[3-(hydoxycarbonylmethyl)phenoxy]ethyl}-N-methylaminomethyl)benzyl] -8-oxoadenine;
2-Butoxy-7,8-dihydro-9-[4-(N-{2-[2-methoxy-5-(methoxycarbonylmethyl)phenoxy]ethyl}-N-methylaminomethyl)benzyl]-8-oxoadenine;
2-Butoxy-7,8-dihydro-9-{6-[4-(3-methoxycarbonylmethylbenzyl)aminobutoxy] pyridin-3-ylmethyl}-8-oxoadenine;
2-Butoxy-7,8-dihydro-9-(6-{3-[N-methyl-N-(3-methoxycarbonylmethyl)phenyloxyethyl]aminopropoxy}pyridin-3-ylmethyl)-8-oxoadenine;
2-Butoxy-7,8-dihydro-9-(6-{3-[N-methyl-N-(3-hydroxycarbonylmethyl)phenyloxyethyl]aminopropoxy}pyridin-3-ylmethyl)-8-oxoadenine;
2-Butoxy-7, 8-dihydro-9-[6-(4-[{N-methyl-N-[3-(methoxycarbonylmethyl)benzyl]}amino]butoxy)pyridin-3-ylmethyl]-8-oxoadenine;
2-Butoxy-7,8-dihydro-9-[6-(4-[{N-[3-(carboxymethyl)benzyl-N-methyl]}amino]butoxy)pyridin-3-ylmethyl]-8-oxoadenine;
2-Butoxy-7,8-dihydro-9-(6-[4-{[N-(3-methoxycarbonylmethylbenzyl)-N-(3-morpholonopropyl)]amino}butoxy]pyridin-3-ylmethyl)-8-oxoadenine;
2-Butoxy-7,8-dihydro-9-(6-{4-[N-(3-hydroxycarbonylmethylbenzyl)-N-(3-morpholinopropyl)]aminobutoxy}pyridin-3-ylmethyl)-8-oxoadenine;
7,8-Dihydro-9-[4-{N-[2-(3-methoxycarbonylmethylphenyl-1-yl)oxyethyl]-N-methylamino}methylbenzyl]-2-(2-methoxyethoxy)-8-oxoadenine;
7,8-Dihydro-9-[4-{N-[2-(3-hydroxycarbonylmethylphenyl-1-yl)oxyethyl]-N-methylamino}methylbenzyl]-2-(2-methoxyethoxy)-8-oxoadenine;
2-Butoxy-7,8-dihydro-9-(4-{4-[3-(methoxycarbonylmethyl)phenoxymethyl]piperidin-1-ylmethyl}benzyl)-8-oxoadenine;
2-Butoxy-7,8-dihydro-9-(4-{4-[3-(carboxymethyl)phenoxymethyl]piperidin-1-ylmethyl}benzyl)-8-oxoadenine;
2-Butoxy-7,8-dihydro-9-[4-(N-{2-[1-methoxy-5-(methoxycarbonylmethyl)phenoxy]ethyl}aminomethyl)benzyl]-8-oxoadenine;
2-Butoxy-7,8-dihydro-9-[4-(N-{2-[1-methoxy-5-(carboxymethyl)phenoxy]ethyl}aminomethyl)benzyl]-8-oxoadenine; 7,8-Dihydro-9-(4-{4-[3-(methoxycarbonylmethyl)phenoxy]piperidin-1-ylmethyl}benzyl)-2-(2-methoxyethoxy)-8-oxoadenine;
7,8-Dihydro-9-(4-{4-[3-(carboxymethyl)phenoxy]piperidinylmethyl}benzyl)-2-(2-methoxyethoxy)-8-oxoadenine;
2-Butoxy-7,8-dihydro-9-(3-methoxycarbonyl-4-{N-[3-(methoxycarbonylmethyl)benzyl]-N-methylaminomethylpropargyl}benzyl)-8-oxoadenine;
2-Butoxy-7,8-dihydro-9-[3-fluoro-4-{4-[3-(methoxycarbonylmethyl)phenoxymethyl] piperidin-1-yl}methylbenzyl]-8-oxoadenine;
2-Butoxy-7,8-dihydro-9-[3-fluoro-4-{4-[3-(carboxymethyl)phenoxymethyl]piperidin-1-yl}methylbenzyl] -8-oxoadenine;
[3-(4-{[4-(6-Amino-2-butoxy-8-oxo-7,8-dihydropurin-9-ylmethyl)benzyl]ethylamino}butoxy)phenyl]acetic acid methyl ester;
[3-(3-{4-[4-(6-Amino-2-butoxy-8-oxo-7, 8-dihydropurin-9-ylmethyl)benzyl]piperazin-1-yl}propoxy)phenyl]acetic acid methyl ester;
[3-(2-{4-[5-(6-Amino-2-butoxy-8-oxo-7,8-dihydropurin-9-ylmethyl)pyridin-2-yl]-[1,4]diazepan-1-yl}ethoxy)phenyl]acetic acid methyl ester;
[3-(2-{4-[4-(6-Amino-2-butoxy-8-oxo-7, 8-dihydropurin-9-yhnethyl)-2-nitrophenyl]-[1,4]diazepan-1-yl}ethoxy)phenyl]acetic acid methyl ester;
[3-(2-{4-[2-Amino-4-(6-amino-2-butoxy-8-oxo-7,8-dihydropurin-9-ylmethyl)phenyl]-[1,4]diazepan-1-yl}ethoxy)phenyl]acetic acid methyl ester;
(3-{2-[(1-{4-[(6-Amino-2-butoxy-8-oxo-7,8-dihydro-9H-purin-9-yl)methyl]benzyl}piperidin-4-yl)(methyl)amino]ethoxy}phenyl)acetic acid methyl ester;
(3-{2-[{2-[{4-[(6-Amino-2-butoxy-8-oxo-7,8-dihydro-9H-purin-9-yl)methyl]benzyl}(methyl)amino]ethyl}(methyl)amino]ethoxy}phenyl)acetic acid methyl ester;
(3-{2-[4-{4-[(6-Amino-2-butoxy-8-oxo-7,8-dihydro-9H-purin-9-yl)methyl]benzyl}(methyl)aminopiperidin-1-yl]ethoxy}phenyl)acetic acid methyl ester;
(3-{2-[{[1-(4-{[6-Amino-2-(2-methoxyethoxy)-8-oxo-7,8-dihydro-9H-purin-9-yl]methyl}benzyl)-4-hydroxypiperidin-4-yl]methyl}(methyl)amino]ethoxy}phenyl)acetic acid methyl ester;
(3-{2-[9-(4-{[6-Amino-2-(2-methoxyethoxy)-8-oxo-7,8-dihydro-9H-purin-9-yl]methyl}benzyl)-1-oxa-4,9-diazaspiro[5.5]undec-4-yl]ethoxy}phenyl)acetic acid methyl ester;
{3-[({3-[(4-{[6-Amino-2-(2-methoxyethoxy)-8-oxo-7,8-dihydro-9H-purin-9-yl]methyl}benzyl)(methyl)amino]propyl}amino)methyl]phenyl}acetic acid methyl ester;
(3-{2-[(3-{[5-(6-Amino-2-butoxy-8-oxo-7,8-dihydropurin-9-ylmethyl)pyridin-2-yl]methylamino}propyl)methylamino]ethoxy}phenyl)acetic acid methyl ester;
(3-{2-[(3-{[5-(6-Amino-2-butoxy-8-oxo-7,8-dihydropurin-9-ylmethyl)pyridin-2-yl]methylamino}propyl)methylamino]ethaxy}phenyl)acetic acid;
7,8-Dihydro-2-methoxyethylamino-9-(4-[4-{2-[3-(methoxycarbonylmethyl)phenoxy]ethyl}piperazinylmethyl]benzyl)-8-oxoadenine; and
7,8-Dihydro-2-(4-pyridylmethylamino)-9-(4-[N-methyl-N-{4-[3-(methoxycarbonylmethyl)phenoxy]butyl}ammomethyl]benzyl)-8-oxoadenine

12. A pharmaceutical composition containing the adenine compound or a pharmaceutically acceptable salt thereof as claimed in any one of claims 1 to 11 as an active ingredient.

13. A TLR7 activator promoter containing the adenine compound or a pharmaceutically acceptable salt thereof as claimed in any one of claims 1 to 11 as an active ingredient.

14. An immuno-modifier containing the adenine compound or a pharmaceutically acceptable salt thereof as claimed in any one of claims 1 to 11 as an active ingredient.

15. A therapeutic or prophylactic agent for allergic diseases, viral diseases or cancers containing the adenine compound or a pharmaceutically acceptable salt thereof as claimed in any one of claims 1 to 11 as an active ingredient.

16. A therapeutic or prophylactic agent for asthma, COPD, allergic rhinitis, allergic conjunctivitis, atopic dermatosis, cancer, hepatitis B, hepatitis C, HIV, HPV, a bacterial infectious disease, or dermatosis containing the adenine compound or a pharmaceutically acceptable salt thereof as claimed in any one of claims 1 to 11 as an active ingredient.

17. A medicament for topical administration containing the adenine compound or a pharmaceutically acceptable salt thereof as claimed in any one of claims 1 to 11 as an active ingredient.
